# EUROPEAN PATENT APPLICATION

(11) **EP 3 159 695 A2**
(43) Date of publication of application: **26.04.2017**
(21) Application number: 16196925.8
(22) Date of filing: 20.06.2014
(51) Int. Cl.: G01N 33/574, C12N 5/09

(54) **METHODS FOR DIAGNOSING PANCREATIC CANCER**

(30) Priority: 20.06.2013 US 201361837358 P
(62) Divisional of application: 14813181.6
(71) Applicant: The Trustees of The University of Pennsylvania, Philadelphia, PA 19104 (US)
(72) Inventor: ZARET, Kenneth S., Philadelphia, PA 19130 (US); KIM, Jungsun, Philadelphia, PA 19014 (US)
(74) Representative: Atkinson, Jennifer

(57) **Abstract**

The present disclosure relates to the use of one or more biomarkers to determine the presence of pancreatic cancer precursor lesions or pancreatic cancer in a subject. This disclosure is based, at least in part, on the discovery that early to invasive stages of pancreatic cancer release or secrete biomarkers that can be detected in biological samples of a subject. Accordingly, in certain non-limiting embodiments, the present disclosure provides for methods and kits for determining the presence of one or more biomarkers in a biological sample of a subject, and methods of using such determinations in selecting a therapeutic regimen for a cancer subject and in methods of treating cancer subjects.

## Description

### PRIORITY CLAIM

This application claims priority to United States Provisional Application No. 61/837,358, filed June 20, 2013, the contents of which are incorporated by reference herein in its entirety.

### GRANT INFORMATION

This invention was made with government support under Grant Number R37GM36477 awarded by the National Institutes of Health. The government has certain rights in the invention.

### BACKGROUND

Pancreatic ductal adenocarcinoma (PDAC) carries a dismal prognosis, with less than a 5% survival rate (Hezel et al., 2006; Maitra and Hruban, 2008; Rustgi, 2006). Diagnosis can be difficult because there are no noticeable symptoms in early stages, and diagnosis is often determined when cancer has already disseminated to other organs. In addition, there is a scarcity of biomarkers for early stage detection of the disease, leading to PDAC usually detected at advanced stages, with limited therapeutic options. In combination with late detection, pancreatic cancer displays a poor response to chemotherapy, radiation therapy and surgery as conventionally used.

Many proteins secreted from pancreatic cancers (Harsha et al., 2009) that may serve as biomarkers have been identified in advanced, invasive PDAC or cell lines thereof, and thus may not represent markers for early stages of the disease. For example, a number of protein biomarkers have been identified in the sera and pancreatic juices from pancreatitis and pancreatic cancer patients. Serum biomarker protein CA-19.9 is presently used to monitor pancreatic cancer but is not useful in early diagnosis (Gattani et al., 1996). Therefore, markers have been sought for precancerous lesions, such as PanINs and intraductal papillary mucinous neoplasms (IPMNs) (Brat et al., 1998; Hruban et al., 2001), but the markers have typically been intracellular or cell surface proteins (Harsha et al., 2009) rather than secreted or released proteins that may provide an improved opportunity for diagnosis, especially in its earlier and potentially curable stages.

The prominent animal model of PDAC is based upon inducing a G12D mutant allele of *Kras* in the mouse pancreatic epithelium (Hingorani et al., 2003), a mutation that frequently occurs in human PDAC. The mice develop pancreatic intra-epithelial neoplasias (PanINs) with prolonged latency and incomplete penetrance of PDAC. PDAC and related tumors develop much more rapidly when *Kras*G12D/+ mice also contain mutations of *Ink4a*/*Arf*, *Tgfbr2, p53*, or *PTEN* (Morris et al., 2010), although these mutations alone do not efficiently cause PDAC. In an effort to develop human models of early pancreatic cancer, PDAC cells have been grafted into immunodeficient mice either as tumor fragments (Rubio-Viqueira et al., 2006), dispersed cells (Kim et al., 2009) or cells sorted for cancer stem cell markers (Hermann et al., 2007; Ishizawa et al., 2010; Li et al., 2007). In these contexts, tumors rapidly arise that resemble the advanced PDAC stages from which the cells were derived and do not undergo the slow growing, early PanIN stages of PDAC (Ding et al., 2010). However, there is a need for a live human cellular model that undergoes the early stages of PDAC and disease progression.

### SUMMARY

The present disclosure is based, at least in part, on the discovery that pancreatic cancer in its early to invasive stages releases or secretes biomarkers that can be detected in a biological sample of a subject. Accordingly, the disclosure provides methods for determining the presence of a biomarker indicative of pancreatic cancer in a biological sample of a subject. In certain embodiments, the detection of one or more biomarkers in a biological sample of a patient indicates the presence of early to invasive stages of pancreatic cancer. Thus, the methods of the disclosure also provide for early prognosis and diagnosis of cancer (*e.g*., identification of a biomarker prior to identification of a tumor by conventional means) and therapy monitoring in a subject.

In one aspect, the present disclosure provides methods of assessing whether a subject has pre-cancerous lesions and is at risk for developing advanced stage pancreatic cancer, including determining the presence of a biomarker in a biological sample obtained from the subject, wherein the presence of the biomarker is an indication that the patient is at increased risk for developing advanced stage cancer. In certain embodiments, the presence of a biomarker indicates early stage pancreatic cancer in the subject. In certain embodiments, the presence of a biomarker indicates advanced stage, invasive and/or metastatic pancreatic cancer in the subject. In certain embodiments, the method is carried out prior to the identification of a primary tumor in the subject.

In yet another aspect, the present disclosure provides methods of assessing the efficacy of a therapeutic or prophylactic therapy for preventing, inhibiting or treating pancreatic cancer, in a subject, including determining the presence and/or level of a biomarker in a biological sample obtained from the subject prior to therapy; and determining the presence and/or level of a biomarker in a biological sample obtained from the subject at one of more time points during therapeutic or prophylactic therapy, wherein the therapy is efficacious for preventing, inhibiting or treating cancer in the subject when there is a lower level of the biomarker in the second or subsequent samples, relative to the first sample.

In certain non-limiting embodiments, the biological sample can be a blood sample and/or a plasma sample. In certain embodiments, the biological sample can be a stool sample. In certain embodiments, the biological sample can be fluid drained from a pancreatic cyst. In other embodiments, the biological sample can be a tissue, *e.g.,* a tissue biopsy. In certain embodiments, one or more biomarkers can be detected in one or more biological samples from a subject. The use of stool, plasma and/or blood as a biological sample makes it possible to eliminate invasiveness of the diagnostic or prognostic procedure, and dramatically improve the burden of the examination on the subject.

In certain embodiments, the biomarker is a protein and the presence of the protein is detected using a reagent which specifically binds with the protein. For example, the reagent can be selected from the group consisting of an antibody, an antibody derivative, an antigen-binding antibody fragment and a non-antibody peptide which specifically binds the protein. In certain embodiments, the antibody or antigen-binding antibody fragment is a monoclonal antibody or antigen-binding fragment thereof, or a polyclonal antibody or antigen-binding fragment thereof. In certain embodiments, the protein biomarker can be detected by biophysical techniques such as mass spectrometry. In certain embodiments, the protein biomarker can be detected by enzyme-linked immunosorbent assay (ELISA).

The biomarker can also be a transcribed polynucleotide or portion thereof, *e.g.,* a mRNA or a cDNA. In certain embodiments, detecting a transcribed polynucleotide includes amplifying the transcribed polynucleotide. In certain embodiments, the nucleic acid biomarker can be detected by RNA in situ hybridization.

The disclosure also provides kits for monitoring, diagnosing or assessing whether a subject has pancreatic cancer, for monitoring the therapeutic treatment of a subject and for assessing the efficacy of a therapeutic treatment regime of a subject, where the kit contains reagents useful for detecting secreted or released biomarkers in a biological sample.

Aspects or embodiments of the invention may also be provided according to the following numbered paragraphs.
1. A method of determining whether a subject has pancreatic cancer, comprising obtaining one or more biological samples from the subject and detecting, in the one or more biological samples, one or more biomarkers selected from the group consisting of MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13, DES, IMMT, TPM1, SNRPE, VCAM1, GRB2, SHROOM3, HMOX1, POSTN, MMP10, MMP-2, THBS2, EWSR1, NOD1, ADAMTS9, AFP, SYNE1, SYNE2, EPHB1, UFD1L, TEAD1, RYR3, CMYA5, MYLK, TOP2B, KIAA1109, ODZ3, PMFBP1, EPHB3, LIMCH1, TCF20, ERP29, OBSCN, LOXL3, MLEC, DNAH1, DNAH5, DNAH12, DNAH17, SCYL2, FKBP10, FLRT3, ZHX2 (AFR1), ZNF804A, ACTN2 and combinations thereof, wherein the detection of the one or more biomarkers is an indication that the subject has pancreatic cancer.
2. A method of determining whether a subject has pancreatic cancer, comprising obtaining one or more biological samples from the subject and detecting, in the one or more biological samples, one or more biomarkers selected from the group consisting of RTTN, DNAH12, TPM1, DNAH1, STARD8, ATP2A1, TOP2B, LIMCH1, SYNE1, THBS2, LOXL3 and combinations thereof, wherein the presence of the one or more biomarkers is an indication that the subject has pancreatic cancer.
3. A method of determining whether a subject has pancreatic cancer, the method comprising obtaining a biological sample from the subject and determining if the biological sample from the subject contains at least three biomarkers, wherein one of the at least three biomarkers is a member of the TGFβ/integrin signaling pathway, one of the at least three biomarkers is a member of the HNF4α transcription factor network and the one of the at least three biomarkers is a member of the Ras/p53/JUN/CTNB1 signaling pathway, and wherein the presence of the at least three biomarkers in the sample is an indication that the subject has pancreatic cancer.
4. The method of paragraphs 1, 2 or 3, wherein the subject is human.
5. The method of paragraphs 1, 2 or 3, wherein the biological sample is selected from the group consisting of a stool sample, a blood sample, a plasma sample, a pancreatic cyst fluid sample and combinations thereof.
6. The method of paragraphs 1, 2 or 3, wherein the biomarker is a protein.
7. The method of paragraph 6, wherein the presence of the protein biomarker is detected using a reagent which specifically binds to the protein biomarker.
8. The method of paragraph 7, wherein the reagent is a monoclonal antibody or antigen-binding fragment thereof, or a polyclonal antibody or antigen-binding fragment thereof.
9. The method of paragraph 6, wherein the presence of the protein is detected by enzyme-linked immunosorbent assay.
10. The method of paragraphs 1, 2 or 3, wherein the biomarker comprises a transcribed polynucleotide or portion thereof.
11. A kit for diagnosing whether a subject has pancreatic cancer or assessing the efficacy of a therapeutic treatment, comprising reagents useful for detecting one or more biomarkers selected from the group consisting of MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13, DES, IMMT, TPM1, SNRPE, VCAM1, GRB2, SHROOM3, HMOX1, POSTN, MMP10, MMP-2, THBS2, EWSR1, NOD1, ADAMTS9, AFP, SYNE1, SYNE2, EPHB1, UFD1L, TEAD1, RYR3, CMYA5, MYLK, TOP2B, KIAA1109, ODZ3, PMFBP1, EPHB3, LIMCH1, TCF20, ERP29, OBSCN, LOXL3, MLEC, DNAH1, DNAH5, DNAH12, DNAH17, SCYL2, FKBP10, FLRT3, ZHX2 (AFR1), ZNF804A, ACTN2 and combinations thereof, in one or more biological samples from the subject.
12. The kit of paragraph 11, comprising one or more of packaged probe and primer sets, arrays/microarrays, biomarker-specific antibodies or beads for detecting the one or more biomarkers.
13. The kit of paragraph 11, comprising at least one monoclonal antibody or antigen-binding fragment thereof, or a polyclonal antibody or antigen-binding fragment thereof, for detecting the one or more biomarkers to be identified.
14. A method of determining whether a subject has pancreatic cancer comprising, obtaining a biological sample from the subject and determining if the biological sample from the subject contains at least two biomarkers, wherein one of the at least two biomarkers is a member of the TGFβ/integrin signaling pathway and the other biomarker of the at least two biomarkers is a member of the HNF4α transcription factor network, and wherein the presence of the at least two biomarkers in the sample is an indication that the subject has pancreatic cancer.
15. A method of determining whether a subject has pancreatic cancer, the method comprising obtaining a biological sample from the subject and determining if the biological sample from the subject contains at least two biomarkers, wherein one of the at least two biomarkers is a member of the Ras/p53/JUN/CTNB1 signaling pathway and the other biomarker of the at least two biomarkers is a member of the HNF4α transcription factor network, and wherein the presence of the at least two biomarkers in the sample is an indication that the subject has pancreatic cancer.
16. A method of determining whether a subject has pancreatic cancer, the method comprising obtaining a biological sample from the subject and determining if the biological sample from the subject contains at least two biomarkers, wherein one of the at least two biomarkers is a member of a pathway selected from the group consisting of the Ras/p53/JUN/CTNB1 signaling pathway, the HNF4α transcription factor network, the TGFβ/integrin signaling pathway and combinations thereof and the other biomarker of the at least two biomarkers is selected from the group consisting of MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13 and combinations thereof, wherein the presence of the at least two biomarkers in the sample is an indication that the subject has pancreatic cancer.
17. A method of assessing the efficacy of a therapy for preventing or treating pancreatic cancer in a subject, comprising:
   (a) determining the level of one or more biomarkers in a biological sample obtained from the subject, wherein the biomarkers are selected from the group consisting of MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13, DES, IMMT, TPM1, SNRPE, VCAM1, GRB2, SHROOM3, HMOX1, POSTN, MMP10, MMP-2, THBS2, EWSR1, NOD1, ADAMTS9, AFP, SYNE1, SYNE2, EPHB1, UFD1L, TEAD1, RYR3, CMYA5, MYLK, TOP2B, KIAA1109, ODZ3, PMFBP1, EPHB3, LIMCH1, TCF20, ERP29, OBSCN, LOXL3, MLEC, DNAH1, DNAH5, DNAH12, DNAH17, SCYL2, FKBP10, FLRT3, ZHX2 (AFR1), ZNF804A, ACTN2 and combinations thereof, prior to therapy; and
   (b) determining the level of the one or more biomarkers in a biological sample obtained from the subject, at one of more time points during therapy,
   wherein the therapy is efficacious for preventing or treating the cancer in the subject when there is a lower level of the one or more biomarkers in the second or subsequent samples, relative to the first sample.
18. A method for producing a pancreatic tumor cell model system comprising:
   (a) isolating pancreatic ductal adenocarcinoma cells from a pancreatic ductal adenocarcinoma sample;
   (b) overexpressing Klf4, Sox2, Oct4 and c-Myc in the isolated pancreatic ductal adenocarcinoma cells to produce induced pluripotent stem cells;
   (c) injecting the induced pluripotent stem cells into an immunocompromised animal to produce a teratoma in the animal;
   (d) isolating the teratoma from the animal; and
   (e) culturing the teratoma to obtain the pancreatic tumor cell model system.
19. A pancreatic tumor cell model system generated by the method of paragraph 18.
20. The use of the pancreatic tumor cell model system of paragraph 19 to identify one or more biomarkers for pancreatic cancer.

### BRIEF DESCRIPTION OF THE DRAWINGS

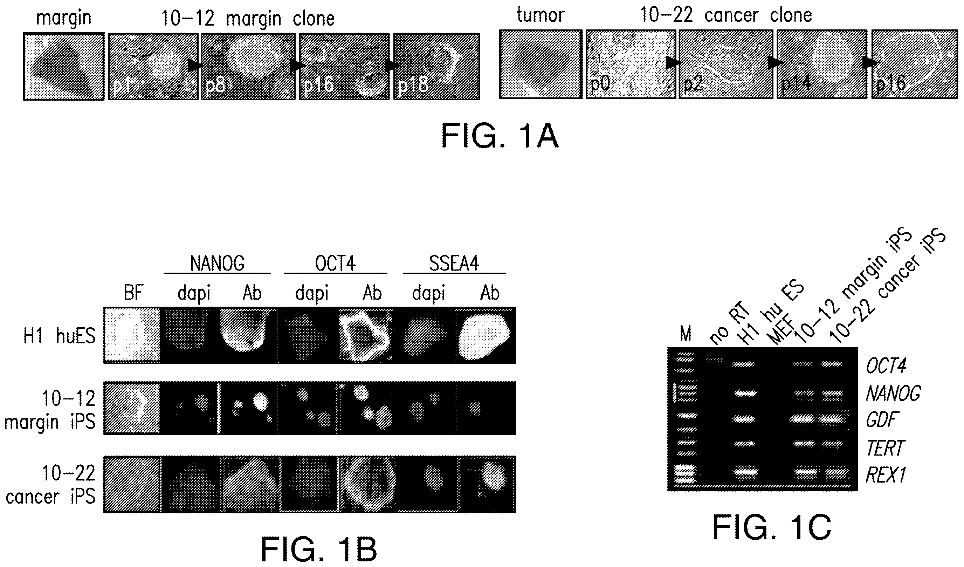

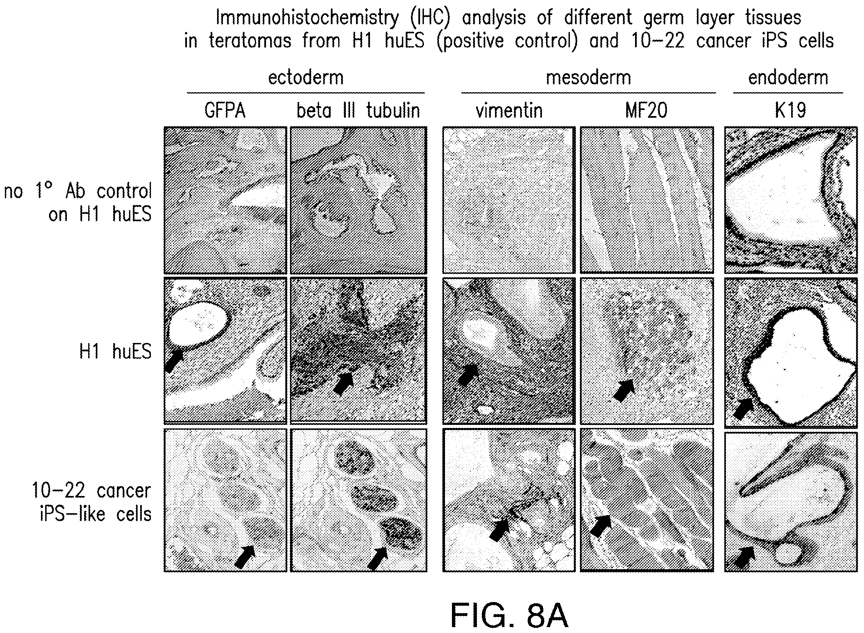

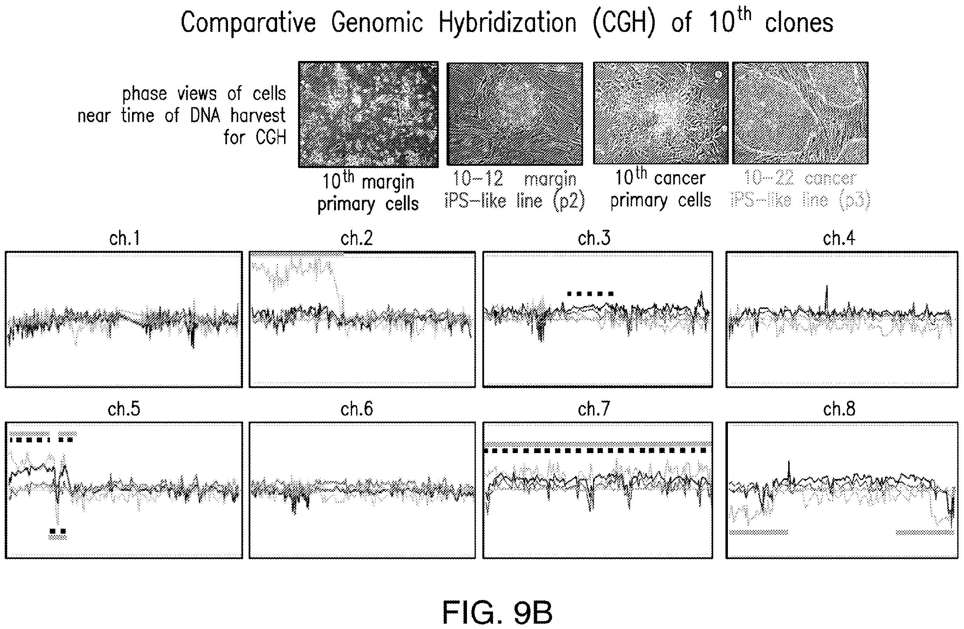

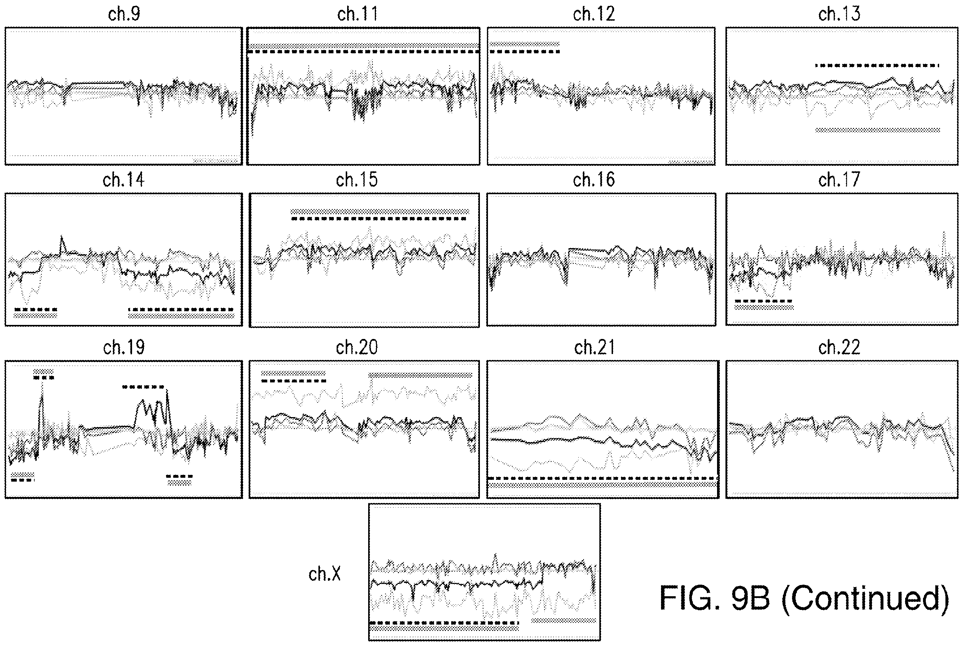

**Figure 1A-I.** Establishing iPS-like lines from patient-matched margin and pancreatic ductal adenocarcinoma. A. Cells from pancreatic cancer and margin tissues were reprogrammed and different passages of the 10-12 margin and 10-22 cancer iPS-like clones from patient #10 are shown. B, C. Expression of pluripotency markers in 10-12 margin and 10-22 cancer iPS-like lines by immunostaining (B) and RT-PCR (C). D. Three month teratomas in NSG mice from 10-12 margin and 10-22 cancer iPS-like lines showed differentiation into tissues of all three germ layer lineages (also see Figure 8A). E. Expression of differentiation markers for endoderm (*CXCR4*), mesoderm (*RUNX1*), and ectoderm (*SOX1, PAX6*) relative to *GAPDH* in embryoid bodies from H1 huES cells and 10-12 margin and 10-22 cancer iPS-like lines cultured for 12-14 days. Error bars, mean ± SD. F, G. DNA sequence tracks revealing *KRAS* mutation and karyotype showing subtetraploidy in the 10-22 cancer iPS-like line (F) and wild type *KRAS* and normal karyotype in the 10-12 margin iPS-like line (G). H. PCR analysis of *CDKN2A* (*p16Ink4a*) exon 2 reveals a heterozygous deletion in 10-22 cells. I. Comparative genomic hybridization showing normal profiles for the 10th primary margin cells (a) and the 10-12 margin iPS-like line (b). Gross chromosomal rearrangements are evident in the 10th primary cancer cell culture (c), which was mixed with normal stromal cells; the rearrangements are evident more clearly in the 10-22 cancer iPS-like line (d) (also see Figure 9B), demonstrating that the 10-22 line is clonally derived representative of the initial tumor epithelial cell population. The locations of *PTEN* and *DPC4 (SMAD4)* loci are shown in regions of chromosome loss in the 10th cancer and 10-22 cells.

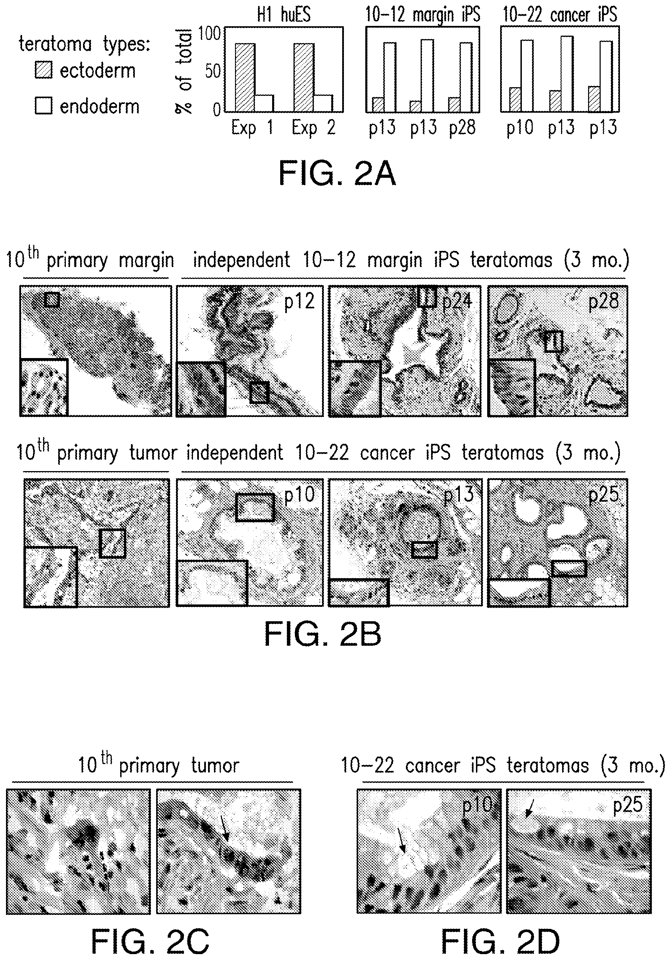

**Figure 2A-D.** The 10-22 iPS-like cells preferentially generate ductal structures in teratomas. A. Summaries of teratoma tissue types arising in 3 months in immunodeficient mice based on the number of histologic structures seen. Teratomas from huES cells are mostly ectodermal in appearance, whereas teratomas from 10-12 and 10-22 iPS-like lines are mostly endodermal/ductal. B. H&E staining of the margin and tumor tissue of patient #10 and of teratomas formed by 10-12 margin and 10-22 cancer iPS-like lines after 3 months. The latter lines create tubular, duct-like structures independent of passage number. Notably, the 10-22 cancer teratomas show a much a higher degree of differentiation than the primary tumor. C. The primary tumor shows an invasive phenotype (dotted line) and high nuclear to cytoplasmic ratio (arrow). D. 10-22 tumor iPS teratomas at 3 months show a low nuclear to cytoplasmic ratio, intracellular mucins (arrows), and a more differentiated phenotype.

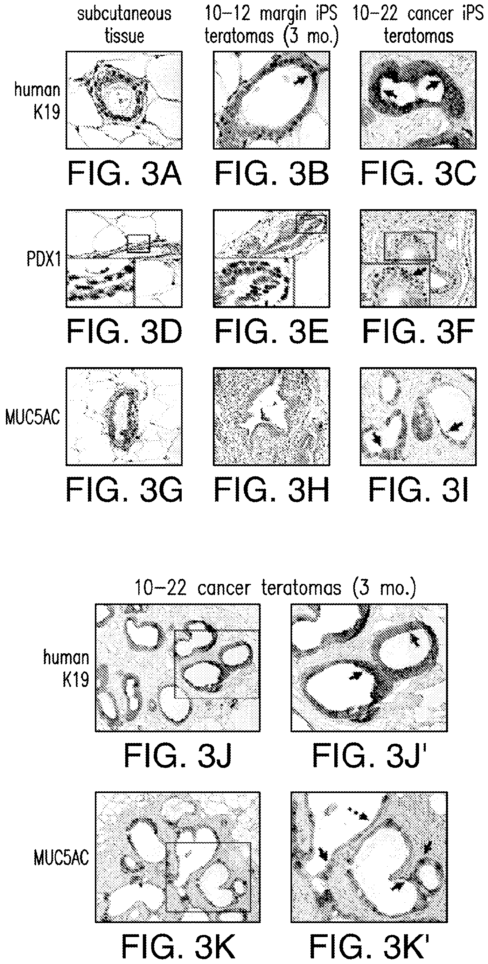

**Figure 3A****-K'.** Teratomas at three months from 10-22 cancer iPS-like cells exhibit PanIN-like structures and marker expression. A-C. No K19 staining in mouse subcutaneous tissue (negative control), weak K19 staining of 10-12 margin iPS-like teratomas at three months, and strong K19 staining of 10-22 cancer iPS-like teratomas at 3 months. D-I. Nuclear staining of PDX1 (arrow) and cytoplasmic staining of MUC5AC (arrow) only in teratomas of 10-22 cancer iPS-like teratomas at 3 months. J, K. Higher magnification shows uniform K19 staining (J, J', arrow) and heterogeneous MUC5AC staining (K, K') of teratomas from 10-22 cancer iPS-like cells.

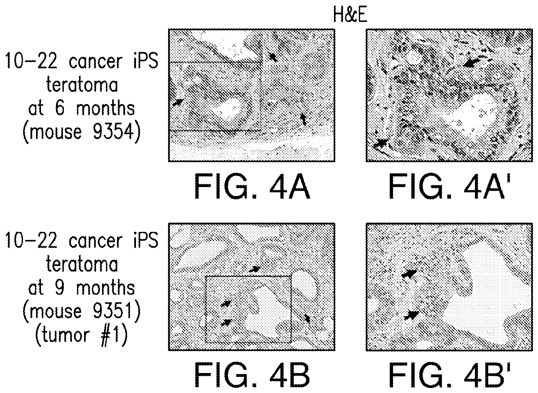

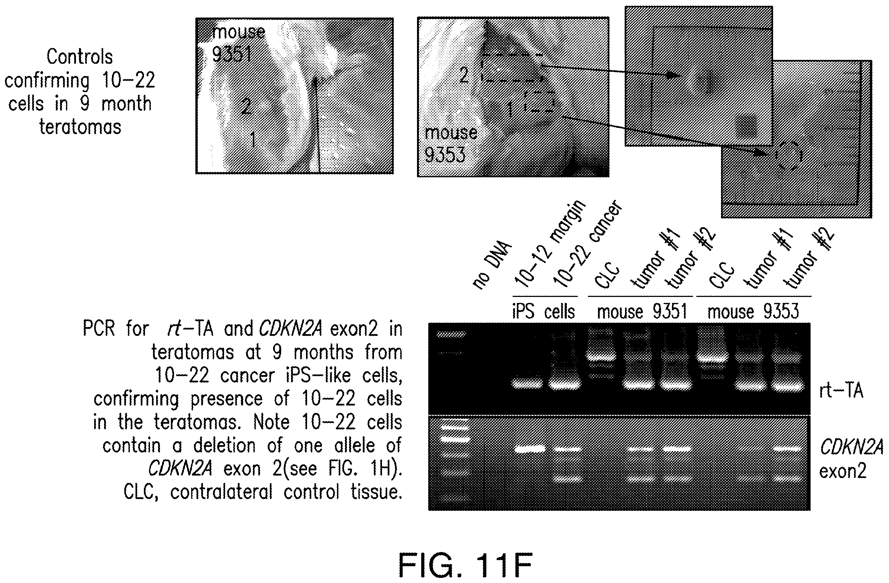

**Figure 4A-R.** Teratomas at 6 and 9 months from 10-22 cancer iPS-like cells exhibit invasive stages of pancreatic cancer. A-B, H&E staining of 10-22 teratomas in NSG mice at 6 months (A, A') and 9 months (B, B'). Arrows indicate epithelial cells with a more dysmorphic phenotype than at 3 months; nuclei are heterotypic and epithelia with hypochromic nuclei are invading the stroma (A, B, arrows). C-R immunohistochemistry showing epithelial cells positive for K19, MUC5AC, PDX1, and SOX9 in tumors arising at 6 and 9 months in NSG mice injected with 10 22 cells. See Figure 11F for genetic confirmation of 10-22 cells in the tumors.

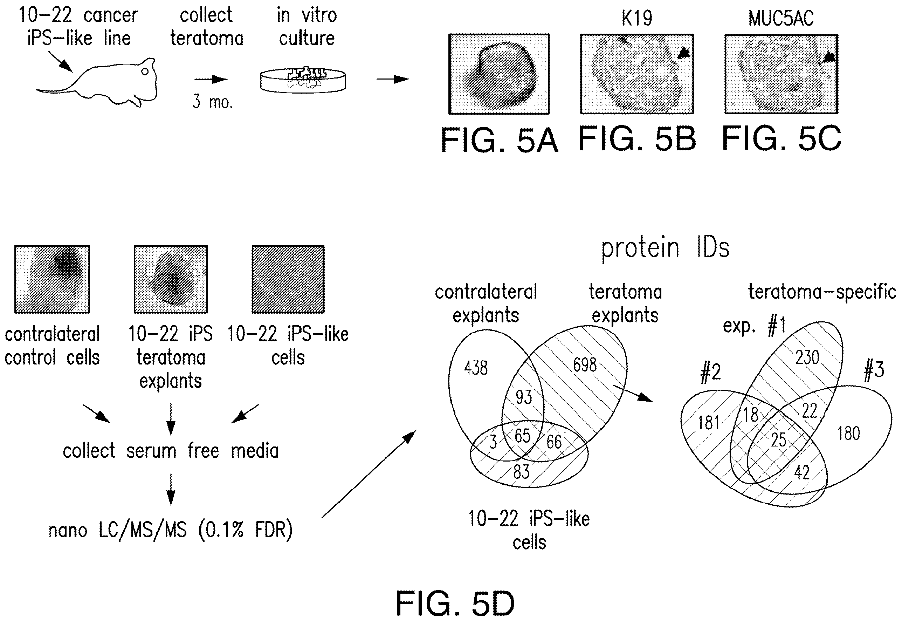

**Figure 5A-F.** Proteins secreted and HNF4 regulatory network within teratoma explants of 10-22 iPS-like cells at 3 months. Scheme for in vitro explants of three month teratomas from 10-22 cancer iPS-like lines. A-C. Whole mount view of teratoma explant from 10-22 iPS like cells (A), along with the explant sectioned and stained for K19 (B), and MUC5AC (C); arrows indicate positive domains. D. Proteomic analysis of conditioned media of 3 independent teratoma explants (#7761, 9223, and 9225) from 10-22 cancer iPS-like cells. Conditioned media was analyzed by nanoLC/MS/MS and protein candidates were identified by a Sequest search in the IPI DB. Using UniProKB TrEMBL (downloaded on Oct, 2011), peptides were included in the list that either consists of uniquely human sequences or sequences that are common to humans and mice. Mouse-specific sequences were excluded. Protein I.D.'s of conditioned media from teratoma explants were compared to those in conditioned media from explants of contralateral control tissue and of media from undifferentiated 10-22 ells (left Venn diagram). The total 698 secreted or released proteins specific to the teratomas are indicated in the three different experiments (right Venn diagram). Proteins secreted from at least two teratoma explants are summarized in Tables 4 and 5 and were used for pathway analysis. E. Numerous proteins fall into linked pathways for TGFβ and integrin signaling. Lines and arrows connecting molecules indicate direct interactions and dashed lines and arrows indicate indirect interactions. F. All proteins in bold are secreted or released specifically from the 10-22 teratoma explants and fall within a network controlled by the transcription factor HNF4α (Table 9). Asterisks denote proteins whose genes are directly bound by HNF4α.

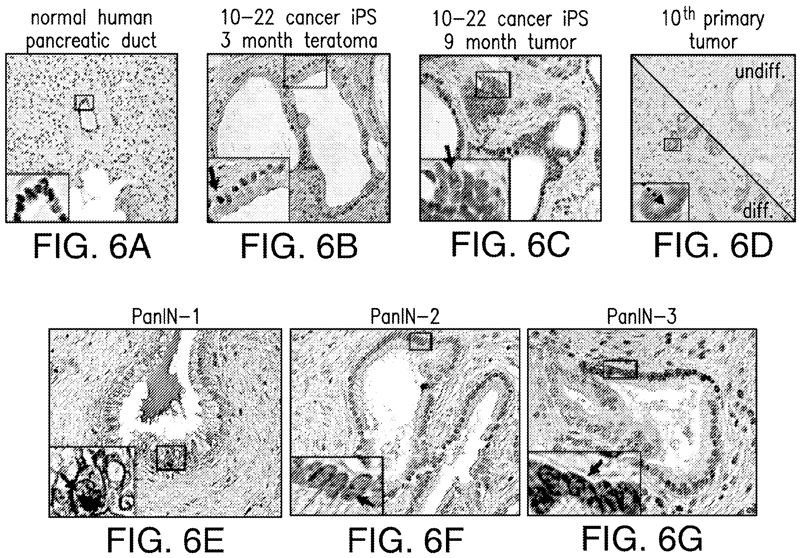

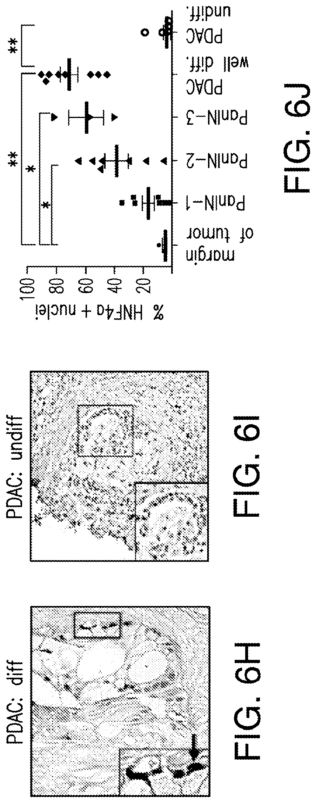

**Figure 6A****-P'.** Activation of HNF4α in PanIN cells and well differentiated early pancreatic cancers in human clinical samples and a mouse model of human PDAC. A-D. Immunohistochemistry for HNF4α showing the absence of staining in the main human pancreatic mass and ducts (A), strong nuclear staining in PanIN-like structures in a 3 month teratoma (B, arrow) and in invasive cells in a 9 month tumor (C, arrow) generated from NSG mice injected with 10-22 cells, and mostly cytoplasmic staining in small, moderately differentiated ducts (D, image section below the diagonal, brown, dashed arrow) and an absence of staining in undifferentiated ducts (D, image section above the diagonal) of the 10th patient's pancreatic tumor epithelium E-I. HNF4α is weakly labeled in human PanIN-1 cells (E, arrow, N=8), strongly in PanIN2 (F, arrow, N=7), PanIN3 (G, arrow, N=3), and mucinous well differentiated human PDAC (H, arrow, N=8) but decreased or not in undifferentiated/or poorly differentiated human PDAC (I, N=8). J, Percentage of HNF4α positive nuclei to total epithelial nuclei counted (See Table 14) in multiple PanIN and PDAC samples on human tissue microarrays. Error bars, SEM. The differences in HNF4α expression is statistically significant between margin and PanIN-2 and -3 stages (* P<0.05); margin and well-differentiated PDAC (** P=5.46E-06); well differentiated PDAC and poorly/undifferentiated PDAC (**P=3.05E-06), as per two-tailed Student's t-tests. K-P. In a mouse model of PDAC, HNF4α is expressed weakly at the PanIN1 (K, K'), strongly at the PanIN2-3 stages (L, L', M, M') and in differentiated portions of tumors (PDAC) (O, O'), and weakly or not expressed in undifferentiated portions of the same tumor (P, P').

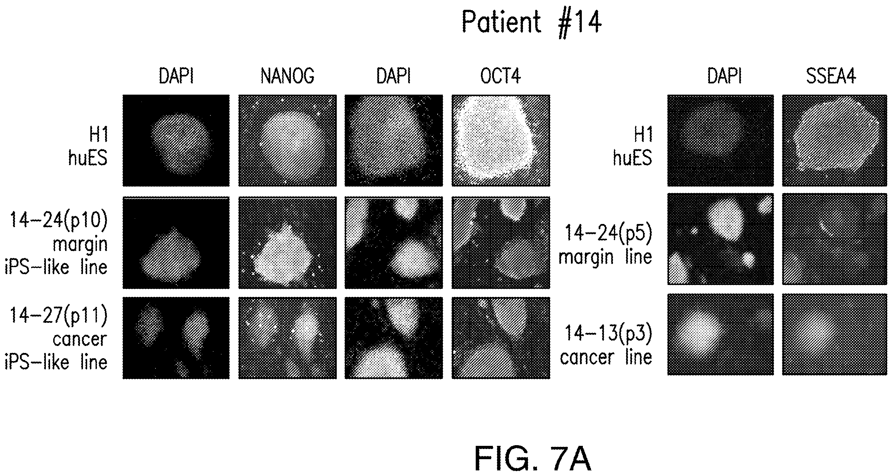

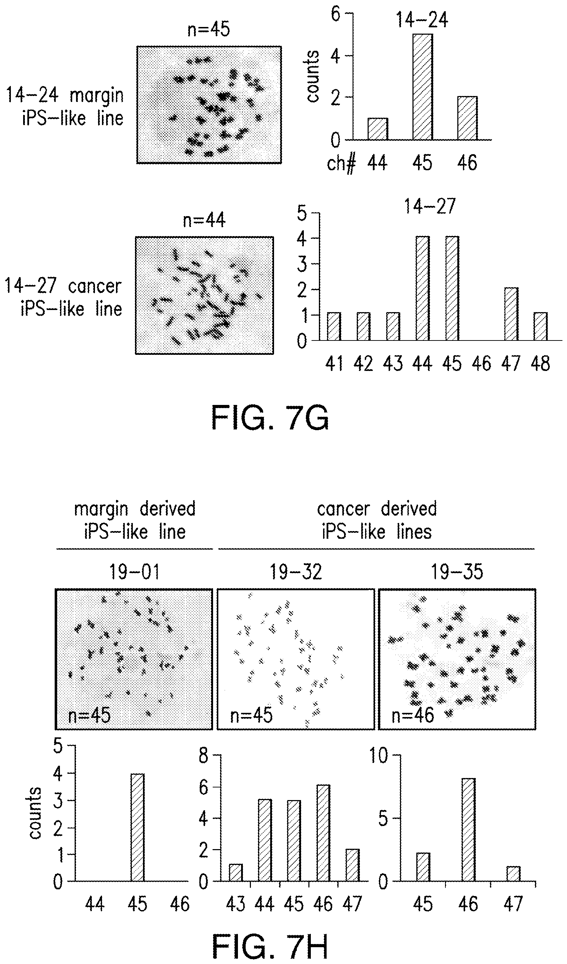

**Figure 7A-H.** A-B. Generating iPS-like lines from patient #14. Pancreatic margin and cancer iPS-like lines were generated from patient #14 and characterized by immunostaining for NANOG, OCT4, SSEA4 (A) and qRT-PCR for pluripotency genes (B). C-D. Pancreatic margin and cancer iPS-like lines were generated from patient #19, characterized by immunostaining for OCT4, NANOG, and TRA-1-60 (C), RT-PCR for pluripotency (D). E. Embryoid bodies were generated from 14-24 margin iPS-like line for 15 days and checked for the expression of differentiation markers using qRT-PCR. Expression levels are relative to *Gapdh.* Error bars are the mean ± SD (E). F. Teratoma assays showed the differentiation of 14-24 margin and 14-27 cancer derived iPS-like lines into multilineage cells; but note that in limited experiments with the 14-27 line, neural derivatives were not detected. G-H, Karyotype analysis showed that 14-24 margin and 14-27 cancer iPS-like lines had subdiploidy (G) and 19th lines showed diploidy (H).
**Figure 8A-C.** A. Immunohistochemistry (IHC) analysis of different germ layer tissues on 10-22 cancer iPS teratoma tissue after 3 months. H1 huES teratoma tissue was used as a positive control. Glial Fibrillary Acidic protein (GFAP) and beta III tubulin were used for ectoderm, vimentin and MF20 were for mesoderm, and K19 was used for endoderm. B. Immunohistochemical staining for NANOG (upper panels) and OCT4 (lower panels), with the H1 huES line as a positive control and the primary tumor #10 experimental sample, analyzed at the same time. The data indicate that NANOG is not expressed in the primary #10 tumor and that OCT4 is expressed weakly in sporadic cells. The absence of NANOG expression is consistent with the hypothesis that the primary tumor #10 was not composed largely of pancreatic cancer stem cells (Lonardo et al. 2011). C. Analysis of the CpG methylation state of 10-12 margin and 10-22 cancer iPS-like lines at the designated CpG sites (ovals, below position with respect to the transcription start) in the 5' upstream of the human NANOG and OCT4 genes by bisulfite pyrosequencing. Genomic DNA from H1 huES cells was used for positive control and genomic DNA from parental primary tumor of patient #10 was used for a negative control. The schematic diagram of upstream region of NANOG and OCT4 examined in this study. The regions amplified by PCR are enlarged. The graphs show the percent methylated C's in the designated CpG positions. At NANOG, all 9 CpG sites tested were methylated in the parental #10 tumor DNA and demethylated in the 10-22 iPS-like line, comparable to that seen in the H1 human ES control cells. At OCT4, C's at positions -497 and -532 exhibited marked demethylation in the 10-22 iPS-like line, while at position -161 there was modest demethylation. The H1 control was not markedly demethylated at the -497 and -532 sites, but was at other sites tested. Methylation of the NANOG locus provides further evidence that the primary tumor #10 was not composed of pancreatic cancer stem cells.

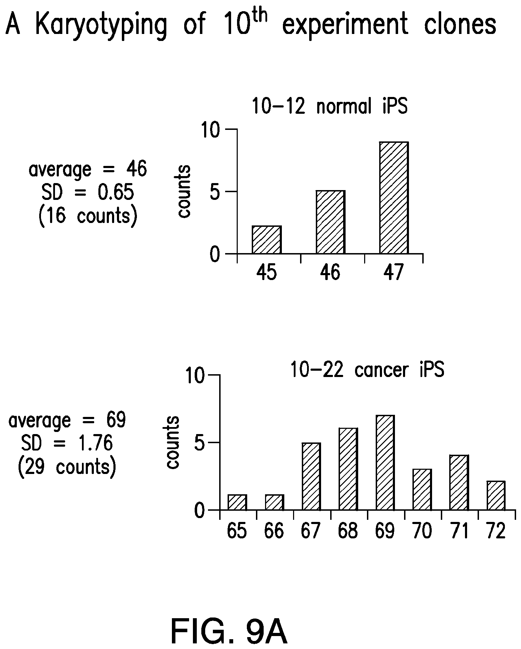

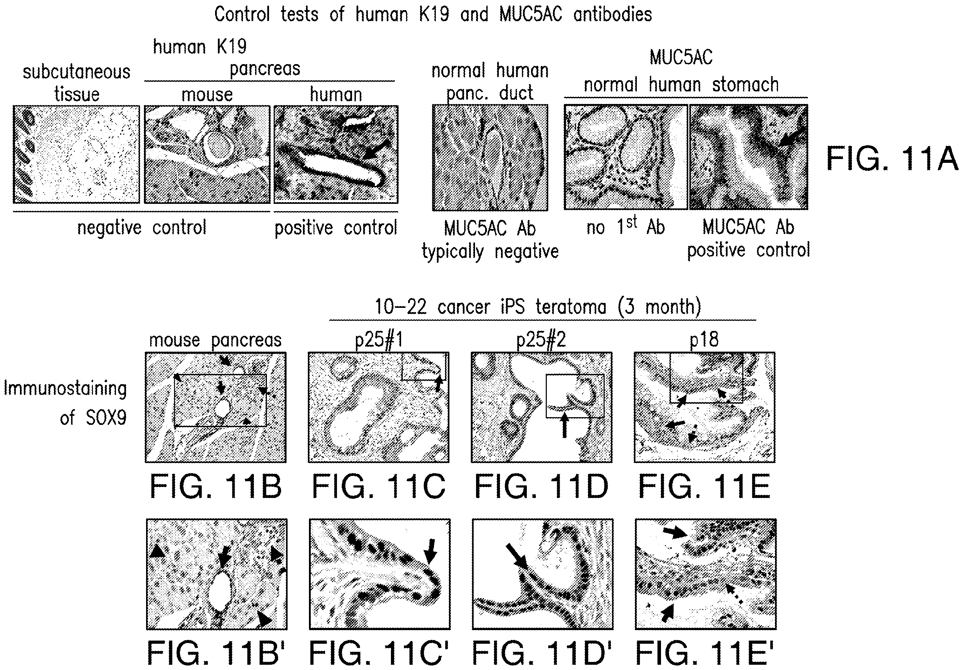

**Figure 9A-B.** A. Karyotype analysis of 10-12 margin and 10-22 cancer iPS-like clones. B. Comparative genomic hybridization assay of 10-12 margin, 10-22 cancer iPS-like clones. Of the 23 chromosomal aberrations seen in the epithelial cell cultures of the 10th primary cancer, 20 aberrations were present in the 10-22 cancer iPS-like line, as seen by the green and red double bars. Dashed and solid bars denote aberrations unique to the primary cancer or cell line, respectively. See Fig. 11 for ch10 and ch18. Note that the chromosomal aberrations seen in the 10th primary cancer cultures (solid CGH traces), which were composed mainly of the cancer epithelial cells but also contained stromal cells, are amplified in the 10-22 iPS line (dashed CGH traces), a clone from the cancer culture.

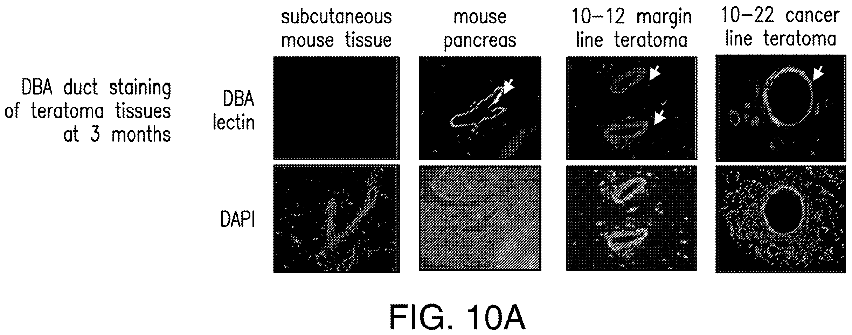

**Figure 10A-I.** A. 10-12 margin and 10-22 cancer teratoma ductal structures at three months expressed DBA lectin. Contralateral control subcutaneous fat tissue was used as a negative control and mouse pancreas was used as a positive control. B-G. The PanIN-like structures derived from teratomas from the 10-22 cancer iPS-like line at three months in independent NSG mice. Compared to the poorly differentiated structures in the parental primary tumor of patient #10 (B), the 10-22 cancer iPS-like lines generated PanIN stages in independent mice regardless passage number of initial 10-22 cancer iPS-like line (C-G). H. PCR for rt-TA in LCM-dissected 10-22 cancer iPS teratoma tissue at three months. PCR for rt-TA on laser capture microdissected PanIN-like duct, stroma, and mouse duct. All epithelial cells were removed from the region prior to removing stromal cells, to avoid possible cross-contamination. I. Immunostaining of rt-TA on PanIN-like epithelial and stroma cells derived from 10-22 cancer iPS teratoma tissue at three months. The fibroblast distal region was negative for rt-TA. The data show that at least part of the stroma surrounding the PanIN-like epithelium in the teratoma is derived from the 10-22 human iPS-like cell line.
**Figure 11A-F.** A. Validation of antibodies against K19 and MUC5AC. Species-specificity of anti-human K19 was tested on mouse subcutaneous tissue, mouse and human pancreatic tissue at 1:2000 dilution ratios. The specificity of the MUC5AC antibody was tested in normal human pancreas and normal human stomach. B-E Immunostaining of SOX9 in PanIN-like ducts of 10-22 cancer iPS teratoma arising at 3 months in three different NSG mice. As a positive control, mouse pancreas was stained with SOX9. Subsets of duct (arrows) and centroacinar cells (arrowheads) in mouse pancreas moderately expressed SOX9 (B-B', brown). Islet cells didn't express SOX9 (B-B', dashed arrows). PanIN-like ducts expressed SOX9 (brown, arrows, C-E). F. Human origin of 9 month 10-22 teratoma arose from NSG mice injected with 10-22 cancer iPS-like lines was confirmed by PCR for rt-TA and *CDKN2A*. Four tumors derived from two different mice preserved rt-TA sequence and deletions of *CDKN2A*. DNA from contralateral control (CLC) tissue was used for negative control.

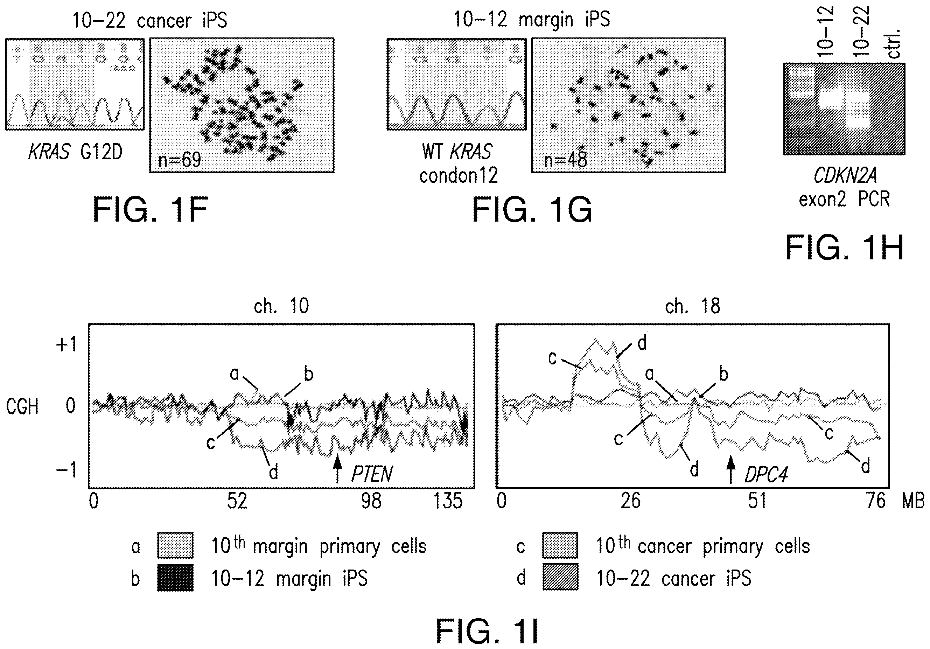

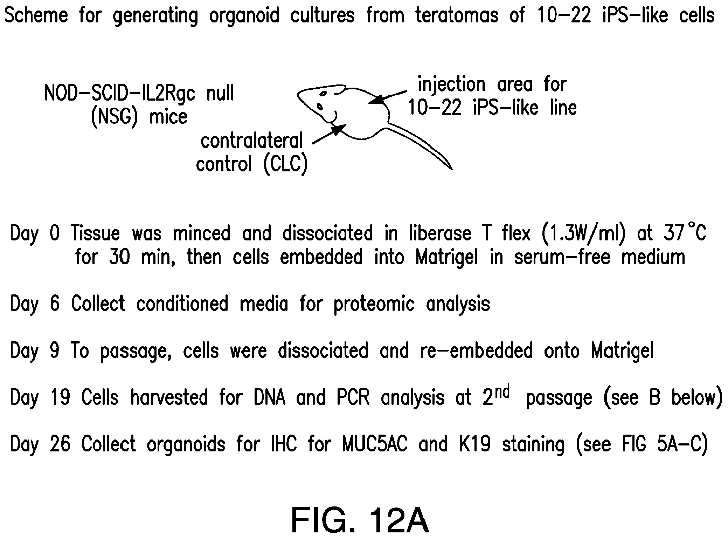

**Figure 12A-D.** A. Scheme for in vitro explants of three month teratomas from 10-22 cancer iPS-like lines. Three independent teratoma tissues were explanted for in vitro culture (#7761, 9223, and 9225). Contralateral control tissue and 10-22 teratoma tissue generated spheres. B. Human origin of 10-22 teratoma in vitro explants was confirmed by RT-PCR for rt-TA and *CDKN2A*, and sequencing for the *KRAS* G12D mutation in the 10-22 iPS-like cells (see Fig. 1F-H). C. Control tests of HNF4α immunostaining on mouse liver tissue and normal pancreas. HNF4α was expressed only in islets and not in acinar or ductal cells in the mouse normal pancreas. Solid arrow indicates the positive cells and dashed arrow indicates the negative cells. D. HNF4α staining on additional 10-22 cancer iPS teratoma tissue derived from independent mice at 3 months. Solid arrow indicates the positive cells.
**Figure 13****.** Statistical analysis of the biomarker TCHP in control and pancreatic cancer samples.
**Figure 14****.** Statistical analysis of the biomarker ABCA13 in control and pancreatic cancer samples.
**Figure 15****.** Statistical analysis of the biomarker STARD8 in control and pancreatic cancer samples.
**Figure 16****.** Statistical analysis of the biomarker ATP2A1 in control and pancreatic cancer samples.
**Figure 17****.** Statistical analysis of the biomarker FKBP10 in control and pancreatic cancer samples.
**Figure 18****.** Statistical analysis of the biomarker SCN8A in control and pancreatic cancer samples.
**Figure 19****.** Statistical analysis of the biomarker TCF20 in control and pancreatic cancer samples.
**Figure 20****.** Statistical analysis of the biomarker SYNE1 in control and pancreatic cancer samples.
**Figure 21****.** Statistical analysis of the biomarker UFD1L in control and pancreatic cancer samples.
**Figure 22****.** Statistical analysis of the biomarker FLRT3 in control and pancreatic cancer samples.
**Figure 23****.** Statistical analysis of the biomarker TOP2B in control and pancreatic cancer samples.
**Figure 24****.** Statistical analysis of the biomarker ZHX2 in control and pancreatic cancer samples.
**Figure 25****.** Statistical analysis of the biomarker LIMCH1 in control and pancreatic cancer samples.
**Figure 26****.** Statistical analysis of the biomarker THBS2 in control and pancreatic cancer samples.
**Figure 27****.** Statistical analysis of the biomarker SHROOM3 in control and pancreatic cancer samples.
**Figure 28****.** Statistical analysis of the biomarker HMOX1 in control and pancreatic cancer samples.
**Figure 29****.** Statistical analysis of the biomarker LOXL3 in control and pancreatic cancer samples.
**Figure 30****.** Statistical analysis of the biomarker OBSCURIN in control and pancreatic cancer samples.
**Figure 31****.** Statistical analysis of the biomarker MALECTIN in control and pancreatic cancer samples.
**Figure 32****.** Statistical analysis of the biomarker DNAH5 in control and pancreatic cancer samples.
**Figure 33****.** Statistical analysis of the biomarker CA19-9 in control and pancreatic cancer samples.
**Figure 34****.** Statistical analysis of the biomarker AFP in control and pancreatic cancer samples.
**Figure 35****.** Statistical analysis of the biomarker RTTN in control and pancreatic cancer samples.
**Figure 36****.** Statistical analysis of the biomarker NLRX1 in control and pancreatic cancer samples.
**Figure 37****.** Statistical analysis of the biomarker DNAH12 in control and pancreatic cancer samples.
**Figure 38****.** Statistical analysis of the biomarker ODZ3 in control and pancreatic cancer samples.
**Figure 39****.** Statistical analysis of the biomarker ADAMST9 in control and pancreatic cancer samples.
**Figure 40****.** Statistical analysis of the biomarker TPM1 in control and pancreatic cancer samples.
**Figure 41****.** Statistical analysis of the biomarker DNAH1 in control and pancreatic cancer samples.
**Figure 42****.** Statistical analysis of the biomarker PMBP1 in control and pancreatic cancer samples.
**Figure 43****.** Statistical analysis of the biomarker DNAH17 in control and pancreatic cancer samples.
**Figure 44****.** Statistical analysis of the biomarker EPHB1 in control and pancreatic cancer samples.
**Figure 45****.** Statistical analysis of the biomarker DOS in control and pancreatic cancer samples.
**Figure 46****.** Statistical analysis of the biomarker MMP2 in control and pancreatic cancer samples.

### DETAILED DESCRIPTION

The present disclosure relates to the use of one or more biomarkers identified herein to detect the presence of pancreatic cancer in a biological sample from a subject. It is based, at least in part, on the discovery that iPS cells created from a human pancreatic ductal adenocarcinoma (PDAC) sample provided a live cell human model for studying early stages of pancreatic cancer. Furthermore, this disclosure is based, at least in part, on the discovery that early to invasive stages of pancreatic cancer released or secreted specific proteins that are detectable in biological samples, *e.g*., blood, of a subject.

Accordingly, the disclosure provides for methods and kits for determining the presence of one or more biomarkers for pancreatic cancer in a biological sample of a subject, and methods of using the presence or level of such biomarkers to predict or diagnose pancreatic cancer in a subject, select a therapeutic regimen for a subject suffering from pancreatic cancer, and treat a subject suffering from pancreatic cancer, wherein the presence of one or more biomarkers in a biological sample (*e.g*., 1, 2, 3, 4, 5, 6, 7, 8, 9, 10, 11, 12, 13, 14, 15, 16, 17, 18, 19, 20, 21, 25, 30 or more), or another defined minimum number depending on the subject, indicates the presence of pancreatic cancer in the subject. The biomarkers that can be used in the methods of the present disclosure are set forth in Tables 4, 5, 6, 7, 8, 9, 10, 11, 12, 13 and 15. In certain embodiments, the biomarkers that can be used in the methods of the present disclosure include RTTN, DNAH12, TPM1, DNAH1, STARD8, ATP2A1, TOP2B, LIMCH1, SYNE1, THBS2 and LOXL3.

Based on the identification of a secreted or released biomarker of the present disclosure in a biological sample of a subject, a diagnosis of pancreatic cancer in the subject can be made, even prior to the development, or identification of, tumor formation, thus allowing for prophylactic therapy in the subject. For example, further or more frequent monitoring, biopsy, surgical resection or other prophylactic measures to prevent tumor formation or identify cancer at a very early stage can be carried out based on the detection of one or more biomarkers in a biological sample.

Furthermore, the effectiveness of cancer therapy can be monitored by evaluating the presence and/or levels of the one or more biomarkers over the course of therapy, and decisions can be made regarding the type, duration and course of therapy based on these evaluations.

In certain embodiments, the subject being tested for the presence of a biomarker in a biological sample, as described herein, can be a subject who is at high risk for developing pancreatic cancer. In certain embodiments, a subject is at high risk for development of pancreatic cancer based on, for example, family history or determination of genetic predisposition. For example, these findings have implications for the management of individuals at high risk for pancreatic cancer, including subjects with kindreds with inherited pancreatic cancer. Based on the identification that the biomarkers are secreted or released from early stage pancreatic cancer, a window of opportunity exists for prophylactic therapy in high-risk subjects in the time-period prior to detection of late-stage, invasive pancreatic cancer.

As exemplified herein, the present disclosure can be used to diagnose pancreatic ductal adenocarcinoma (PDAC), as the vast majority of patients with pancreatic cancer have metastatic disease at the time of diagnosis using current methods. More than 75% of patients who undergo surgical resection of small pancreatic tumors with clear surgical margins and no evidence of metastasis die from metastatic disease within 5 years (Neoptolemos et al., 2004), a finding that is consistent with early spread. Moreover, metastatic PDAC has been documented in a cohort of patients who underwent pancreatectomy for chronic pancreatitis and in whom histologic analysis of the resected pancreas revealed only PanIN lesions (Sakorafas and Sarr, 2003). Accordingly, diagnosis and treatment at a very early stage is important.

### Definitions

As used herein, the use of the word "a" or "an" when used in conjunction with the term "comprising," "comprise," "includes" or "including" in the claims and/or the specification can mean "one," but it is also consistent with the meaning of "one or more," "at least one," and "one or more than one." Certain embodiments of the present disclosure can consist of or consist essentially of one or more elements, method steps and/or methods of the invention. It is contemplated that any method or composition described herein can be implemented with respect to any other method or composition described herein.

As used herein, the term "biomarker" refers to a marker (*e.g*., an expressed gene, including mRNA and/or protein) that allows detection of a disease in an individual, including detection of disease in its early stages. Biomarkers, as used herein, include nucleic acid and/or protein markers, set forth in Tables 4, 5, 6, 7, 8, 9, 10, 11, 12, 13 and 15 or combinations thereof. In certain non-limiting embodiments, a biomarker is a released and/or secreted protein that can be detected in a biological sample of a subject. In certain embodiments, the expression level of a biomarker as determined by mRNA and/or protein levels in tissue or biological sample from an individual to be tested is compared with respective levels in normal tissue or biological sample from the same individual or another healthy individual. In certain embodiments, the presence of a biomarker as determined by mRNA and/or protein levels in a tissue or biological sample from an individual to be tested is compared with the respective presence or absence in normal tissue or biological sample from the same individual or another healthy individual. In certain embodiments, the presence of a biomarker as determined by mRNA and/or protein levels in a tissue or biological sample from an individual indicates that the individual has pancreatic cancer or is at an increased risk for developing late-stage pancreatic cancer.

As used herein, the term "biological sample" refers to a sample of biological material obtained from a subject, *e.g.,* a human subject, including tissue, a tissue sample, a cell sample, a tumor sample, a stool sample and a biological fluid, *e.g*., plasma, serum, blood, urine, lymphatic fluid, ascites, pancreatic cyst fluid and a nipple aspirate. In certain embodiments, the presence of one or more biomarkers is determined in a peripheral blood sample obtained from a subject. In certain embodiments, the presence of one or more biomarkers is detected in a stool sample obtained from a subject. In certain embodiments, the presence of one or more biomarkers is detected in pancreatic cyst fluid obtained from a subject. In certain embodiments, the presence of one or more biomarkers is detected in one or more plasma samples obtained from a subject.

The term "patient" or "subject," as used interchangeably herein, refers to any warm-blooded animal, *e.g*., a human. Non-limiting examples of non-human subjects include non-human primates, dogs, cats, mice, rats, guinea pigs, rabbits, fowl, pigs, horses, cows, goats, sheep, etc.

The term "pancreatic cancer" as described herein refers to any type of cancerous or precancerous tissues arising from normal tissues of the pancreas, including, but not limited to, PanIN lesions, pancreatic ductal adenocarcinoma or pancreatic adenocarcinoma. Other types of pancreatic tumors include acinar-cell carcinoma, serous cystadenoma and pancreatic endocrine tumors. In certain embodiments, the biomarkers of the present diclosure can be used to detect cancers such as biliary cancer and liver cancer.

As used herein "resectable cancer" refers to a subset of cancers that are at an early stage and can be surgically excised. For example and not by way of limitation, stages IA, IB and IIA of pancreatic cancer are typically resectable.

The term "early stage cancer" as used herein refers to cancer prior to metastasis and/or organ extravasion. For example and not by way of limitation, with respect to pancreatic cancer, early stage cancer can include stages IA, IB and IIA.

### Prognostic and Diagnostic Methods

Embodiments of the present disclosure relate to methods for diagnosing pancreatic cancer in a subject. In certain embodiments, a method for diagnosing prostate cancer in a subject is disclosed, where the method includes: obtaining a biological sample from the subject; determining the presence of one or more biomarkers in the biological sample; and diagnosing pancreatic cancer in the subject, wherein the presence of the one or more biomarkers correlates to a positive diagnosis of pancreatic cancer in the subject. The biomarkers that can be used in the methods of the present disclosure are set forth in Tables 4, 5, 6, 7, 8, 9, 10, 11, 12, 13 and 15.

In certain embodiments, a method for diagnosing pancreatic cancer in the subject includes obtaining at least one biological sample from the subject. In certain embodiments, the one or more biomarkers can be detected in blood (including plasma or serum) or in feces (*e.g*., a stool sample), or alternatively at least one biomarker can detected in one sample, *e.g*., the blood, plasma or serum, and at least one other biomarker is detected in another sample, *e.g*., in feces. In certain embodiments, the one or more biomarkers are detected in tissue samples. For example, the biological sample can be a tumor biopsy. In certain embodiments, the one or more biomarkers are detected in pancreatic cyst fluid. The step of collecting a biological sample can be carried out either directly or indirectly by any suitable technique. For example, a blood sample from a subject can be carried out by phlebotomy or any other suitable technique, with the blood sample processed further to provide a serum sample or other suitable blood fraction, *e.g*., plasma, for use in the methods of the presently disclosed subject matter.

In certain embodiments, the methods for detection of one or more biomarkers can be used to monitor the response in a subject to prophylactic or therapeutic treatment (for example, preventative cancer treatment or treatment of diagnosed cancer). In certain embodiments, the present disclosure further provides a method of treatment including measuring the presence of one or more biomarkers in a subject at a first timepoint, administering a therapeutic agent, re-measuring the one or more biomarkers at a second timepoint, comparing the results of the first and second measurements and optionally modifying the treatment regimen based on the comparison. In certain embodiments, the one or more biomarkers are selected from MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13, DES, IMMT, TPM1, SNRPE, VCAM1, GRB2, SHROOM3, HMOX1, POSTN, MMP10, MMP-2, THBS2, EWSR1, NOD1, ADAMTS9, AFP, SYNE1, SYNE2, EPHB1, UFD1L, TEAD1, RYR3, CMYA5, MYLK, TOP2B, KIAA1109, ODZ3, PMFBP1, EPHB3, LIMCH1, TCF20, ERP29, OBSCN, LOXL3, MLEC, DNAH1, DNAH5, DNAH12, DNAH17, SCYL2, FKBP10, FLRT3, ZHX2 (AFR1), ZNF804A, ACTN2 or a combination thereof. In certain embodiments, the one or more biomarkers are selected from RTTN, DNAH12, TPM1, DNAH1, STARD8, ATP2A1, TOP2B, LIMCH1, SYNE1, THBS2, LOXL3 or a combination thereof.

In certain embodiments, the first timepoint is prior to an administration of the therapeutic agent, and the second timepoint is after said administration of the therapeutic agent. In certain embodiments, the first timepoint is prior to the administration of the therapeutic agent to the subject for the first time. In certain embodiments, the dose (defined as the quantity of therapeutic agent administered at any one administration) is increased or decreased in response to the comparison. In certain embodiments, the dosing interval (defined as the time between successive administrations) is increased or decreased in response to the comparison, including total discontinuation of treatment.

In certain embodiments of the present disclosure, the method of diagnosing, prognosing or screening for pancreatic cancer in a subject includes, (a) obtaining a biological sample from the subject; (b) determining the level of one or more biomarkers in a biological sample of the subject selected from Tables 4, 5, 6, 7, 8, 9, 10, 11, 12, 13, 15 or a combination thereof; and (c) comparing the level of the one or more biomarkers to a reference sample, wherein an increase in the level of the one or more biomarkers indicates the presence of pancreatic cancer in the subject. In certain embodiments, the reference sample can be obtained, for example, from a normal biological sample of the subject, *e.g*., adjacent benign tissue, or from subjects that do not have pancreatic cancer.

In certain embodiments of the present disclosure, the method of diagnosing, prognosing or screening for pancreatic cancer in a subject includes (a) obtaining a biological sample from the subject; (b) determining the presence and/or level of one or more biomarkers in a biological sample of the subject selected from Tables 4, 5, 6, 7, 8, 9, 10, 11, 12, 13, 15 or a combination thereof, wherein the detection of the one or more biomarkers indicates the presence of pancreatic cancer in the subject.

In certain embodiments of the present disclosure, the method of diagnosing a subject with pancreatic cancer includes determining the presence of one or more biomarkers in a biological sample from the subject, wherein the one or more biomarkers include one or more biomarkers of the TGFβ/integrin signaling pathway. In certain embodiments, the one or more TGFβ/integrin signaling pathway biomarkers include, but are not limited to, DES, IMMT, TPM1, SNRPE, VCAM1, GRB2, SHROOM3, HMOX1, POSTN, MMP10, MMP-2, THBS2, EWSR1, NOD1, ADAMTS9, AFP, SYNE1, SYNE2, EPHB1, UFD1L, TEAD1, RYR3, CMYA5, MYLK, TOP2B or a combination thereof. In certain embodiments, the one or more TGFβ/integrin signaling pathway biomarkers are SYNE1, THBS2, TOP2B, TPM1 or a combination thereof.

In certain embodiments of the present disclosure, the method of diagnosing a subject with pancreatic cancer includes determining the presence of one or more biomarkers in a biological sample from the subject, wherein the one or more biomarkers include one or more biomarkers of the HNF4α transcription network pathway. In certain embodiments, the one or more HNF4α transcription network pathway biomarkers include, but are not limited to, OBSCN, LOXL3, MLEC, DNAH1, DNAH5, DNAH12, DNAH17, SCYL2, FKBP10, FLRT3, ZHX2(AFR1), ZNF804A, ACTN2 or a combination thereof. In certain embodiments, the one or more HNF4α transcription network pathway biomarkers can be LOXL3, DNAH12, DNAH1 or a combination thereof.

In certain embodiments of the present disclosure, the method of diagnosing a subject with pancreatic cancer includes determining the presence of one or more biomarkers in a biological sample from the subject, wherein the biomarker includes one or more biomarkers selected from the RAS/p53/JUN/CTNB1 signaling pathway. In certain embodiments, the one or more RAS/p53/JUN/CTNB1 signaling pathway biomarkers include, but are not limited to, KIAA1109, ODZ3, PMFBP1, EPHB3, LIMCH1, TCF20, ERP29 or a combination thereof. In certain embodiments, the one or more RAS/p53/JUN/CTNB1 signaling pathway biomarkers is LIMCH1.

In certain embodiments of the present disclosure, the method of diagnosing a subject with pancreatic cancer includes determining the presence of one or more biomarkers in a biological sample from the subject, wherein the biomarker includes one or more biomarkers selected from MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13 or a combination thereof. In certain embodiments, the one or more biomarkers are STARD8 (DLC3), ATP2A1, RTTN or a combination thereof.

In certain embodiments of the present disclosure, the method of diagnosing a subject with pancreatic cancer includes determining the presence of one or more biomarkers in a biological sample from the subject, wherein the biomarker includes one or more biomarkers selected from MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13, DES, IMMT, TPM1, SNRPE, VCAM1, GRB2, SHROOM3, HMOX1, POSTN, MMP10, MMP-2, THBS2, EWSR1, NOD1, ADAMTS9, AFP, SYNE1, SYNE2, EPHB1, UFD1L, TEAD1, RYR3, CMYA5, MYLK, TOP2B, KIAA1109, ODZ3, PMFBP1, EPHB3, LIMCH1, TCF20, ERP29, OBSCN, LOXL3, MLEC, DNAH1, DNAH5, DNAH12, DNAH17, SCYL2, FKBP10, FLRT3, ZHX2 (AFR1), ZNF804A, ACTN2 or a combination thereof.

In certain embodiments of the present disclosure, the method of diagnosing, prognosing or screening for pancreatic cancer in a subject includes, obtaining a biological sample from the subject and determining the presence of one or more biomarkers in a biological sample of the subject selected from RTTN, DNAH12, TPM1, DNAH1, STARD8, ATP2A1, TOP2B, LIMCH1, SYNE1, THBS2, LOXL3 or a combination thereof, wherein the detection of the one or more biomarkers indicates the presence of pancreatic cancer in the subject.

In certain embodiments of the present disclosure, the biomarkers described herein and shown in Tables 4, 5, 6, 7, 8, 9, 10, 11, 12, 13 and 15 can be detected individually, as described above, or in panels including at least two biomarkers, at least three biomarkers, at least four biomarkers, at least five biomarkers, at least six biomarkers, at least seven biomarkers or at least eight biomarkers. For example, when used in a panel test, the levels of at least two biomarkers can be optionally tested from the same biological sample obtained from the subject (*e.g*., by detecting the quantities or amounts of various biomarkers in the same blood sample obtained from a patient) or in different biological samples from the subject. When combined in a panel test, the panel test can include determining the presence and/or levels for each of 2, 3, 4, 5, 6, 10, 15, 20, 25, 35 or more different biomarkers. The combination of multiple biomarkers in a panel test serves to reduce the number of false positives and false negatives should an aberrant value for one particular member of the panel be found.

In certain embodiments of the present disclosure, the method of diagnosing a subject with pancreatic cancer includes determining the presence of two or more biomarkers in a panel of biomarkers in a biological sample from the subject, wherein the panel of biomarkers includes at least one biomarker selected from each of the following signaling pathways or networks: the TGFβ/integrin signaling pathway and the HNF4α transcription factor network. Non-limiting examples of TGFβ/integrin signaling pathway and HNF4α transcription factor network biomarkers are described above and in Tables 4-13 and 15. In certain embodiments, at least one TGFβ/integrin signaling pathway biomarker can be SYNE1, THBS2, TOP2B, TPM1 or a combination thereof. In certain embodiments, the at least one HNF4α transcription network pathway biomarker can be LOXL3, DNAH12, DNAH1 or a combination thereof.

In certain embodiments of the present disclosure, the method of diagnosing a subject with pancreatic cancer includes determining the presence of two or more biomarkers in a panel of biomarkers in a biological sample from the subject, wherein the panel of biomarkers includes at least one biomarker selected from each of the following signaling pathways or networks: the TGFβ/integrin signaling pathway and the RAS/p53/JUN/CTNB1 signaling pathway. Non-limiting examples of TGFβ/integrin signaling pathway and RAS/p53/JUN/CTNB1 signaling pathway biomarkers are described above and in Tables 4-13 and 15. In certain embodiments, the at least one TGFβ/integrin signaling pathway biomarker can be SYNE1, THBS2, TOP2B, TPM1 or a combination thereof. In certain embodiments, the at least one RAS/p53/JUN/CTNB1 signaling pathway biomarker can be LIMCH1.

In certain embodiments of the present disclosure, the method of diagnosing a subject with pancreatic cancer includes determining the presence of two or more biomarkers in a panel of biomarkers in a biological sample from the subject, wherein the panel of biomarkers includes at least one biomarker selected from the TGFβ/integrin signaling pathway and at least one biomarker selected from MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13 or a combination thereof. In certain embodiments, the at least one TGFβ/integrin signaling pathway biomarker can be SYNE1, THBS2, TOP2B, TPM1 or a combination thereof. In certain embodiments, the at least one biomarker can be STARD8 (DLC3), ATP2A1, RTTN or a combination thereof.

In certain embodiments of the present disclosure, the method of diagnosing a subject with pancreatic cancer includes determining the presence of two or more biomarkers in a panel of biomarkers in a biological sample from the subject, wherein the panel of biomarkers includes at least one biomarker selected from each of the following signaling pathways or networks: the HNF4α transcription factor network and the RAS/p53/JUN/CTNB1 signaling pathway. Non-limiting examples of HNF4α transcription factor network and RAS/p53/JUN/CTNB1 signaling pathway biomarkers are described above and in Tables 4-13 and 15. In certain embodiments, the at least one HNF4α transcription network pathway biomarker can be LOXL3, DNAH12, DNAH1 or a combination thereof. In certain embodiments, the at least one RAS/p53/JUN/CTNB1 signaling pathway biomarker can be LIMCH1.

In certain embodiments of the present disclosure, the method of diagnosing a subject with pancreatic cancer includes determining the presence of two or more biomarkers in a panel of biomarkers in a biological sample from the subject, wherein the panel of biomarkers includes at least one biomarker selected from the RAS/p53/JUN/CTNB1 signaling pathway and at least one biomarker selected from MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN and ABCA13. In certain embodiments, the at least one RAS/p53/JUN/CTNB1 signaling pathway biomarker can be LIMCH1. In certain embodiments, the at least one biomarker can be STARD8 (DLC3), ATP2A1, RTTN or a combination thereof.

In certain embodiments of the present disclosure, the method of diagnosing a subject with pancreatic cancer includes determining the presence of two or more biomarkers in a panel of biomarkers in a biological sample from the subject, wherein the panel of biomarkers includes at least one biomarker selected from the HNF4α transcription factor network and at least one biomarker selected from MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN and ABCA13. In certain embodiments, the at least one HNF4α transcription network pathway biomarker can be LOXL3, DNAH12, DNAH1 or a combination thereof. In certain embodiments, the at least one biomarker can be STARD8 (DLC3), ATP2A1, RTTN or a combination thereof.

In certain embodiments of the present disclosure, the method of diagnosing, prognosing or screening for pancreatic cancer in a subject includes determining the presence of three or more or four or more biomarkers in a sample from the subject. For example, but not by way of limitation, the method of diagnosing a subject with pancreatic cancer includes determining the presence of three or more biomarkers in a panel of biomarkers in a biological sample from the subject, wherein the panel of biomarkers includes at least one biomarker selected from each of the following signaling pathways or networks: the TGFβ/integrin signaling pathway, the HNF4α transcription factor network and the RAS/p53/JUN/CTNB1 signaling pathway. In certain embodiments, the at least one TGFβ/integrin signaling pathway biomarker can be SYNE1, THBS2, TOP2B, TPM1 or a combination thereof. In certain embodiments, the at least one HNF4α transcription network pathway biomarker can be LOXL3, DNAH12, DNAH1 or a combination thereof. In certain embodiments, the at least one RAS/p53/JUN/CTNB1 signaling pathway biomarker can be LIMCH1.

In certain embodiments, the method of diagnosing a subject with pancreatic cancer includes determining the presence of three or more biomarkers in a panel of biomarkers in a biological sample from the subject, wherein the panel of biomarkers includes at least one biomarker selected from each of the following signaling pathways or networks: the TGFβ/integrin signaling pathway and the HNF4α transcription factor network, and at least one biomarker selected from MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN and ABCA13. In certain embodiments, the at least one TGFβ/integrin signaling pathway biomarker can be SYNE1, THBS2, TOP2B, TPM1 or a combination thereof. In certain embodiments, the at least one HNF4α transcription network pathway biomarker can be LOXL3, DNAH12, DNAH1 or a combination thereof. In certain embodiments, the at least one biomarker can be STARD8 (DLC3), ATP2A1, RTTN or a combination thereof.

In certain embodiments, the method of diagnosing a subject with pancreatic cancer includes determining the presence of three or more biomarkers in a panel of biomarkers in a biological sample from the subject, wherein the panel of biomarkers includes at least one biomarker selected from each of the following signaling pathways or networks: the HNF4α transcription factor network and the RAS/p53/JUN/CTNB1 signaling pathway, and at least one biomarker selected from MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN and ABCA13. In certain embodiments, the at least one RAS/p53/JUN/CTNB1 signaling pathway biomarker can be LIMCH1. In certain embodiments, the at least one HNF4α transcription network pathway biomarker can be LOXL3, DNAH12, DNAH1 or a combination thereof. In certain embodiments, the at least one biomarker can be STARD8 (DLC3), ATP2A1, RTTN or a combination thereof.

In certain embodiments, the method of diagnosing a subject with pancreatic cancer includes determining the presence of three or more biomarkers in a panel of biomarkers in a biological sample from the subject, wherein the panel of biomarkers includes at least one biomarker selected from each of the following signaling pathways or networks: the TGFβ/integrin signaling pathway and the RAS/p53/JUN/CTNB1 signaling pathway, and at least one biomarker selected from MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN and ABCA13. In certain embodiments, the at least one RAS/p53/JUN/CTNB1 signaling pathway biomarker can be LIMCH1. In certain embodiments, the at least one TGFβ/integrin signaling pathway biomarker can be SYNE1, THBS2, TOP2B, TPM1 or a combination thereof. In certain embodiments, the at least one biomarker can be STARD8 (DLC3), ATP2A1, RTTN or a combination thereof.

In certain embodiments, the method of diagnosing a subject with pancreatic cancer includes determining the presence of four or more biomarkers in a panel of biomarkers in a biological sample from the subject, wherein the panel of biomarkers includes at least one biomarker selected from each of the following signaling pathways or networks: the TGFβ/integrin signaling pathway, the HNF4α transcription factor network and the RAS/p53/JUN/CTNB1 signaling pathway, and at least one biomarker selected from MANF, ZNF485, IMPA1, SVEP1, KIAA1671, KIAA1529, GNN, DOS, STARD8 (DLC3), SCN8A, U2SURP, TCHP, RAD51C, ATP2A1, NLRX1, ZNF160, RTTN, ABCA13 or combination thereof. In certain embodiments, the at least one RAS/p53/JUN/CTNB1 signaling pathway biomarker can be LIMCH1. In certain embodiments, the at least one TGFβ/integrin signaling pathway biomarker can be SYNE1, THBS2, TOP2B, TPM1 or a combination thereof. In certain embodiments, the at least one HNF4α transcription network pathway biomarker can be LOXL3, DNAH12, DNAH1 or a combination thereof. In certain embodiments, the at least one biomarker can be STARD8 (DLC3), ATP2A1, RTTN or a combination thereof.

In certain embodiments, the method of diagnosing a subject with pancreatic cancer includes determining the presence of six or more biomarkers in a panel of biomarkers in a biological sample from the subject. For example, and not by way of limation, the six or more biomarkers can be selected from the following signaling pathways or networks: the TGFβ/integrin signaling pathway, the HNF4α transcription factor network, the RAS/p53/JUN/CTNB1 signaling pathway or a combination thereof, and/or selected from MANF, ZNF485, IMPA1, SVEP1, KIAA1671, KIAA1529, GNN, DOS, STARD8 (DLC3), SCN8A, U2SURP, TCHP, RAD51C, ATP2A1, NLRX1, ZNF160, RTTN, ABCA13 or a combination thereof. In certain embodiments, the method includes determining the presence of six or more biomarkers in a panel of biomarkers in a biological sample from the subject, wherein the panel of biomarkers includes at least two biomarkers selected from each of the following signaling pathways or networks: the TGFβ/integrin signaling pathway, the HNF4α transcription factor network and the RAS/p53/JUN/CTNB1 signaling pathway.

In certain embodiments, the method of diagnosing a subject with pancreatic cancer includes determining the presence of eight or more biomarkers in a panel of biomarkers in a biological sample from the subject. For example, and not by way of limation, the eight or more biomarkers can be selected from the following signaling pathways or networks: the TGFβ/integrin signaling pathway, the HNF4α transcription factor network, the RAS/p53/JUN/CTNB1 signaling pathway or a combination thereof, and/or selected from MANF, ZNF485, IMPA1, SVEP1, KIAA1671, KIAA1529, GNN, DOS, STARD8 (DLC3), SCN8A, U2SURP, TCHP, RAD51C, ATP2A1, NLRX1, ZNF160, RTTN, ABCA13 or a combination thereof. In certain embodiments, the method includes determining the presence of eight or more biomarkers in a panel of biomarkers in a biological sample from the subject, wherein the panel of biomarkers includes at least two biomarkers selected from each of the following signaling pathways or networks: the TGFβ/integrin signaling pathway, the HNF4α transcription factor network and the RAS/p53/JUN/CTNB1 signaling pathway, and at least two biomarkers selected from MANF, ZNF485, IMPA1, SVEP1, KIAA1671, KIAA1529, GNN, DOS, STARD8 (DLC3), SCN8A, U2SURP, TCHP, RAD51C, ATP2A1, NLRX1, ZNF160, RTTN and ABCA13.

The present disclosure further provides methods for differentially diagnosing a subject with early stage (e.g., resectable cancer) or advanced stage (invasive and/or metastatic cancer) cancer. In certain embodiments, the method to diagnose a subject with advanced stage pancreatic cancer includes determining the presence of one or more biomarkers in a biological sample from the subject, wherein the detection of the one or more biomarkers is an indication that the subject has metastatic pancreatic cancer. For example, by not by way of limitation, the one or more biomarkers can include DNAH12, DNAH1, STARD8, ATP2A1, TOP2B, THBS2 or a combination thereof.

In certain embodiments, the method to diagnose a subject with early stage and/or resectable pancreatic cancer includes determining the presence of one or more biomarkers in a biological sample from the subject, wherein the detection of the one or more biomarkers is an indication that the subject has early stage and/or resectable pancreatic cancer. For example, by not by way of limitation, the one or more biomarkers can include RTTN, DNAH12, TPM1, DNAH1, STARD8, ATP2A1, TOP2B, SYNE1, THBS2, LOXL3 or a combination thereof.

In certain embodiments, the information provided by the methods described herein can be used by the physician in determining the most effective course of treatment (*e.g*., preventative or therapeutic). A course of treatment refers to the measures taken for a patient after the assessment of increased risk for development of pancreatic cancer is made. For example, when a subject is identified to have an increased risk of developing cancer, the physician can determine whether frequent monitoring for biomarker detection is required as a prophylactic measure. Also, when the subject is determined to have pancreatic cancer (*e.g*., based on the presence of one or more biomarkers in a biological sample from a subject), it can be advantageous to follow such detection with a biopsy, surgical treatment, chemotherapy, radiation, immunotherapy, biological modifier therapy, gene therapy, vaccines and the like, or adjust the span of time during which the patient is treated.

### Biomarker Detection

A biomarker used in the methods of the disclosure can be identified in a biological sample using any method known in the art. Determining the presence of a biomarker, protein or degradation product thereof, the presence of mRNA or pre-mRNA, or the presence of any biological molecule or product that is indicative of biomarker expression, or degradation product thereof, can be carried out for use in the methods of the disclosure by any method described herein or known in the art.

### Protein Detection Techniques

Methods for the detection of protein biomarkers are well known to those skilled in the art, and include but are not limited to mass spectrometry techniques, 1-D or 2-D gel-based analysis systems, chromatography, enzyme linked immunosorbent assays (ELISAs), radioimmunoassays (RIA), enzyme immunoassays (EIA), Western Blotting, immunoprecipitation and immunohistochemistry. These methods use antibodies, or antibody equivalents, to detect protein, or use biophysical techniques. Antibody arrays or protein chips can also be employed, see for example U.S. Patent Application Nos: 2003/0013208A1; 2002/0155493A1, 2003/0017515 and U.S. Pat. Nos. 6,329,209 and 6,365,418, herein incorporated by reference in their entireties.

ELISA and RIA procedures can be conducted such that a biomarker standard is labeled (with a radioisotope such as ¹²⁵I or ³⁵S, or an assayable enzyme, such as horseradish peroxidase or alkaline phosphatase), and, together with the unlabeled sample, brought into contact with the corresponding antibody, whereon a second antibody is used to bind the first, and radioactivity or the immobilized enzyme assayed (competitive assay). Alternatively, the biomarker in the sample is allowed to react with the corresponding immobilized antibody, radioisotope or enzyme-labeled anti-biomarker antibody is allowed to react with the system, and radioactivity or the enzyme assayed (ELISA-sandwich assay). Other conventional methods can also be employed as suitable.

The above techniques can be conducted essentially as a "one-step" or "two-step" assay. A "one-step" assay involves contacting antigen with immobilized antibody and, without washing, contacting the mixture with labeled antibody. A "two-step" assay involves washing before contacting the mixture with labeled antibody. Other conventional methods can also be employed as suitable.

In certain embodiments, a method for measuring biomarker expression includes the steps of: contacting a biological sample, *e.g*., blood and/or plasma, with an antibody or variant (*e.g*., fragment) thereof which selectively binds the biomarker, and detecting whether the antibody or variant thereof is bound to the sample. A method can further include contacting the sample with a second antibody, *e.g*., a labeled antibody. The method can further include one or more steps of washing, *e.g*., to remove one or more reagents.

It can be desirable to immobilize one component of the assay system on a support, thereby allowing other components of the system to be brought into contact with the component and readily removed without laborious and time-consuming labor. It is possible for a second phase to be immobilized away from the first, but one phase is usually sufficient.

It is possible to immobilize the enzyme itself on a support, but if solid-phase enzyme is required, then this is generally best achieved by binding to antibody and affixing the antibody to a support, models and systems for which are well-known in the art. Simple polyethylene can provide a suitable support.

Enzymes employable for labeling are not particularly limited, but can be selected, for example, from the members of the oxidase group. These catalyze production of hydrogen peroxide by reaction with their substrates, and glucose oxidase is often used for its good stability, ease of availability and cheapness, as well as the ready availability of its substrate (glucose). Activity of the oxidase can be assayed by measuring the concentration of hydrogen peroxide formed after reaction of the enzyme-labeled antibody with the substrate under controlled conditions well-known in the art.

Other techniques can be used to detect a biomarker according to a practitioner's preference based upon the present disclosure. One such technique that can be used for detecting and quantitating biomarker protein levels is Western blotting (Towbin et al., Proc. Nat. Acad. Sci. 76:4350 (1979)). Cells can be frozen, homogenized in lysis buffer, and the lysates subjected to SDS-PAGE and blotting to a membrane, such as a nitrocellulose filter. Antibodies (unlabeled) are then brought into contact with the membrane and assayed by a secondary immunological reagent, such as labeled protein A or anti-immunoglobulin (suitable labels including ¹²⁵I, horseradish peroxidase and alkaline phosphatase). Chromatographic detection can also be used. In certain embodiments, immunodetection can be performed with antibody to a biomarker using the enhanced chemiluminescence system (*e.g*., from PerkinElmer Life Sciences, Boston, Mass.). The membrane can then be stripped and re-blotted with a control antibody, *e.g*., anti-actin (A-2066) polyclonal antibody from Sigma (St. Louis, Mo.).

Immunohistochemistry can be used to detect the expression and/ presence of a biomarker, *e.g*., in a biopsy sample. A suitable antibody is brought into contact with, for example, a thin layer of cells, followed by washing to remove unbound antibody, and then contacted with a second, labeled, antibody. Labeling can be by fluorescent markers, enzymes, such as peroxidase, avidin or radiolabeling. The assay is scored visually, using microscopy and the results can be quantitated.

Other machine or autoimaging systems can also be used to measure immunostaining results for the biomarker. As used herein, "quantitative" immunohistochemistry refers to an automated method of scanning and scoring samples that have undergone immunohistochemistry, to identify and quantitate the presence of a specified biomarker, such as an antigen or other protein. The score given to the sample is a numerical representation of the intensity of the immunohistochemical staining of the sample, and represents the amount of target biomarker present in the sample. As used herein, Optical Density (OD) is a numerical score that represents intensity of staining. As used herein, semi-quantitative immunohistochemistry refers to scoring of immunohistochemical results by human eye, where a trained operator ranks results numerically (*e.g*., as 1, 2 or 3).

Various automated sample processing, scanning and analysis systems suitable for use with immunohistochemistry are available in the art. Such systems can include automated staining (see, *e.g*., the Benchmark system, Ventana Medical Systems, Inc.) and microscopic scanning, computerized image analysis, serial section comparison (to control for variation in the orientation and size of a sample), digital report generation, and archiving and tracking of samples (such as slides on which tissue sections are placed). Cellular imaging systems are commercially available that combine conventional light microscopes with digital image processing systems to perform quantitative analysis on cells and tissues, including immunostained samples. See, *e.g*., the CAS-200 system (Becton, Dickinson & Co.).

Antibodies against biomarkers can also be used for imaging purposes, for example, to detect the presence of a biomarker in cells of a subject. Suitable labels include radioisotopes, iodine (¹²⁵I, ¹²¹I), carbon (¹⁴C), sulphur (³⁵S), tritium (³H), indium (¹¹²In), and technetium (^{99m}Tc), fluorescent labels, such as fluorescein and rhodamine and biotin. Immunoenzymatic interactions can be visualized using different enzymes such as peroxidase, alkaline phosphatase, or different chromogens such as DAB, AEC or Fast Red.

For *in vivo* imaging purposes, antibodies are not detectable, as such, from outside the body, and so must be labeled, or otherwise modified, to permit detection. Markers for this purpose can be any that do not substantially interfere with the antibody binding, but which allow external detection. Suitable markers can include those that can be detected by X-radiography, NMR or MRI. For X-radiographic techniques, suitable markers include any radioisotope that emits detectable radiation but that is not overtly harmful to the subject, such as barium or caesium, for example. Suitable markers for NMR and MRI generally include those with a detectable characteristic spin, such as deuterium, which can be incorporated into the antibody by suitable labeling of nutrients for the relevant hybridoma, for example.

The size of the subject, and the imaging system used, will determine the quantity of imaging moiety needed to produce diagnostic images. In the case of a radioisotope moiety, for a human subject, the quantity of radioactivity injected will normally range from about 5 to 20 millicuries of technetium-99m.

The labeled antibody or antibody fragment will then preferentially accumulate at the location of cells which contain a biomarker. The labeled antibody or variant thereof, *e.g*., antibody fragment, can then be detected using known techniques. Antibodies include any antibody, whether natural or synthetic, full length or a fragment thereof, monoclonal or polyclonal, that binds sufficiently strongly and specifically to the biomarker to be detected. An antibody can have a K_{d} of at most about 10⁻⁶M, 10⁻⁷M, 10⁻⁸M, 10⁻⁹M, 10⁻¹⁰M, 10⁻¹¹M, 10⁻¹²M. The phrase "specifically binds" refers to binding of, for example, an antibody to an epitope or antigen or antigenic determinant in such a manner that binding can be displaced or competed with a second preparation of identical or similar epitope, antigen or antigenic determinant.

Antibodies and derivatives thereof that can be used encompasses polyclonal or monoclonal antibodies, chimeric, human, humanized, primatized (CDR-grafted), veneered or single-chain antibodies, phase produced antibodies (*e.g*., from phage display libraries), as well as functional binding fragments, of antibodies. For example, antibody fragments capable of binding to a biomarker, or portions thereof, including, but not limited to Fv, Fab, Fab' and F(ab')₂ fragments can be used. Such fragments can be produced by enzymatic cleavage or by recombinant techniques. For example, papain or pepsin cleavage can generate Fab or F(ab')₂ fragments, respectively. Other proteases with the requisite substrate specificity can also be used to generate Fab or F(ab')₂ fragments. Antibodies can also be produced in a variety of truncated forms using antibody genes in which one or more stop codons have been introduced upstream of the natural stop site. For example, a chimeric gene encoding a F(ab')₂ heavy chain portion can be designed to include DNA sequences encoding the CH, domain and hinge region of the heavy chain.

Synthetic and engineered antibodies are described in, *e.g*., Cabilly et al., U.S. Pat. No. 4,816,567 Cabilly et al., European Patent No. 0,125,023 B1; Boss et al., U.S. Pat. No. 4,816,397; Boss et al., European Patent No. 0,120,694 B1; Neuberger, M. S. et al., WO 86/01533; Neuberger, M. S. et al., European Patent No. 0,194,276 B1; Winter, U.S. Pat. No. 5,225,539; Winter, European Patent No. 0,239,400 B1; Queen et al., European Patent No. 0451216 B1; and Padlan, E. A. et al., EP 0519596 A1. See also, Newman, R. et al., BioTechnology, 10: 1455-1460 (1992), regarding primatized antibody, and Ladner et al., U.S. Pat. No. 4,946,778 and Bird, R. E. et al., Science, 242: 423-426 (1988)) regarding single-chain antibodies.

In certain embodiments, agents that specifically bind to a polypeptide other than antibodies are used, such as peptides. Peptides that specifically bind can be identified by any means known in the art, *e.g*., peptide phage display libraries. Generally, an agent that is capable of detecting a biomarker polypeptide, such that the presence of a biomarker is detected and/or quantitated, can be used. As defined herein, an "agent" refers to a substance that is capable of identifying or detecting a biomarker in a biological sample (*e.g*., identifies or detects the mRNA of a biomarker, the DNA of a biomarker, the protein of a biomarker). In certain embodiments, the agent is a labeled or labelable antibody which specifically binds to a biomarker polypeptide.

In addition, a biomarker can be detected using Mass Spectrometry such as MALDI/TOF (time-of-flight), SELDI/TOF, liquid chromatography-mass spectrometry (LC-MS), gas chromatography-mass spectrometry (GC-MS), high performance liquid chromatography-mass spectrometry (HPLC-MS), capillary electrophoresis-mass spectrometry, nuclear magnetic resonance spectrometry, or tandem mass spectrometry (*e.g.,* MS/MS, MS/MS/MS, ESI-MS/MS, etc.). *See* for example, U.S. Patent Application Nos: 20030199001, 20030134304, 20030077616, which are herein incorporated by reference.

Mass spectrometry methods are well known in the art and have been used to quantify and/or identify biomolecules, such as proteins (*see, e.g*., Li et al. (2000) Tibtech 18:151-160; Rowley et al. (2000) Methods 20: 383-397; and Kuster and Mann (1998) Curr. Opin. Structural Biol. 8: 393-400). Further, mass spectrometric techniques have been developed that permit at least partial de novo sequencing of isolated proteins. Chait et al., Science 262:89-92 (1993); Keough et al., Proc. Natl. Acad. Sci. USA. 96:7131-6 (1999); reviewed in Bergman, EXS 88:133-44 (2000).

In certain embodiments, a gas phase ion spectrophotometer is used. In other embodiments, laser-desorption/ionization mass spectrometry is used to analyze the sample. Modem laser desorption/ionization mass spectrometry ("LDI-MS") can be practiced in two main variations: matrix assisted laser desorption/ionization ("MALDI") mass spectrometry and surface-enhanced laser desorption/ionization ("SELDI"). In MALDI, the analyte is mixed with a solution containing a matrix, and a drop of the liquid is placed on the surface of a substrate. The matrix solution then co-crystallizes with the biological molecules. The substrate is inserted into the mass spectrometer. Laser energy is directed to the substrate surface where it desorbs and ionizes the biological molecules without significantly fragmenting them. However, MALDI has limitations as an analytical tool. It does not provide means for fractionating the sample, and the matrix material can interfere with detection, especially for low molecular weight analytes. See, *e.g.,* U.S. Pat. No. 5,118,937 (Hillenkamp et al.), and U.S. Pat. No. 5,045,694 (Beavis & Chait).

For additional information regarding mass spectrometers, *see, e.g*., Principles of Instrumental Analysis, 3rd edition. Skoog, Saunders College Publishing, Philadelphia, 1985; and Kirk-Othmer Encyclopedia of Chemical Technology, 4th ed. Vol. 15 (John Wiley & Sons, New York 1995), pp. 1071-1094.

Detection of the presence of a marker or other substances will typically involve detection of signal intensity. This, in turn, can reflect the quantity and character of a polypeptide bound to the substrate. For example, in certain embodiments, the signal strength of peak values from spectra of a first sample and a second sample can be compared (*e.g*., visually, by computer analysis etc.), to determine the relative amounts of a particular biomarker. Software programs such as the Biomarker Wizard program (Ciphergen Biosystems, Inc., Fremont, Calif.) can be used to aid in analyzing mass spectra. The mass spectrometers and their techniques are well known to those of skill in the art.

Any person skilled in the art understands, any of the components of a mass spectrometer (*e.g*., desorption source, mass analyzer, detect, etc.) and varied sample preparations can be combined with other suitable components or preparations described herein, or to those known in the art. For example, in certain embodiments a control sample can contain heavy atoms (*e.g*., ¹³C) thereby permitting the test sample to be mixed with the known control sample in the same mass spectrometry run.

In certain embodiments, a laser desorption time-of-flight (TOF) mass spectrometer is used. In laser desorption mass spectrometry, a substrate with a bound marker is introduced into an inlet system. The marker is desorbed and ionized into the gas phase by laser from the ionization source. The ions generated are collected by an ion optic assembly, and then in a time-of-flight mass analyzer, ions are accelerated through a short high voltage field and let drift into a high vacuum chamber. At the far end of the high vacuum chamber, the accelerated ions strike a sensitive detector surface at a different time. Since the time-of-flight is a function of the mass of the ions, the elapsed time between ion formation and ion detector impact can be used to identify the presence or absence of molecules of specific mass to charge ratio.

In certain embodiments, the relative amounts of one or more biomarkers present in a first or second sample is determined, in part, by executing an algorithm with a programmable digital computer. The algorithm identifies at least one peak value in the first mass spectrum and the second mass spectrum. The algorithm then compares the signal strength of the peak value of the first mass spectrum to the signal strength of the peak value of the second mass spectrum of the mass spectrum. The relative signal strengths are an indication of the amount of the biomarker that is present in the first and second samples. A standard containing a known amount of a biomarker can be analyzed as the second sample to better quantify the amount of the biomarker present in the first sample. In certain embodiments, the identity of the biomarkers in the first and second sample can also be determined.

### RNA Detection Techniques

Any method for qualitatively or quantitatively detecting a nucleic acid biomarker can be used. Detection of RNA transcripts can be achieved, for example, by Northern blotting, wherein a preparation of RNA is run on a denaturing agarose gel, and transferred to a suitable support, such as activated cellulose, nitrocellulose or glass or nylon membranes. Radiolabeled cDNA or RNA is then hybridized to the preparation, washed and analyzed by autoradiography.

Detection of RNA transcripts can further be accomplished using amplification methods. For example, it is within the scope of the present disclosure to reverse transcribe mRNA into cDNA followed by polymerase chain reaction (RT-PCR); or, to use a single enzyme for both steps as described in U.S. Pat. No. 5,322,770, or reverse transcribe mRNA into cDNA followed by symmetric gap ligase chain reaction (RT-AGLCR) as described by R. L. Marshall, et al., PCR Methods and Applications 4: 80-84 (1994).

In certain embodiments, quantitative real-time polymerase chain reaction (qRT-PCR) is used to evaluate mRNA levels of biomarker. The levels of a biomarker and a control mRNA can be quantitated in cancer tissue or cells and adjacent benign tissues. In one specific embodiment, the levels of one or more biomarkers can be quantitated in a biological sample.

Other known amplification methods which can be utilized herein include but are not limited to the so-called "NASBA" or "3SR" technique described in PNAS USA 87: 1874-1878 (1990) and also described in Nature 350 (No. 6313): 91-92 (1991); Q-beta amplification as described in published European Patent Application (EPA) No. 4544610; strand displacement amplification (as described in G. T. Walker et al., Clin. Chem. 42: 9-13 (1996) and European Patent Application No. 684315; and target mediated amplification, as described by PCT Publication WO9322461.

In situ hybridization visualization can also be employed, wherein a radioactively labeled antisense RNA probe is hybridized with a thin section of a biopsy sample, washed, cleaved with RNase and exposed to a sensitive emulsion for autoradiography. The samples can be stained with haematoxylin to demonstrate the histological composition of the sample, and dark field imaging with a suitable light filter shows the developed emulsion. Non-radioactive labels such as digoxigenin can also be used.

Another method for evaluation of biomarker expression is to detect mRNA levels of a biomarker by fluorescent in situ hybridization (FISH). FISH is a technique that can directly identify a specific region of DNA or RNA in a cell and therefore enables to visual determination of the biomarker expression in tissue samples. The FISH method has the advantages of a more objective scoring system and the presence of a built-in internal control consisting of the biomarker gene signals present in all non-neoplastic cells in the same sample. Fluorescence in situ hybridization is a direct in situ technique that is relatively rapid and sensitive. FISH test also can be automated. Immunohistochemistry can be combined with a FISH method when the expression level of the biomarker is difficult to determine by immunohistochemistry alone.

Alternatively, mRNA expression can be detected on a DNA array, chip or a microarray. Oligonucleotides corresponding to the biomarker(s) are immobilized on a chip which is then hybridized with labeled nucleic acids of a test sample obtained from a subject. Positive hybridization signal is obtained with the sample containing biomarker transcripts. Methods of preparing DNA arrays and their use are well known in the art. (See, for example, U.S. Pat. Nos. 6,618,6796; 6,379,897; 6,664,377; 6,451,536; 548,257; U.S. 20030157485 and Schena et al. 1995 Science 20:467-470; Gerhold et al. 1999 Trends in Biochem. Sci. 24, 168-173; and Lennon et al. 2000 Drug discovery Today 5: 59-65, which are herein incorporated by reference in their entirety). Serial Analysis of Gene Expression (SAGE) can also be performed (See for example U.S. Patent Application 20030215858).

To monitor mRNA levels, for example, mRNA can be extracted from the biological sample to be tested, reverse transcribed and fluorescent-labeled cDNA probes are generated. The microarrays capable of hybridizing to a biomarker, cDNA can then probed with the labeled cDNA probes, the slides scanned and fluorescence intensity measured. This intensity correlates with the hybridization intensity and expression levels.

Types of probes for detection of RNA include cDNA, riboprobes, synthetic oligonucleotides and genomic probes. The type of probe used will generally be dictated by the particular situation, such as riboprobes for in situ hybridization, and cDNA for Northern blotting, for example. In certain embodiments, the probe is directed to nucleotide regions unique to the particular biomarker RNA. The probes can be as short as is required to differentially recognize the particular biomarker mRNA transcripts, and can be as short as, for example, 15 bases; however, probes of at least 17 bases, at least 18 bases and at least 20 bases can be used. In certain embodiments, the primers and probes hybridize specifically under stringent conditions to a nucleic acid fragment having the nucleotide sequence corresponding to the target gene. As herein used, the term "stringent conditions" means hybridization will occur only if there is at least 95% or at least 97% identity between the sequences.

The form of labeling of the probes can be any that is appropriate, such as the use of radioisotopes, for example, ³²P and ³⁵S. Labeling with radioisotopes can be achieved, whether the probe is synthesized chemically or biologically, by the use of suitably labeled bases.

### Kits

In certain non-limiting embodiments, the present disclosure provides for a kit for determining whether a subject has pancreatic cancer includes a means for detecting one or more biomarkers selected from the biomarkers set forth in Tables 4, 5, 6, 7, 8, 9, 10, 11, 12, 13 and 15, or a combination thereof. The disclosure further provides for kits for determining the efficacy of a therapy for preventing or treating pancreatic cancer in a subject.

Types of kits include, but are not limited to, packaged probe and primer sets (*e.g*., TaqMan probe/primer sets), arrays/microarrays, biomarker-specific antibodies and beads, which further contain one or more probes, primers or other detection reagents for detecting one or more biomarkers of the present disclosure.

In a certain, non-limiting embodiment, a kit can include a pair of oligonucleotide primers suitable for polymerase chain reaction (PCR) or nucleic acid sequencing, for detecting one or more biomarker(s) to be identified. A pair of primers can include nucleotide sequences complementary to a biomarker set forth in Tables 4, 5, 6, 7, 8, 9, 10, 11, 12, 13 and 15, and can be of sufficient length to selectively hybridize with said biomarker. Alternatively, the complementary nucleotides can selectively hybridize to a specific region in close enough proximity 5' and/or 3' to the biomarker position to perform PCR and/or sequencing. Multiple biomarker-specific primers can be included in the kit to simultaneously assay large number of biomarkers. The kit can also include one or more polymerases, reverse transcriptase and nucleotide bases, wherein the nucleotide bases can be further detectably labeled.

In certain embodiments, a primer can be at least about 10 nucleotides or at least about 15 nucleotides or at least about 20 nucleotides in length and/or up to about 200 nucleotides or up to about 150 nucleotides or up to about 100 nucleotides or up to about 75 nucleotides or up to about 50 nucleotides in length.

In certain embodiments, the oligonucleotide primers can be immobilized on a solid surface or support, for example, on a nucleic acid microarray, wherein the position of each oligonucleotide primer bound to the solid surface or support is known and identifiable.

In a certain, non-limiting embodiment, a kit can include at least one nucleic acid probe, suitable for in situ hybridization or fluorescent in situ hybridization, for detecting the biomarker(s) to be identified. Such kits will generally include one or more oligonucleotide probes that have specificity for various biomarkers.

In certain non-limiting embodiments, a kit can include a primer for detection of a biomarker by primer extension.

In certain non-limiting embodiments, a kit can include at least one antibody for immunodetection of the biomarker(s) to be identified. Antibodies, both polyclonal and monoclonal, specific for a biomarker, can be prepared using conventional immunization techniques, as will be generally known to those of skill in the art. The immunodetection reagents of the kit can include detectable labels that are associated with, or linked to, the given antibody or antigen itself. Such detectable labels include, for example, chemiluminescent or fluorescent molecules (rhodamine, fluorescein, green fluorescent protein, luciferase, Cy3, Cy5 or ROX), radiolabels (³H, ³⁵S, ³²P, ¹⁴C, ¹³¹I) or enzymes (alkaline phosphatase, horseradish peroxidase).

In a certain non-limiting embodiment, the biomarker-specific antibody can be provided bound to a solid support, such as a column matrix, an array, or well of a microtiter plate. Alternatively, the support can be provided as a separate element of the kit.

In certain non-limiting embodiments, a kit can include one or more primers, probes, microarrays, or antibodies suitable for detecting one or more biomarkers set forth in Tables 4, 5, 6, 7, 8, 9, 10, 11, 12, 13, 15 or combinations thereof.

In certain non-limiting embodiments, a kit can include one or more primers, probes, microarrays, or antibodies suitable for detecting one, two, three, four, five, six, seven, eight, nine, ten, eleven, twelve, thirteen, fourteen or more of the following biomarkers: MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13, DES, IMMT, TPM1, SNRPE, VCAM1, GRB2, SHROOM3, HMOX1, POSTN, MMP10, MMP-2, THBS2, EWSR1, NOD1, ADAMTS9, AFP, SYNE1, SYNE2, EPHB1, UFD1L, TEAD1, RYR3, CMYA5, MYLK, TOP2B, KIAA1109, ODZ3, PMFBP1, EPHB3, LIMCH1, TCF20, ERP29, OBSCN, LOXL3, MLEC, DNAH1, DNAH5, DNAH12, DNAH17, SCYL2, FKBP10, FLRT3, ZHX2 (AFR1), ZNF804A and ACTN2.

In certain non-limiting embodiments, a kit can include one or more primers, probes, microarrays, or antibodies suitable for detecting one, two, three, four, five, six or seven of the following biomarkers: KIAA1109, ODZ3, PMFBP1, EPHB3, LIMCH1, TCF20, ERP29.

In certain non-limiting embodiments, a kit can include one or more primers, probes, microarrays, or antibodies suitable for detecting one, two, three, four, five, six, seven, eight, nine, ten or eleven of the following biomarkers: RTTN, DNAH12, TPM1, DNAH1, STARD8, ATP2A1, TOP2B, LIMCH1, SYNE1, THBS2 and LOXL3.

In certain embodiments, a kit can include one or more primers, probes, microarrays, or antibodies suitable for detecting one or more biomarkers of the TGFβ/integrin signaling pathway, including but not limited to, DES, IMMT, TPM1, SNRPE, VCAM1, GRB2, SHROOM3, HMOX1, POSTN, MMP10, MMP-2, THBS2, EWSR1, NOD1, ADAMTS9, AFP, SYNE1, SYNE2, EPHB1, UFD1L, TEAD1, RYR3, CMYA5, MYLK, TOP2B or a combination thereof.

In certain embodiments, a kit can include one or more primers, probes, microarrays, or antibodies suitable for detecting one or more biomarkers of the RAS/p53/JUN/CTNB1 signaling pathway, including but not limited to, KIAA1109, ODZ3, PMFBP1, EPHB3, LIMCH1, TCF20, ERP29 or a combination thereof.

In certain embodiments, a kit can include one or more primers, probes, microarrays, or antibodies suitable for detecting one or more biomarkers of the HNF4α transcription network pathway, including but not limited to, OBSCN, LOXL3, MLEC, DNAH1, DNAH5, DNAH12, DNAH17, SCYL2, FKBP10, FLRT3, ZHX2(AFR1), ZNF804A, ACTN2 or a combination thereof.

In certain embodiments, a kit can include one or more primers, probes, microarrays, or antibodies suitable for detecting one or more biomarkers selected from MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13 or a combination thereof.

In certain embodiments, a kit can include two or more primers, probes, microarrays, or antibodies suitable for detecting two or more biomarkers, where the kit includes at least one or more biomarkers from each of the following signaling pathways or networks: the TGFβ/integrin signaling pathway, *e.g*., SYNE1, THBS2, TOP2B and/or TPM1, and the HNF4α transcription factor network, *e.g*., LOXL3, DNAH12 and/or DNAH1.

In certain embodiments, a kit can include two or more primers, probes, microarrays, or antibodies suitable for detecting two or more biomarkers, where the kit includes at least one or more biomarkers from each of the following signaling pathways or networks: the TGFβ/integrin signaling pathway, *e.g*., SYNE1, THBS2, TOP2B and/or TPM1, and the RAS/p53/JUN/CTNB1 signaling pathway, *e.g*., LIMCH1.

In certain embodiments, a kit can include two or more primers, probes, microarrays, or antibodies suitable for detecting two or more biomarkers, where the kit includes at least one or more biomarkers from each of the following signaling pathways or networks: the HNF4α transcription factor network, *e.g*., LOXL3, DNAH12 and/or DNAH1, and the RAS/p53/JUN/CTNB1 signaling pathway, *e.g*., LIMCH1.

In certain embodiments, a kit can include two or more primers, probes, microarrays, or antibodies suitable for detecting two or more biomarkers, where the kit includes at least one or more biomarkers from the TGFβ/integrin signaling pathway, *e.g*., SYNE1, THBS2, TOP2B and/or TPM1, and at least one or more biomarkers selected from MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13 or a combination thereof.

In certain embodiments, a kit can include two or more primers, probes, microarrays, or antibodies suitable for detecting two or more biomarkers, where the kit includes at least one or more biomarkers from the HNF4α transcription factor network, *e.g*., LOXL3, DNAH12 and/or DNAH1, and at least one or more biomarkers selected from MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13 or a combination thereof.

In certain embodiments, a kit can include two or more primers, probes, microarrays, or antibodies suitable for detecting two or more biomarkers, where the kit includes at least one or more biomarkers from the RAS/p53/JUN/CTNB1 signaling pathway, *e.g*., LIMCH1, and at least one or more biomarkers selected from MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13 or a combination thereof.

In certain embodiments, a kit can include three or more primers, probes, microarrays, or antibodies suitable for detecting three or more biomarkers, where the kit includes at least one or more biomarkers from each of the following signaling pathways or networks: the TGFβ/integrin signaling pathway, the HNF4α transcription factor network and the RAS/p53/JUN/CTNB1 signaling pathway.

In certain embodiments, a kit can include three or more primers, probes, microarrays, or antibodies suitable for detecting three or more biomarkers, where the kit includes at least one or more biomarkers from each of the following signaling pathways or networks: the TGFβ/integrin signaling pathway and the HNF4α transcription factor network, and at least one biomarker selected from MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN and ABCA13.

In certain embodiments, a kit can include three or more primers, probes, microarrays, or antibodies suitable for detecting three or more biomarkers, where the kit includes at least one or more biomarkers from each of the following signaling pathways or networks: the TGFβ/integrin signaling pathway and the RAS/p53/JUN/CTNB1 signaling pathway, and at least one biomarker selected from MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN and ABCA13.

In certain embodiments, a kit can include three or more primers, probes, microarrays, or antibodies suitable for detecting three or more biomarkers, where the kit includes at least one or more biomarkers from each of the following signaling pathways or networks: the HNF4α transcription factor network and the RAS/p53/JUN/CTNB1 signaling pathway, and at least one biomarker selected from MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13 or a combination thereof.

In certain embodiments, a kit can include four or more primers, probes, microarrays, or antibodies suitable for detecting four or more biomarkers, where the kit includes at least one or more biomarkers from each of the following signaling pathways or networks: the TGFβ/integrin signaling pathway, the HNF4α transcription factor network and the RAS/p53/JUN/CTNB1 signaling pathway, and at least one biomarker selected from MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13 or a combination thereof.

In certain non-limiting embodiments, where the measurement means in the kit employs an array, the set of biomarkers set forth above can constitute at least 10 percent or at least 20 percent or at least 30 percent or at least 40 percent or at least 50 percent or at least 60 percent or at least 70 percent or at least 80 percent of the species of markers represented on the microarray. In certain non-limiting embodiments, a biomarker detection kit can include one or more detection reagents and other components (*e.g*., a buffer, enzymes such as DNA polymerases or ligases, chain extension nucleotides such as deoxynucleotide triphosphates, and in the case of Sanger-type DNA sequencing reactions, chain terminating nucleotides, positive control sequences, negative control sequences, and the like) necessary to carry out an assay or reaction to detect a biomarker. A kit can also include additional components or reagents necessary for the detection of a biomarker, such as secondary antibodies for use in western blotting immunohistochemistry. A kit can further include one or more other biomarkers or reagents for evaluating other prognostic factors, e.g., tumor stage.

A kit can further contain means for comparing the biomarker with a standard, and can include instructions for using the kit to detect the biomarker of interest. For example, the instructions can describe that the presence of a biomarker, set forth herein, is indicative that the subject has or will develop pancreatic cancer.

### Reports, Programmed Computers and Systems

The results of a test (*e.g.,* an individual's risk for cancer, such as pancreatic cancer), or an individual's predicted drug responsiveness (*e.g*., response to chemotherapy), based on assaying one or more biomarkers set forth in Tables 4, 5, 6, 7, 8, 9, 10, 11, 12, 13 and 15, and/or any other information pertaining to a test, can be referred to herein as a "report." A tangible report can optionally be generated as part of a testing process (which can be interchangeably referred to herein as "reporting," or as "providing" a report, "producing" a report or "generating" a report).

Examples of tangible reports can include, but are not limited to, reports in paper (such as computer-generated printouts of test results) or equivalent formats and reports stored on computer readable medium (such as a CD, USB flash drive or other removable storage device, computer hard drive, or computer network server, etc.). Reports, particularly those stored on computer readable medium, can be part of a database, which can optionally be accessible via the internet (such as a database of patient records or genetic information stored on a computer network server, which can be a "secure database" that has security features that limit access to the report, such as to allow only the patient and the patient's medical practitioners to view the report while preventing other unauthorized individuals from viewing the report, for example). In addition to, or as an alternative to, generating a tangible report, reports can also be displayed on a computer screen (or the display of another electronic device or instrument).

A report can include, for example, an individual's risk for cancer, such as pancreatic cancer, or can just include presence, absence or levels of one or more biomarkers set forth in Tables 4, 5, 6, 7, 8, 9, 10, 11, 12, 13 and 15 (for example, a report on computer readable medium such as a network server can include hyperlink(s) to one or more journal publications or websites that describe the medical/biological implications, such as increased or decreased disease risk, for individuals having certain biomarkers or levels of certain biomarkers). Thus, for example, the report can include disease risk or other medical/biological significance (*e.g*., drug responsiveness, suggested prophylactic treatment, etc.) as well as optionally also including the biomarker information, or the report can just include biomarker information without including disease risk or other medical/biological significance (such that an individual viewing the report can use the biomarker information to determine the associated disease risk or other medical/biological significance from a source outside of the report itself, such as from a medical practitioner, publication, website, etc., which can optionally be linked to the report such as by a hyperlink).

A report can further be "transmitted" or "communicated" (these terms can be used herein interchangeably), such as to the individual who was tested, a medical practitioner (*e.g*., a doctor, nurse, clinical laboratory practitioner, genetic counselor, etc.), a healthcare organization, a clinical laboratory and/or any other party or requester intended to view or possess the report. The act of "transmitting" or "communicating" a report can be by any means known in the art, based on the format of the report. Furthermore, "transmitting" or "communicating" a report can include delivering a report ("pushing") and/or retrieving ("pulling") a report. For example, reports can be transmitted/communicated by various means, including being physically transferred between parties (such as for reports in paper format) such as by being physically delivered from one party to another, or by being transmitted electronically or in signal form (e.g., via e-mail or over the internet, by facsimile and/or by any wired or wireless communication methods known in the art) such as by being retrieved from a database stored on a computer network server, etc.

In certain exemplary embodiments, the disclosed subject matter provides computers (or other apparatus/devices such as biomedical devices or laboratory instrumentation) programmed to carry out the methods described herein. For example, in certain embodiments, the disclosed subject matter provides a computer programmed to receive (*i*.*e*., as input) the identity of the one or more biomarkers disclosed herein, alone or in combination with other biomarkers, and provide (*i*.*e*., as output) the disease risk (*e.g*., risk of pancreatic cancer) or other result (e.g., disease diagnosis or prognosis, drug responsiveness, etc.) based on the level or identity of the biomarker(s). Such output (*e.g*., communication of disease risk, disease diagnosis or prognosis, drug responsiveness, etc.) can be, for example, in the form of a report on computer readable medium, printed in paper form, and/or displayed on a computer screen or other display.

Certain further embodiments of the disclosed subject matter provide a system for determining an individual's cancer risk, or whether an individual will benefit from chemotherapy treatment (or other therapy) or prophylactic treatment. Certain exemplary systems include an integrated "loop" in which an individual (or their medical practitioner) requests a determination of such individual's cancer risk (or drug response), this determination is carried out by testing a sample from the individual, and then the results of this determination are provided back to the requester. For example, in certain systems, a sample (*e.g*., stool, blood, etc.) is obtained from an individual for testing (the sample can be obtained by the individual or, for example, by a medical practitioner), the sample is submitted to a laboratory (or other facility) for testing (*e.g*., determining the biomarker(s) disclosed herein, alone or in combination with one or more other biomarkers), and then the results of the testing are sent to the patient (which optionally can be done by first sending the results to an intermediary, such as a medical practitioner, who then provides or otherwise conveys the results to the individual and/or acts on the results), thereby forming an integrated loop system for determining an individual's cancer risk (or drug response, etc.). The portions of the system in which the results are transmitted (*e.g.*, between any of a testing facility, a medical practitioner, and/or the individual) can be carried out by way of electronic or signal transmission (*e.g*., by computer such as via e-mail or the internet, by providing the results on a website or computer network server which can optionally be a secure database, by phone or fax, or by any other wired or wireless transmission methods known in the art).

In certain embodiments, the system is controlled by the individual and/or their medical practitioner in that the individual and/or their medical practitioner requests the test, receives the test results back, and (optionally) acts on the test results to reduce the individual's disease risk, such as by implementing a disease management system.

The various methods described herein, such as correlating the presence or absence or level of a biomarker with an altered (*e.g*., increased or decreased) risk (or no altered risk) for cancer, *e.g*., pancreatic cancer, can be carried out by automated methods such as by using a computer (or other apparatus/devices such as biomedical devices, laboratory instrumentation, or other apparatus/devices having a computer processor) programmed to carry out any of the methods described herein. For example, computer software (which can be interchangeably referred to herein as a computer program) can perform correlating the presence or absence of a biomarker in an individual with an altered (*e.g*., increased or decreased) risk (or no altered risk) for cancer, *e.g*., pancreatic cancer for the individual. Accordingly, certain embodiments of the disclosed subject matter provide a computer (or other apparatus/device) programmed to carry out any of the methods described herein.

The following Examples are offered to more fully illustrate the disclosure, but are not to be construed as limiting the scope thereof.

### EXAMPLE 1: An iPS cell line from human pancreatic ductal adenocarcinoma undergoes early to invasive stages of pancreatic cancer progression.

### MATERIALS AND METHODS

### General cell culture

293T cells and human pancreatic ductal carcinoma cell lines PanC-1 and MIAPaCa-2 were maintained in 90% Dulbecco's modified essential medium (Invitrogen, Carlsbad, CA) supplemented with 10% FBS (Hyclone, Logan, UT) and fed every other day. Irradiated mouse embryonic fibroblast (MEF) cells were purchased from R&D systems (Minneapolis, MN) and maintained in 85% DMEM (Invitrogen) supplemented with 15% FBS (Hyclone) on 0.1% gelatin (Millipore, Billerica, MA) pre-treated tissue culture dishes. Plated irradiated MEFs were used within 5 days.

### H1 huES/ human iPS culture

H1 huES (Thomson et al., 1998) and iPS-like clones were maintained in 80% Dulbecco's modified essential medium (DMEM) /F12 supplemented with 20% KNOCKOUT serum replacement, 0.1 mM nonessential amino acids (Invitrogen), 0.1 mM -mercaptoethanol (Sigma, St. Louis, MO), and 10 ng/ml human basic fibroblast growth factor (bFGF) (Invitrogen). Human ES/iPS-like clones were grown onto irradiated MEFs in 0.1% gelatinized tissue culture dishes. Cells were fed every day and passaged once a week. For passaging, human ES cells were detached by treatment with 1 mg/ml collagenase IV (Invitrogen) for 3 min at 37 °C, centrifuged, resuspended with human ES media supplemented with 10 µm Y27632 (Calbiochem, Darmstadt, Germany), and then seeded onto irradiated MEFs. The iPS-like lines were passaged mechanically with needles every 5-7 days. For RNA purification and differentiation, to remove MEFs, human ES and iPS-like lines were cultured on hES-qualified Matrigel (BD Bioscience, San Jose, CA) coated tissue culture dishes under mTeSR1 media (Stem Cell Technologies, BC, Canada).

### Lentivirus production and titration

The mouse tet-Oct4, -Sox2, -Klf4, and C-MyC lentiviral vectors are donated from the Jaenisch lab (Brambrink et al., 2008). The pWPT rtTA vector was generated by ligating the rtTA2-M2 gene, isolated from pUHrT 62-1 vector (Urlinger et al., 2000), into the PWT-GFP backbone vector, with GFP removed. 293T cells were plated at a density of 8 x105 cells per 100 mm dish. The next day, cells were transfected with 2.5 µg vector (PWPT-rtTA or TetO-four factor), 1.7 µg psPAX2 packaging vector, and 0.8 µg PMDG envelope vector with 30 µl Fugene6 (Roche, Basel, Switzerland), according to supplier's instructions. Sixteen hours post-transfection, medium was removed and fresh media was added, and cells were further cultured for 60 h. Finally, the virus supernatant was collected, and centrifuged at 4° C for 10 min. The supernatant was filtered through 0.45 µm, aliquoted, directly tested for virus titer, or kept until use. Tet0-GFP lentivirus was concomitantly produced to monitor the virus titer. The titer of lentivirus was checked by flow cytometery and microscopy 2-3 days post-infection. Generally 5-7 MOI of each lentivirus was used for infection. The lentivirus average titer of PanC-1 and MIAPaCa-2 control PDAC cells were 3 x108 infection units (IU) /ml and 3x109 IU/ml, respectively. The lentivirus titer on HT1080 fibroblasts was 2.5 x108 IU/ml.

### Isolation and culture of human pancreas epithelial tumor cells

Human pancreatic ductal adenocarcinoma and pancreatic margin tissue was obtained by surgical dissection under the patient's informed consent by Fox Chase Cancer Center. Patients signed consent and sample acquisition was approved by the Institutional Review Board in accordance to institutional sample procurement procedures. Tissue specimens were obtained from the operating room immediately after resection. 1-2 cc of tissue were taken from the center of the cancer and 1-2 cc of tissue were taken from the margin furthest from the cancer and immediately placed into sterile F12 media/or Leibovitz's L-15 media (Invitrogen) supplemented with 100 U/ml Penicillin, 100 µg/ml Streptomycin, 10 µg/ml Gentamycin, 2.5 µg/ml Fungizone, 10 µg/ml Ciprofloxacin, 100 U/ml Nystatin (Invitrogen) until dissociation. Tissue was dissociated in 0.7 mg/ml liberase H1 (Roche, Switzerland) as a supplier's protocol. Briefly, after rinsing twice with Hank's balanced buffered saline solution (HBSS, Invitrogen), tissue was transferred to liberase working solution (0.7 mg/ml liberase HI, DNase I 100 µg/ml, 25mM HEPES in HBSS) and minced with a scalpel. The minced tissue was transferred to a glass vial and incubated at 37° C (time varied depending on tissue size, maximum 1 hour). The liberase activity was then inhibited in quenching buffer (HBSS supplemented with 10% FBS) and passed through a 380 µm filter (Sigma) to remove tissue debris. The dissociated cells were rinsed with liberase quenching buffer followed by centrifugation. After washing twice with quenching buffer, dissociated cells were resuspended in completed defined KSFM (Invitrogen) supplemented with 5 ng/ml human EGF (BD biosciences) and 50 ng/ml cholera toxin (Sigma), 50 µg/ml bovine pituitary extract (Invitrogen), and seeded onto 5 µg/cm2 rat collagen I (BD biosciences) pre-coated tissue culture dishes, and cultured in a 37° C CO₂ incubator. Cells were fed every other day until infection. In cases of a long delivery time for the tissue, to remove autolyzed cells, minced tissue was digested in mild conditions (liberase HI 0.5 mg/ml approximately for 30 min) first and then passed through a 380 µm filter. In this condition, the dissociated single cells usually included autolyzed cells as well as blood cells but not many cancer cells. Therefore, to remove the autolyzed cells, dissociated cells were collected separately or discarded. Tissue trunk retained on top of 380 µm filter after 1st digestion was collected and digested with 0.7 mg/ml liberase H1 working solution at 37° C for 30-40 min (depending on tissue size), quenched, washed and then cultured as described above.

### Generation of iPS-like clones from human primary pancreatic ductal cancer and margin

### epithelial cells

Cultured pancreatic cancer and margin epithelial cells were infected with Tet0-Oct4, Tet0-Sox2, TetO-Klf4, TetO-Myc viruses, along with the PWPT-rtTA virus, 3-4 days after plating. Twenty four hours later, the cells were re-infected with the same viruses. After 48 hours post-2nd infection, the infected cells were detached and counted to reveal that approximately 100,000 cells survived from each margin or tumor sample. The cells were resuspended in human ES media (80% DMEM/F12 supplemented with 20% Knockout Serum Replacer, 1 mM L-glutamine, 0.1 mM non-essential amino acid, 0.1 mM beta-meracapto ethanol (BME), 10 ng/ml basic Fgf (Invitrogen) and plated onto irradiated mouse embryonic fibroblasts on 0.1% gelatinized tissue culture dishes. Cells were fed every day. ES-like flat colonies were picked with 22 gauge needles from days 12 to 36 postsecondary infection, deposited onto irradiated MEFs, fed every day, and passaged mechanically with needles every 5-7 days. The colonies were frozen down around passage 3-4, and the stable clones were frozen down after passage 10 (See above for ES/iPS cell culture).

### Pyrosequencing for KRAS codon 12 mutation

The *KRAS* codon12 mutation in primary tumor tissue was examined by pyrosequencing (Kanda et al., 2012). Twenty five nanograms of genomic DNAs isolated from paraffin slides were PCR amplified with PyroMark polymerase chain reaction kit (Qiagen) according to manufacturer's protocol. After amplification, 3 ul of reaction was loaded onto agarose gel to check PCR product. Ten microliters of biotinylated PCR product were immobilized onto Streptavidin Sepharose HP beads (GE Healthcare Bio-Sciences) and annealed with sequencing primer designed with PyroMark Assay Design Software (Qiagen) (See Table 1 for primers) as described above. Pyrosequencing for *KRAS* codon 12 was done in a PyroMark MD (Qiagen). All experiments were repeated three times with different batch of PCR products. Genomic DNAs from 10-12 margin iPSlike line and 10th margin primary epithelial cells cultured were used for biological negative control (2% base line-for GAT G12D, 0.5% base line for GGT G12V mutation). No DNA amplified PCR product and no primer annealed PCR product were used as technical negative controls.

### RT-PCR of RNA

For RT-PCR/qRT-PCR, H1 and iPS-like clones were cultured onto irradiated MEF cells. For CGH analysis, H1/H9 human ES cells and iPS like clones were cultured onto Matrigel under mTeSR media (Stem Cell Technologies) without mouse feeder layers. Total RNA was isolated by RNeasy Micro Kit (Qiagen, Valencia, CA), and 100 ng total RNA were reverse transcribed by using the iScript cDNA Synthesis kit (Bio rad, Hercules, CA). The cDNA (2 ng) was subjected to real time PCR on an iCycler (Bio-rad) with either SYBR green primers set or Taqman probes. For gene expression levels with Taqman probes, the delta Ct method was used with either *Gapdh* or *beta-actin* as a reference. The RT-PCR products of SYBR green primers were visualized on 2% agarose gels after staining with ethidium bromide to be sure of proper target amplification. The primer pairs are shown in Table 1.

### Immunostaining/Immunohistochemistry (IH)

For immunostaining on H1/iPS, cells were with fixed with 4% paraformaldehyde/PBS for 15 minutes at room temperature (RT) and permeabilized with 0.1% Triton X-100/PBS for 10 min at RT. After washing with PBS twice, a blocking solution (4% normal goat serum (Sigma) in PBS) was applied for 30 min at RT. Primary antibodies including SSEA4 (Millipore, 1:100), NANOG (Abcam, 1:100), and OCT4 (Abcam,1:100) were applied on the fixed cells and incubated at 4° C for overnight. After rinsing, the Alexa 488-conjugated goat anti mouse IgG or goat anti rabbit IgG antibodies were applied for 1 hour at RT. DAPI was applied at the final wash to stain nuclei. For immunohistochemistry, paraffin sectioned slides were antigen retrieved by boiling in 10 mM citric acid buffer (pH 6) in a microwave oven for 15 min. Next, the endogenous peroxidase activity in tissue slides was quenched in hydrogen peroxide solution for 15 min at RT. Tissues were blocked with protein blocker (Thermo Scientific) for 10 min, followed by avidin/biotin blocking (Vector lab, Burlingame, CA) for 15 min. Primary antibodies including K19 (Abcam, 1;2000), MUC5AC (Vector lab, 1:100), PDX-1 (Santa Cruz, 1:1000), SOX9 (Santa Cruz 1:500), HNF-4α (Santa Cruz, 1:250-1:500), MF20 (Hybridoma Bank 1:40), Vimentin (Stemgent, Cambridge, MA, 1:500), Beta III tubulin (Abcam, 1:2000), GFAP (Cell Signaling, 1:200), diluted in PBS supplemented with 0.1% BSA and 0.2% Triton-X 100, were applied and incubated for 12-16 hours at 4° C. After washing twice, tissues were incubated with biotinylated anti-mouse IgG (Vector lab) at 37° C for 30 min. Tissue sections were conjugated with avidin-Horseradish peroxidase (HRP) by using VectaStain Elite ABC kit (vector lab) at 37° C for 30 min, followed by developing with DAB peroxidase susbrate kit (Vector Lab) for peroxidase for 1-2 min. Developed tissue sections were stained with hematoxylin for nucleus, dehydrated, and mounted. For rt-TA (MoBiTec, German, 1:50) staining, fresh frozen tissue was embedded in OCT and sectioned 8 µM thick. The sections were fixed with acetone for 5 min, washed with PBS, and processed for IHC as described above. For immunostaining of NANOG (Abcam 1:1000) and OCT4 (Abcam 1:2000) on the 10th primary tumor tissue, frozen tissue was embedded in OCT and sectioned 8 µM thick. For positive controls, H1 human ES cells were embedded in 4% agarose, snap frozen in OCT, and sectioned 10 µM thick. All sections were fixed with 4% PFA and processed for IHC as above. For immunostaining of NANOG (Abcam 1:1000) and OCT4 (Abcam 1:2000) on 10th primary tumor tissue, frozen tissue was embedded OCT and sectioned 8 µM thick. For positive control, H1 human ES cells were embedded onto 4% agarose, snap frozen in OCT, and sectioned 10 µM thick. All sections were fixed with 4% PFA and processed for IHC as above.

### Teratoma assays

Female 4-6 week old NOD-SCID-IL2Rgc null (NSG) mice (University of Pennsylvania, Xenograft core) (Shultz et al., 2005) were used for subcutaneous injection of human pancreatic iPS-like lines. Briefly, 24h before injection, doxycycline was withdrawn from the culture media. Confluent cells from all wells of a six well plate were detached by rinsing the cells with DMEM/F12, adding collagenase (1 mg/ml), incubating 3 min at 37° C, and collected by centrifugation. The cells were resuspended in 420 µl of complete human ES media and injected subcutaneously in a female NSG mouse. In some cases, 300 µl of the cell pellet was mixed with 120 µl of Matrigel prior to injection; this had no effect on whether the 10-22 cells generated PanINs, but it did cause the lesions to be less dispersed. Twelve weeks (3 months) or 273 days (9 months) after injection, the injection area was collected, fixed with 4% paraformaldehyde, and embedded in paraffin. Paraffin blocks were sectioned and stained with hematoxylin and eosin for analysis.

### Differentiation of H1/iPS-like clones into embryoid bodies

Briefly, cells were harvested from one sub-confluent six well plate using Accutase solution (Innovative Cell Technologies, San Diego, CA) and rinsed with huES media. Clumps of cells were dissociated cells by passing through a 70 µm filter. Dissociated cells were cultured onto ultra-low attachment 6 well plate (Corning, Oneonta, NY) under huES media without Fgf2 and KSR but supplemented with 20% FBS or aggrewell media (Stem Cell Technologies) for 2-3 weeks, then collected for RNA for qRT-PCR.

### Comparative genomic hybridization

Total genomic DNA was isolated from cultured primary cancer and margin epithelial cells and cultures of iPS-like clones by proteinase K/phenol-chloroform. Genomic DNA was amplified using Agilent Oligonucleotide Array-Based CGH for Genomic DNA Analysis enzymatic labeling kit (Agilent, Santa Clara CA), according to the manufacturer. Labeled genomic DNA was cohybridized with human genome CGH Microarray kit 44K (Agilent) by the Penn Microarray facility. Arrays were scanned with an Agilent Scanner System. Data were analyzed by using Partek Genomics Suite (Partek, Saint Louis, MO).

### CpG methylation sequencing

Total genomic DNA was purified from H1 huES cells, 10-12 margin iPS-like cells, and 10-22 cancer iPS-like cells by phenol extraction. Parental primary cancer genomic DNA was isolated from the tissue embedded into paraffin or OCT blocks. Bisulfite conversion with 1 µg genomic DNA was carried out using CpGenome™ DNA Modification Kit (Millipore) as described by the manufacturer. *NANOG* and *OCT4* upstream regions were amplified with 30 ng converted DNA using the primers previously published or designed with PyroMark Assay Design Software (Qiagen, Valencia, CA) (See Table 1 for primers). Amplified PCR products were subjected to bisulfite pyrosequencing as described (Tost and Gut, 2007). Briefly, 10 ul of PCR products amplified with biotin conjugated primers immobilized to Streptavidin Sepharose HP beads (GE Healthcare Bio-Sciences AB, Sweden) for 10 min. The immobilized PCR product was separated into single strand, released onto the Pyromark plate containing sequencing primers in a PyroMark® Q96 Vacuum Workstation (Qiagen) and annealed with sequencing primer by cooling to room temperatures after incubation at 80° C. Pyrosequencing for corresponding CpG sites was done in a PSQ 96 instrument (Qiagen). The percentage methylation at each CpG site was determined using the Q-CpG methylation software (Qiagen).

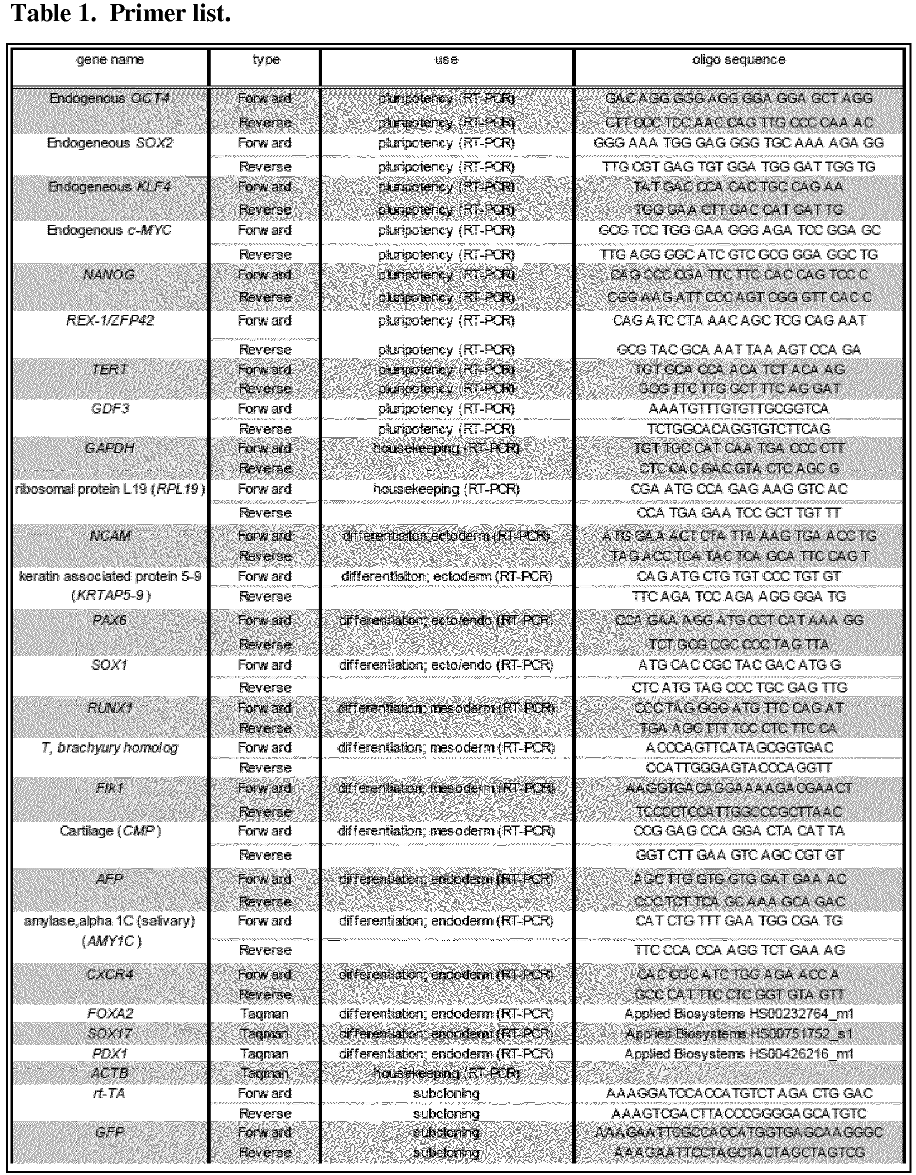

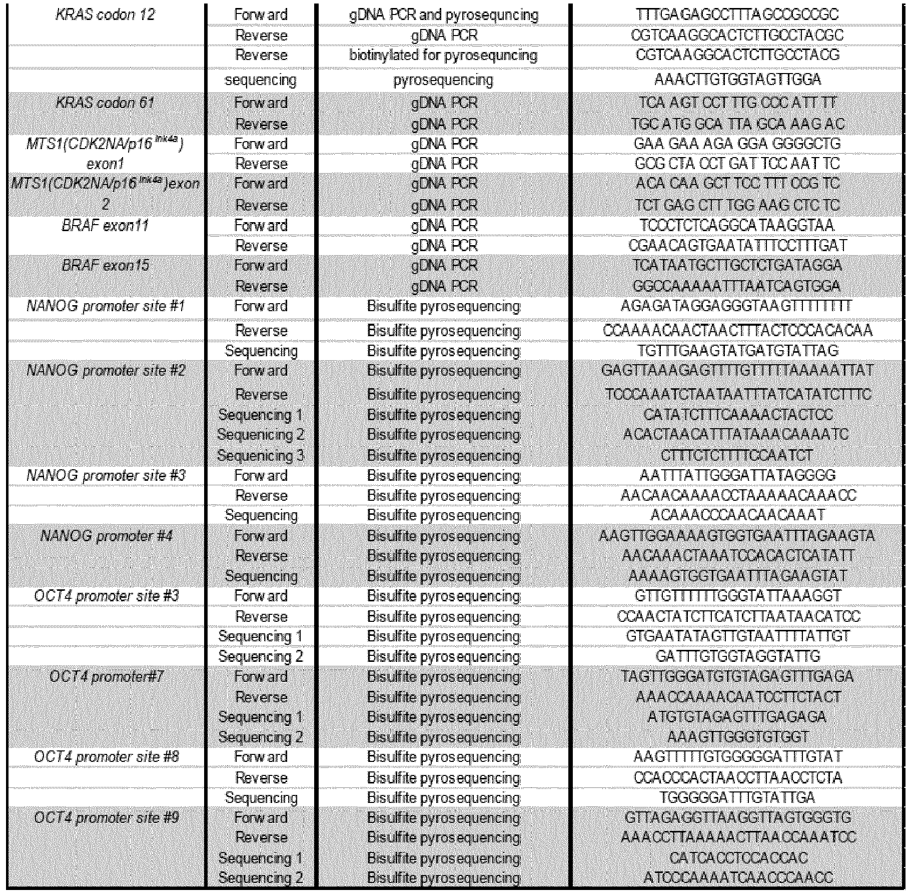

### In vitro culture of 10-22 cancer iPS-like line teratomas

After thirteen to fourteen weeks, teratomas were harvested from NSG mice harboring 10-22 cells. Dissected teratoma tissue was minced and dissociated in liberase T- flex (1.3W/ml) at 37 °C for 30 min. Subsequently, the reaction was quenched and washed with 15% FBS/DMEM. Dissociated cells were plated as either whole cell culture or single cell, passed through 40 µm filter. Dissociated teratoma tissue was embedded as described previously (Lee et al., 2007). Briefly, prechilled 4 well plates (Nunc, Rochester, NY) were coated with a thin layer of 150 µl Matrigel (BD Biosciences) for 15 min at 37 °C incubator. Dissociated cells were pelleted by centrifugation at 1200 rpm for 5 min at 4 °C, resuspended into 200 µl of Matrigel, and incubated at 37 °C for 30 min to allow the cell-Matrigel complex to gel. Explants were fed with 500 µl of serum-free culture media. Six days postplating, serum free-conditioned media was collected, centrifuged to remove cell clumps, supernatant was collected, and kept at -80 °C for proteomic analysis. Explants were fed every 4-5 days for further culturing. To prepare a negative control for proteomic analysis, 10-22 (p10 and p27) iPS-like lines collected mechanically using stem cell passaging tool (Invitrogen) and cultured onto a plate pre-coated with 5 µg/cm2 rat collagen in serum free DMEM media for 2 days. Media was collected, filtered through 0.45 µm filter, and kept at -80 °C for proteomic analysis.

### Proteomic analysis

Frozen media was divided into three tubes; each contained proteins corresponding to 30-60 µg protein. Each sample was precipitated with acetone to concentrate proteins and remove salts and lipid soluble contaminants. Briefly, four volumes of chilled acetone were added to a sample and incubated sample at -20° C for overnight. The samples were pelleted at 16,000 g for 10 min at 4° C followed by washing with a solution of acetone and water (4:1). The pellet was air-dried for 10 min and denaturized by boiling in NuPage LDS sample buffer including reducing agent (Invitrogen) for 5 min. Each sample was subjected into Nupage 10% Bis-Tris Gel and run at 100 mV with Nupage MOPS SDS Running buffer (Invitrogen) for almost 2 hours. The gel was stained with Simplyblue safestain (Invitrogen) according to manufacturer's recommendation for MS analysis. The 5x5 mm pieces were excised and stored in 2% acetic acid solution. The excised gel samples were digested with trypsin (Strader et al., 2006). 5 µl trypsin digested samples were injected with autosampler (Eksigent technologies, Dublin, CA) and a 10 cm C18 column was used to separate the digested peptides. Nano LC (Eksigent) was run at 200 nl/min flow rate for 100 min gradient. Online nanospray was used to spray the separated peptides into LTQ (Thermo Electron) and the raw data was acquired with Xcalibur. Sequest software was used to search database Uniprot and IPI and generated srf files for each sample. Scaffold 3.3 was used to combine and analyze the Sequest generated srf files quantitatively based on spectrum count. Cutoffs was peptide p-value >95% and protein p-value > 99%. To further sort human proteins out of the mouse and bovine, the peptide sequences were blasted using UniProKB TrEMBL (downloaded on Oct, 2011) allowing no mismatch. The human peptides and human and mouse common peptides were used to identify proteins secreted from the teratoma and 10-22 iPS-like line. To distinguish the proteins that have peptides only common in both human and mouse from the protein derived from mouse background, all proteins were subtracted with the proteins secreted from contralateral control. A hierarchical clustering was applied using MeV (using Pearson Correlation as a metric) (data not shown). Proteins secreted from at least two teratoma explants were used for Ingenuity Pathway Analysis (http://www.ingenuity.com).

### PanIN and PDAC mouse model

*Pdx-Cre; LSL-KrasG12D; p53fl*/*+; RosaLSL-YFP* mice (Rhim et al., 2012) aged 2-2.5 and 4-6 months were analyzed for HNF4α expression at the PanIN and tumor stages, respectively. After euthanasia, pancreases were washed and incubated in Zinc-formalin (Polysciences, Warrington, PA) at 4° C for two hours. After washes and dehydration with ethanol, tissues were paraffin embedded and sectioned. After deparaffinization with xylene, tissue sections were antigen retrieved in R-buffer A using a steam retriever (both from Electron Microscopy Sciences, Hatfield, PA). Slides were blocked in 5% donkey serum in 0.1% Triton-X100 in PBS for one hour then incubated with primary antibodies (Chicken α GFP (Abcam, 1:500) and goat α HNF4α (1:200) for one hour at RT. Slides were then incubated with secondary antibodies (FITC conjugated donkey α chicken (1:200; Life Technologies, Grand Island, NY); Alexa Fluor 594 conjugated donkey α goat (1:200; Jackson Immunoresearch, West Grove, PA); DAPI (1:1000)) in block solution for 1h at RT, followed by washes and mounting of coverslips. Images were visualized using an Olympus IX71 inverted fluorescence microscope and captured and merged using Olympus DP Manager software (v.3.1.1).

### INTRODUCTION

Cancer phenotypes can be suppressed in certain medulloblastoma cells, RAS-induced melanoma cells, and embryonal carcinoma cells and renal tumor cells when they are reprogrammed to pluripotency by nuclear transfer (Blelloch et al., 2004; Hochedlinger et al., 2004; Li et al., 2003; McKinnell et al., 1969). The resultant pluripotent cells can then differentiate into multiple early developmental cell types of the embryo. Such embryos die partly through organogenesis, presumably due to re-expression of the cancer phenotype. It is remarkable that, in certain circumstances, the pluripotency network can suppress the cancer phenotype sufficiently to allow early tissue differentiation. Using iPS cell technology (Takahashi and Yamanaka, 2006), cancer cell lines have been made into iPS cells (Carette et al., 2010; Miyoshi et al., 2010). However, no iPS cell lines from solid primary human cancers have been reported. Not limited to one theory, creating iPS cells from an epithelial tumor would allow the cells to be propagated indefinitely in the pluripotent state and that, upon differentiation, a subset of the cells would undergo early developmental stages of the human cancer, providing a live cell human model of early stages of the disease.

### RESULTS

### Creating iPS-like cell lines from human pancreatic ductal adenocarcinoma.

Human pancreatic ductal adenocarcinoma samples were obtained immediately after resection (Table 2). Histologically normal pancreatic tissues at the margin of the specimens were used as controls. Epithelial cells were isolated and cultured in serum-free medium with cholera toxin to impair the growth of fibroblasts. Two successive infections of the pancreatic cancer and margin cells were performed with 5 lentiviruses separately encoding doxycycline-inducible mouse *Oct4, Sox2, Klf4,* and *c-Myc,* and the rt-TA transactivator, while genomic DNA was isolated from the pancreatic specimen margin and cancer epithelial cells that had been cultured separately. Pyrosequencing analysis revealed *KRAS* mutant cells in 7 of 9 of the initial tumor samples (Tables 2, 3). ES-like colonies began to arise after 7 days, with fewer colonies arising from cancer epithelial cells. ES-like clones from cancer or margins started to differentiate and disappear 4 days after withdrawal of doxycycline. Thus, colonies were maintained in the presence of a low doxycycline concentration (50 ng/ml) and called the resulting lines "iPS-like" because of their dependency upon doxycycline. iPS-like lines were established from 4 of the 9 tumor epithelial specimens and corresponding margin iPS-like lines from 3 of the specimens (Figure 1A, Table 2).

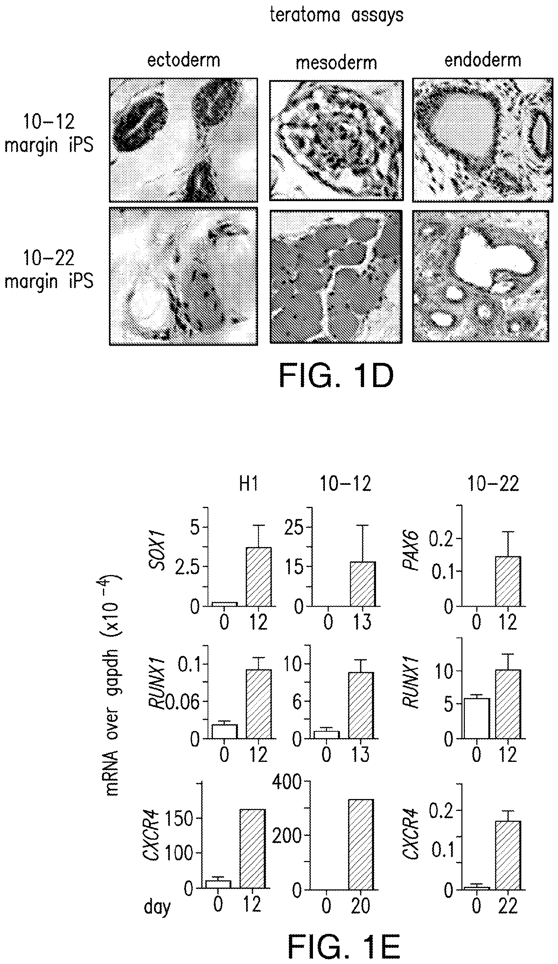

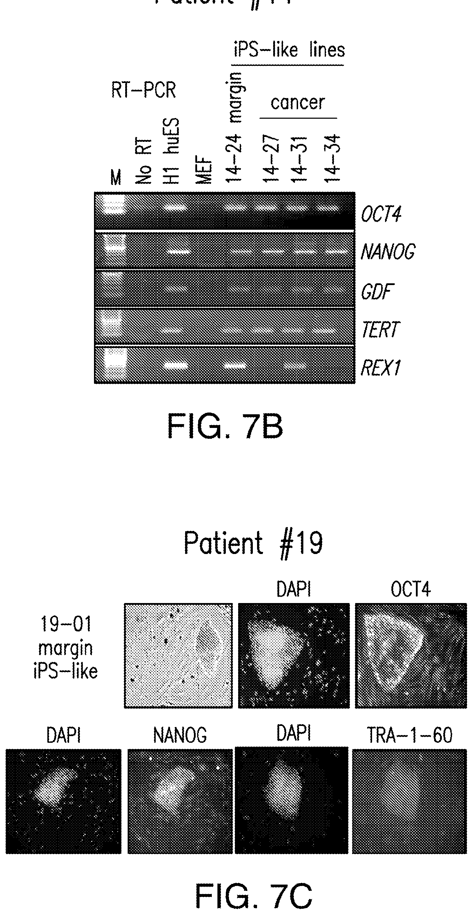

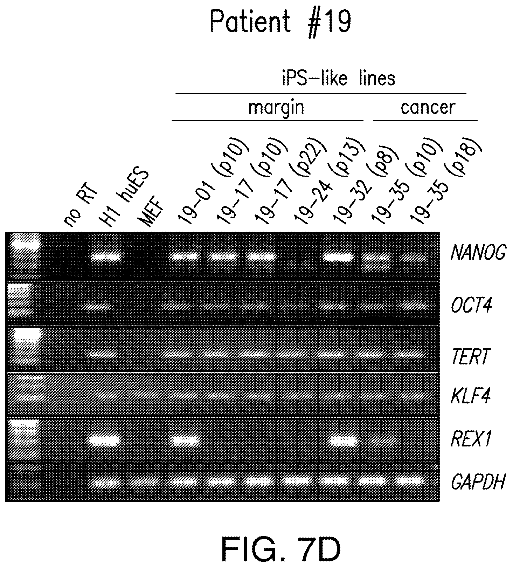

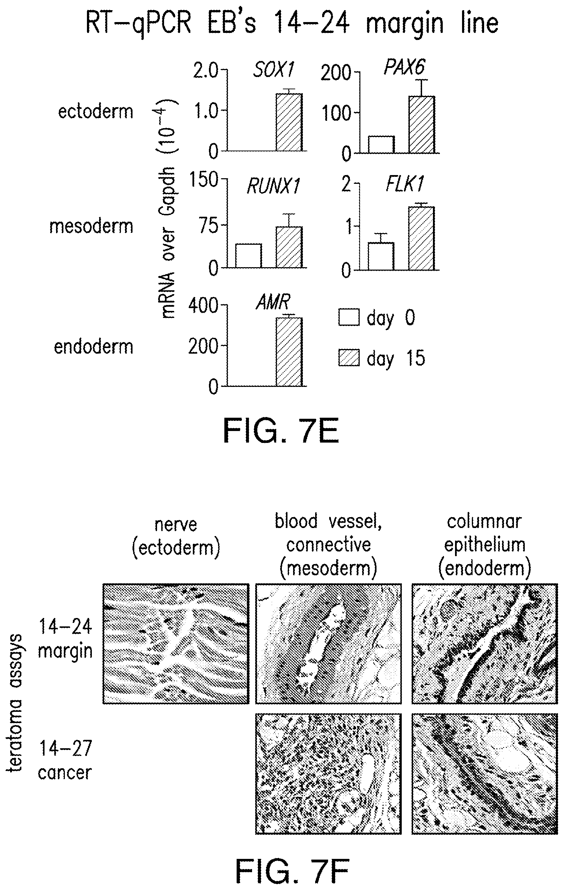

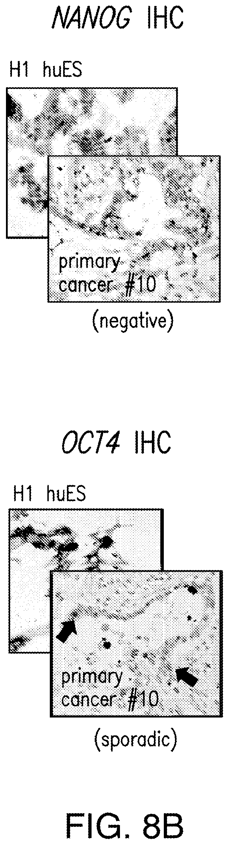

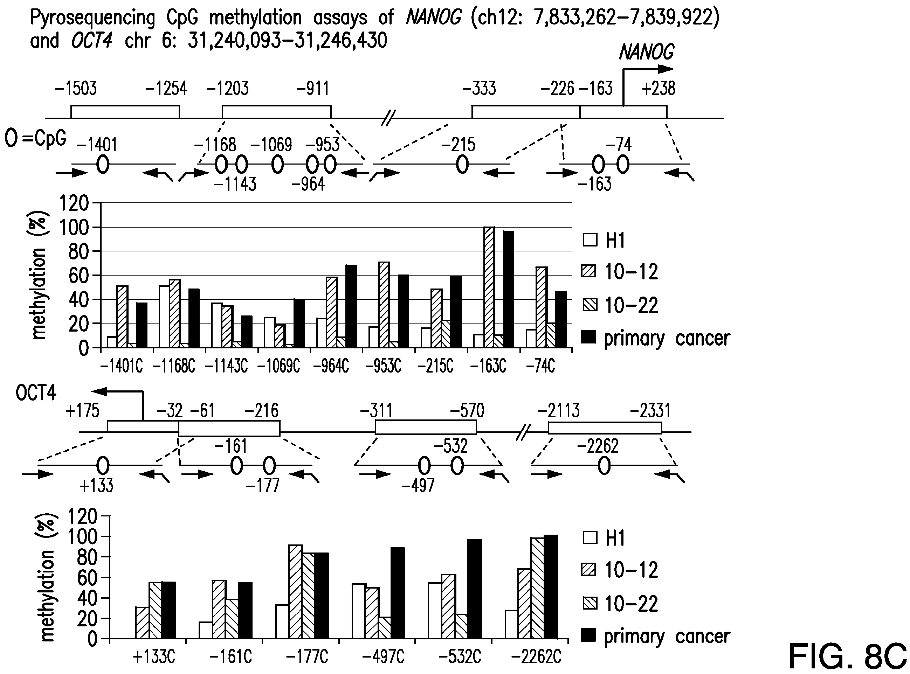

The cells' pluripotency were characterized by RT-PCR, immunostaining, embryoid body formation, teratoma assays, karyotyping, and a subset by CpG methylation analysis. Most iPS-like lines expressed endogenous pluripotency marker RNAs and protein (Figure 1B, C; Figure 7A, B, C and D). Teratoma assays in immunodeficient NSG mice and embryoid body assays revealed that the 10-12 pancreatic margin and 10-22 cancer iPS-like lines from the 10th patient (Figure 1D, E; Figure 8A) and the 14-24 pancreatic margin and 14-27 cancer iPS-like clones from the 14th patient (Figure 7E, F) could generate tissues of multiple germ layers, demonstrating pluripotency. The original tumor #10 was negative for NANOG expression and exhibited sporadic expression of POU5F1/OCT4 (Figure 8B). Detailed pyrosequencing analysis of CpG methylation showed that the 10-22 iPS-like line exhibited demethylation at 9 of 9 sites at the NANOG promoter, compared to the primary tumor, and at 3 of 6 sites at the OCT4 promoter; by comparison, the H1 huES line was similarly demethylated at NANOG and demethylated at 4 of 6 sites at POU5F1 (Figure 8C). Thus, the iPS-like lines exhibit diverse characteristics of reprogrammed pluripotent cells.

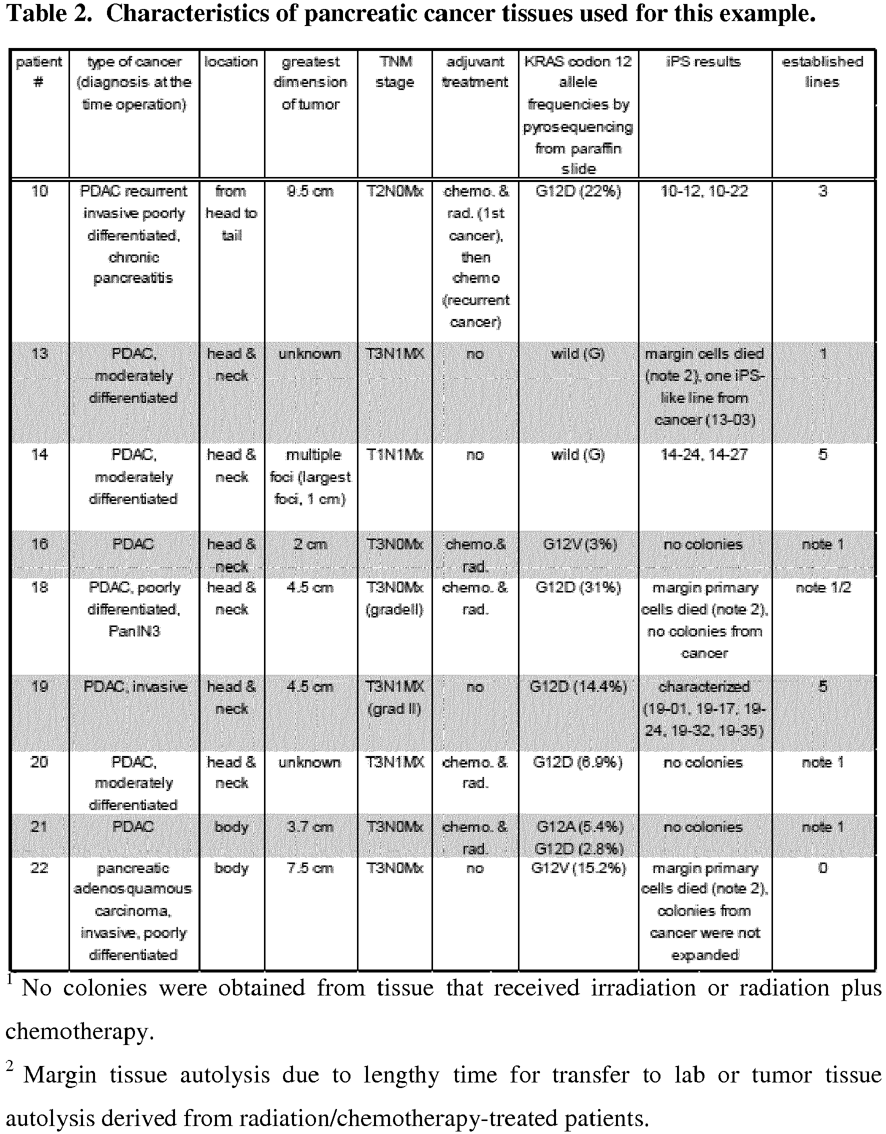

All iPS-like lines were screened for mutations in KRAS, CDKN2A, and BRAF, which are common genetic alterations in pancreatic cancer (Moskaluk et al., 1997) (Table 3). The 10-22 iPS-like line, derived from the recurrent, invasive, and poorly differentiated PDAC of the 10th patient, harbors the same KRAS G12D mutation seen in the initial tumor epithelial population (Figure 1F; Table 2, 3). 10-22 cells also possess a CDKN2A heterozygous deletion (Figure 1H; Table 3) and a comparative genomic hybridization (CGH) pattern with numerous chromosomal aberrations, more exaggerated than that of the primary cancer culture, and a correspondingly aberrant karyotype (Figure 1I; Figure 3). The exaggeration of the primary cancer CGH pattern in the 10-22 iPS-like line is expected because the primary cancer culture contained some stromal cells and thus was contaminated by cells of a normal genotype. Specifically, 23 gross chromosomal aberrations were detected in the PDAC epithelial cell population of the 10th tumor and 20 were represented in the 10-22 line (Fig. 1I, Fig. 9B). Decreased CGH signals in the primary tumor cells and the 10-22 line spanned *PTEN* and *DPC4 (SMAD4),* consistent with observations of allelic loss of these loci in human pancreatic cancers (Hahn et al., 1996; Hahn et al., 1995). By contrast, the isogenic 10-12 pancreatic margin iPS-like line had wild type *KRAS,* a flat CGH chromosomal profile, similar to the parental margin cell culture, and a normal karyotype (Figure 1G, H, I; Figure 9; Table 3). The 10-12 and 10-22 iPS-like lines were derived from pancreatic margin and cancer epithelial cells, respectively, with the 10-22 line harboring the marked genomic rearrangements seen in the initial advanced tumor epithelial population. The iPS-like lines from the 14th patient (Figure 7A, B), with moderately differentiated PDAC containing scattered 1 mm foci, showed point mutations in *CDKN2A* and *BRAF* but a wild type *KRAS,* as with the parental epithelial culture (Tables 2, 3), and flat CGH profiles (data not shown). A pair of margin and cancer derived iPS-like lines from the 19th patient (Figure 7C, D) also had somatic mutations in *CDKN2A,* with wild type *KRAS* and *BRAF* (Table 3). Because the 10-22 cancer iPS-like line from the 10th patient contained the *KRAS* mutant background, typical of PDAC, a detailed study was performed of that line and its isogenic 10-12, *KRAS* wt margin iPS-like clone.

**Table 3. Characteristics of iPS-like lines from human pancreatic cancer and margin samples.**

| patient # | label | source | karyotype (aver.) | CGH | point mutation | | | | | |
|---|---|---|---|---|---|---|---|---|---|---|
| | | | | | KRAS | | CDKN2A | | *BRAF* | |
| | | | | | codon 12 (GGT; gly) | codon 61 (CAA; gln) | exon 1 | exon 2 | exon 11 | exon 15 |
| 10 | 10-12 | margin iPS-like line | 46 | normal | WT | WT | no mutation observed | no mutation observed | WT | WT |
| | 10-22 | cancer iPS-like line | 69 | dislocated | hetero. G12D | WT | hetero. mutation | deletion | WT | WT |
| 14 | Primary tissue/bells | margin tissue | ND | ND | ND | ND | hetero. 5' UTR-49 C>T | no mutation observed | ND | ND |
| | | PanIN2/3 tissue | ND | ND | WT | ND | hetero. 5' UTR-49 C>T | no mutation observed | IVS10 - 18T>G | ND |
| | | PDAC tissue | ND | ND | WT | ND | hetero. 5' UTR-49 C>T | no mutation observed | IVS10 - 18T>G | ND |
| | | primary cancer epithelial culture | ND | normal | WT | WT | hetero. 5' UTR-49 C>T | no mutation observed | IVS10 - 18T>G | no mutation observed |
| | 14-24 | margin iPS- like line | 45 | X- chromosome depletion | WT | WT | hetero. 5' UTR-49 C>T | no mutation observed | IVS10 - 18T>G | no mutation observed |
| | 14-27 | cancer iPS-like line | 44 | normal | WT | WT | hetero. 5' UTR-49 C>T | no mutation observed | IVS10 - 18T>G | no mutation observed |
| 19 | primary tissue | margin | ND | ND | ND | ND | ND | ND | no mutation observed | no mutation observed |
| | | PDAC | ND | ND | WT | ND | ND | ND | no mutation observed | no mutation observed |
| | 19-01 | margin iPS-like line | 45 | ND | WT | WT | Ala28Ala IVS1-69A>G IVS1-31A>T | Ala139Thr | no mutation observed | no mutation observed |
| | 19-17 | margin iPS-like line | ND | ND | WT | WT | ND | Gly127Trp. nonsense mutation (Aa 132 stop codon) | no mutation observed | no mutation observed |
| | 19-24 | margin iPS-like line | ND | ND | WT | ND | ND | no mutation observed | ND | no mutation observed |
| | 19-35 | cancer iPS-like line | 46 | ND | WT | ND | ND | Gly127Trp nonsense mutation (Aa 132 stop codon) | no mutation observed | no mutation observed |

### 10-22 iPS-like cells from advanced human PDAC, upon differentiation, undergo the early stages of pancreatic cancer.

Despite being derived from cancer, the 10-22 cancer iPS-like line as well as its companion 10-12 margin line gave rise to much smaller subcutaneous teratomas, at three months, compared to those seen from the control H1 human ES cells. More striking, there was a much higher proportion of endodermal, DBA lectin-positive ductal structures in teratomas from the 10-12 and 10-22 iPS-like lines (Figures 2A; Figure 10A), whereas H1 cells generated mostly neuronal lineages, as seen previously (Bock et al., 2011). These results are in accordance with reports on the propensity of other iPS cell lines to differentiate into the lineage from which they were derived (Bar-Nur et al., 2011; Kim et al., 2011).

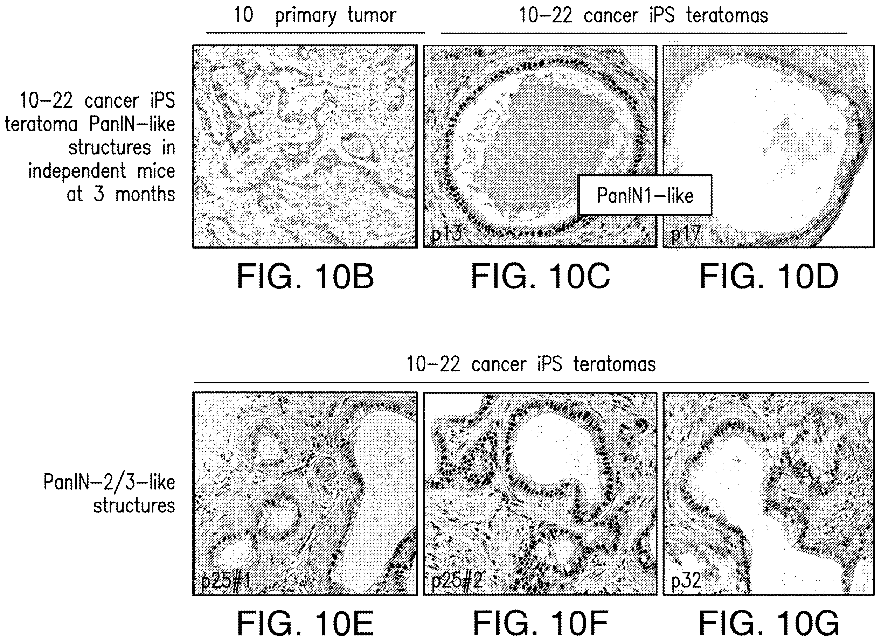

The endodermal teratomas arising from the margin (10-12) and tumor (10-22) iPS lines with the original tumor of the 10th patient were compared. The original tumor exhibited many areas of poorly differentiated foci and infiltration, although occasionally showed a more organized epithelium, but no PanINs (Figure 2B, C; Figure 10B). The tumor epithelium exhibited irregularly shaped and hyperchromatic nuclei and a high nuclear to cytoplasmic ratio (Figure 2C, arrow), along with cytoplasmic protrusions indicative of an invasive phenotype (dotted line in Figure 2C). By contrast, teratoma ductal tissues at 3 months from the 10-22 cancer iPS-like line had a high level of architectural organization, with abundant gland formation and a more differentiated cytology compared to the primary tumor (Figure 2B). This included an increased cytoplasm and smaller, less hyperchromatic nuclei with even nuclear membrane contours, a decreased nuclear to cytoplasmic ratio, and increased cell polarity, indicative of PanIN stages. Abundant mucin was present in the apical aspect of the cells (arrows in Figure 2D). There was no evidence of acinar differentiation or neuroendocrine neoplasia. PanINs are graded by the extent of dysmorphic structures compared to normal ducts (Hruban et al., 2001) and a range of PanIN1-, PanIN2-, and PanIN3-like structures was observed in the histology of the endodermal teratomas from the 10-22 iPS-like line at 3 months; though predominantly structures resembling the PanIN2 and PanIN3 stages (Figure 10C-G). Not limited to a particular theory, these findings suggested that the 10-22 cancer iPS-like line generates ductal structures that resemble PanIN (Maitra and Hruban, 2008).

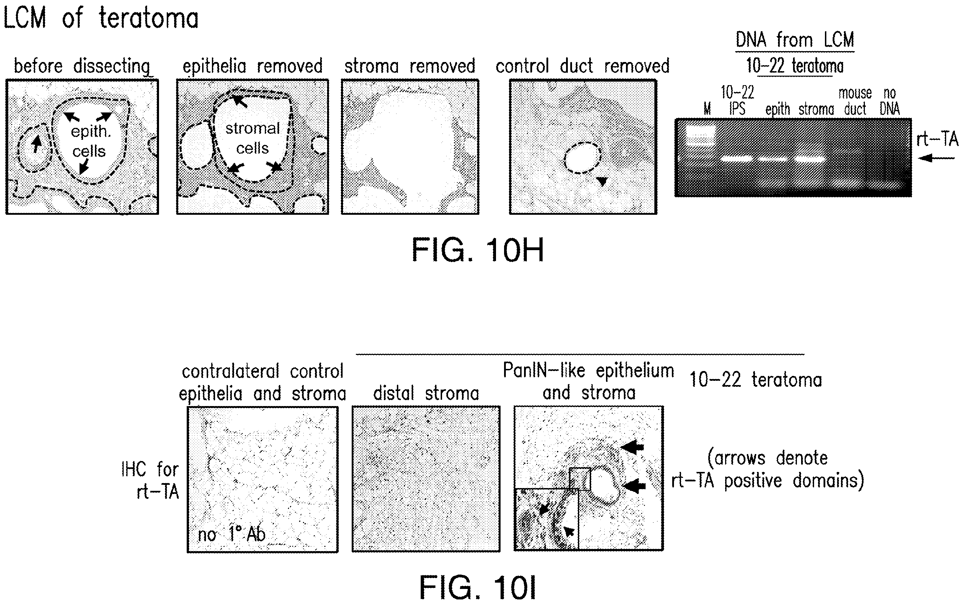

PanIN-like teratomas formed in 9 of 10 teratoma experiments at 3 months, regardless of the passage number of the 10-22 cells. By contrast, the 10-12 pancreatic margin iPS-like line did not generate PanIN-like structures in teratomas (n=4) (Figure 2B). Also, iPS-like lines from the 14th and 19th tumors, containing predisposing mutations, but not of *KRAS,* did not generate PanIN-like lesions (n=5, data not shown), and therefore were not studied further. PCR of DNA obtained by laser capture microdissection showed that the stromal cells surrounding the ductal epithelium in the PanIN-like teratomas of the 10-22 line contained the rt-TA lentiviral DNA, and thus were derived from the starting 10-22 cells (Figure 10H). This was confirmed by detecting rT-TA expression in the PanIN-like epithelium as well as throughout the local stroma, but not in the distal stromal portions of subcutaneous tissue (Figure 10I). Recently, stromal-like cells surrounding PDAC have been found to be derived by EMT from the pancreatic cancer epithelium in a mouse model (Rhim et al., 2012). However, the study observed very few EMT-derived stromal cells at the PanIN stages. Taken together, it seems that most but perhaps not all of the human stromal cells in the PanIN regions of the teratomas were derived by local co-differentiation of the pluripotent 10-22 cells into epithelial and mesenchymal tissues.

Characterizing the ductal structures in teratomas of the 10-22 cancer iPS-like line in further detail, they were found to express keratin 19 (K19) as well as nuclear PDX1, the pancreatic determination factor, and nuclear SOX9 (Figures 3A-F,J; Figure 11A-E). PDX1 is expressed at very low levels in adult pancreatic duct cells, it is not expressed in adult exocrine cells, and it is up-regulated in pancreatitis, PanIN, and PDAC (Miyatsuka et al., 2006). SOX9 is a coordinate effector, with mutant *Kras,* of precursor pancreatic lesions in a mouse model (Kopp et al., 2012). Teratomas from the 10-12 pancreatic margin iPS-like cells did not express the gastric mucin MUC5AC, whereas the PanIN-like structures of the teratomas from the 10-22 cells expressed abundant MUC5AC (Figures 3G-I, K, brown staining; Figure 11A), as observed in PanIN lesions and well differentiated PDAC (Kim et al., 2002). Considering the histology and CGH genomic profile of the parental pancreatic cancer tissue from which the 10-22 iPS-like line was derived, the data indicate that the 10-22 iPS-like line from poorly differentiated, late stage PDAC can differentiate into PanIN lesions associated with the early stage of the disease.

### The 10-22 iPS-like line PDAC progresses to the invasive stage of human pancreatic cancer.

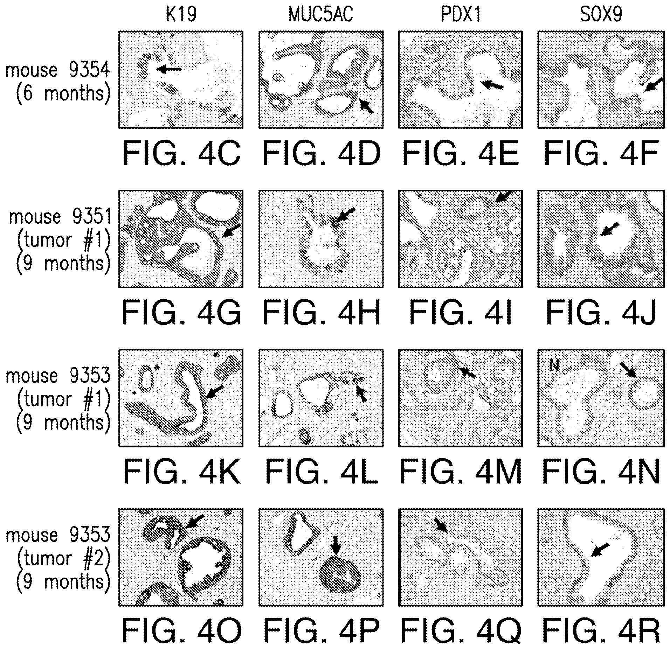

To assess whether PanIN-like structures from the 10-22 line could progress to later stages of PDAC, teratomas grown for 6-9 months in NSG mice were investigated. By 9 months, two solid, palpable tumors arose in each of two injected mice (3-6 mm diameter), all with a genotype characteristic of 10-22 cells (Figure 11F). While palpable tumors were not evident at 6 months, histological analysis showed highly glandular structures with nuclear heterotypia and hypochromia at both 6 and 9 months (Figure 4A, B). The epithelial cells were positive for K19, MUC5AC, PDX1, and SOX9 (Figure 4C-R, arrows). Notably, structures indicative of a locally invasive phenotype were observed (Figure 4A, B; arrows). The PanIN-like stage of teratomas from the 10-22 iPS-like line is succeeded in vivo by the invasive stage of PDAC, indicating that the 10-22 iPS-like model undergoes a spectrum of pancreatic carcinogenesis.

### Cultured organoids of PanIN-like cells from 10-22 line teratomas secrete or release proteins indicative of early stage pancreatic cancer.

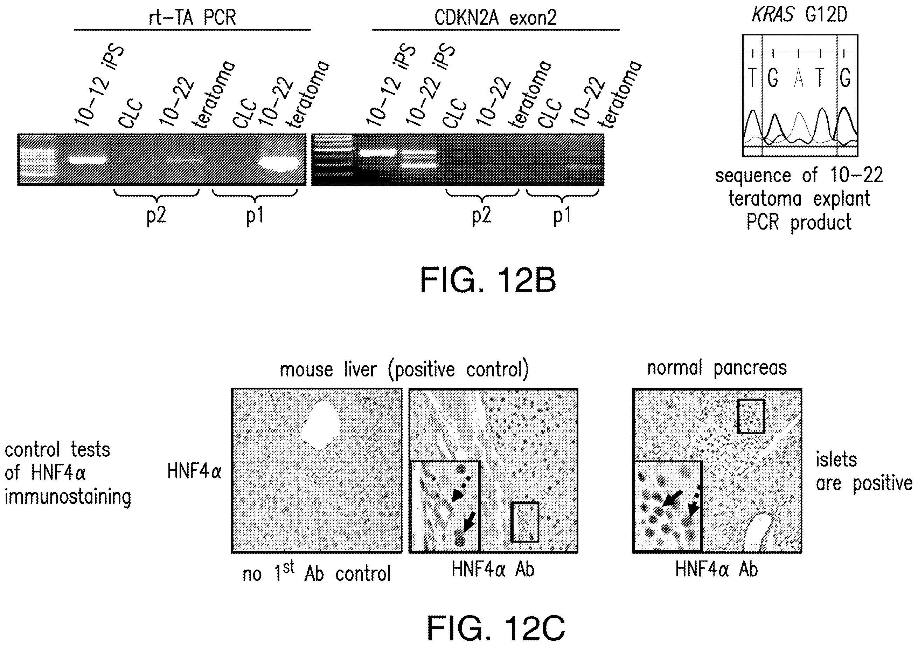

A system where the PanIN-like structures occurring within teratomas from the 10-22 cells could be studied as a live, in vitro model of early stage human pancreatic cancer was generated. Accordingly, tissues from teratomas 3 months after injection, along with contralateral control tissue were harvested, and conditions were established where the tissues were embedded separately into Matrigel and cultured in vitro (Figure 12A). PCR analysis of DNA from the resulting sphere-like organoids confirmed the 10-22 line genotype, which was absent from contralateral control explants (Figure 12B). The organoids from the 10-22 cancer iPS-like cells retained the expression of human K19 and MUC5AC (Figure 5A-C, sections).

Given the poor prognosis of PDAC, the organoid system was used to identify biomarkers and pathways that could permit early detection and facilitate disease monitoring after therapy. NanoLC/MS/MS was used to examine the proteins that were secreted or released from explants of three independent teratomas that were cultured for 6 days (Figure 5D, left). The data were compared to proteins secreted or released from explants of contralateral control tissue cultured similarly and from the parent 10-22 iPS-like line cultured in the undifferentiated state. Peptides with perfect matches to human peptides and that were specific to the human PanIN-like teratoma explants were selected (Figure 5D, right). Of the 25 proteins that were secreted or released from all three 10-22 teratoma explants (Figure 5D, right; Table 4), 8 were previously reported to be expressed at the RNA or protein levels within PanIN, IPMN, and/or PDAC, whereas the remaining proteins have not been reported (Harsha et al., 2009) (Table 5). Of the 82 proteins that were identified from the intersection of pairs of 10-22 teratoma explants (Figure 5D, right; Tables 6-8), 15 proteins have been previously reported in PanIN/IPMN/PDAC (Harsha et al., 2009) (Table 5). While certain mRNAs were previously identified in PanIN, such as PGAM-M and VWF (Buchholz et al., 2005), the corresponding proteins were determined to be secreted or released from cells and remained stable in the medium.

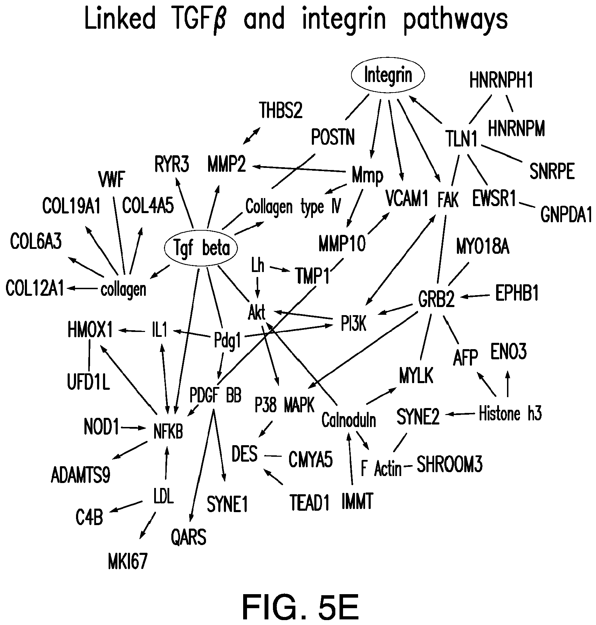

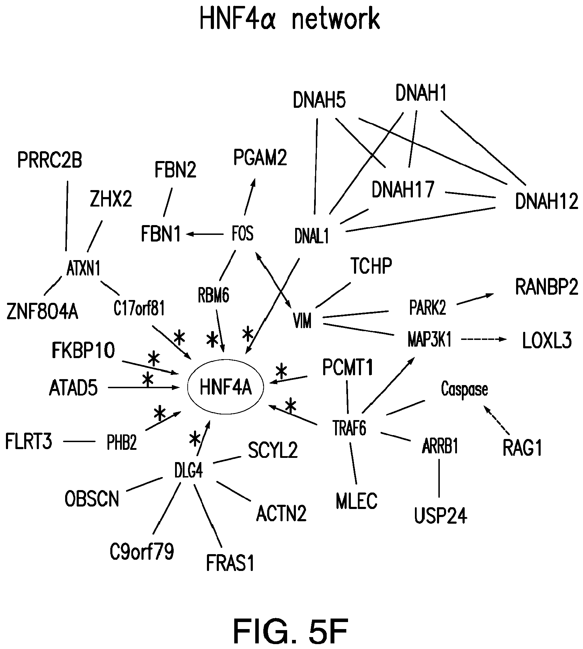

Of the total 107 proteins secreted or released from at least two 10-22 teratoma explants (Figure 5), Ingenuity pathway analysis revealed that 42 fall into interconnected TGFβ1 and Integrin signaling networks (Figure 5E, for 38 proteins, and Table 13). These pathways have been previously reported in PanIN, IPMN, and PDAC (Bardeesy et al., 2006; Jones et al., 2008). Additionally, 7 proteins that were secreted or released from the teratoma explants were identified as falling into the interconnected Ras/p53/JUN/CTNB1 signaling pathway (Table12). In summary, numerous proteins were discovered that are secreted or released from the 10-22 live cell model of early stage human pancreatic cancer, as well as evidence of well-documented pathways involved in early cancer progression.

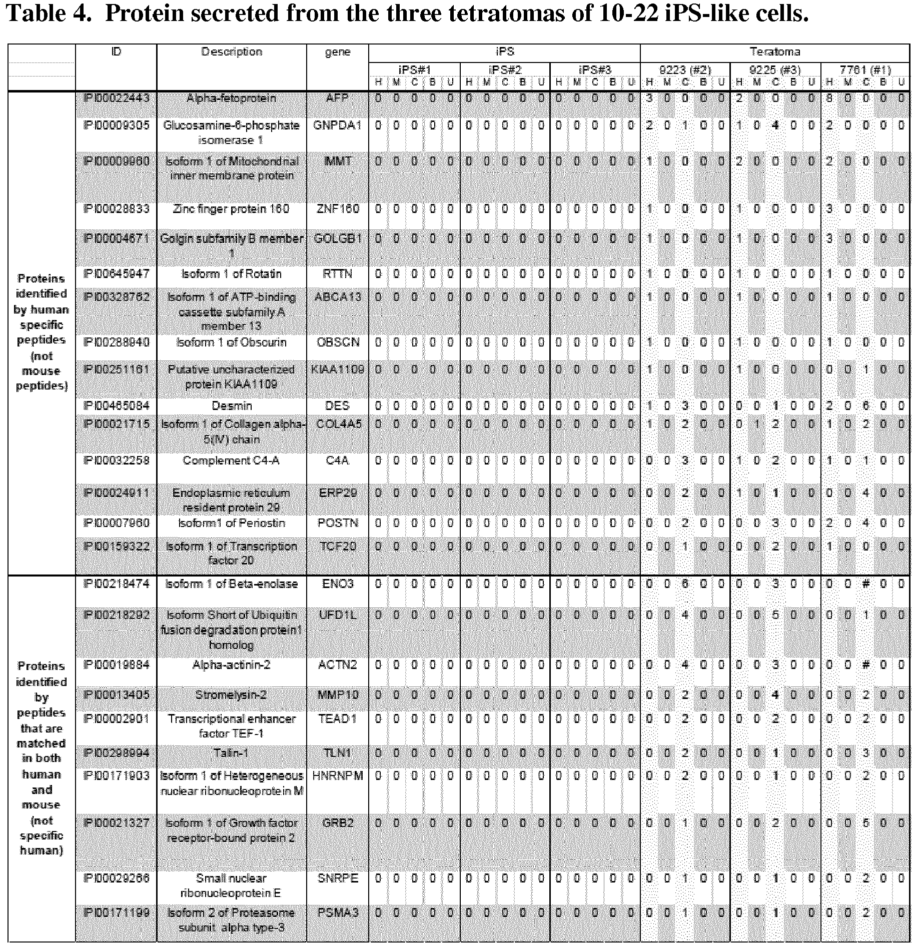

Numbers highlighted indicate the number of peptides providing a protein identification only in conditioned medium from explants cultured in serum-free medium from three independent tetratomas of 10-22 iPS-like cells (mouse #9223, 9225 and 7761), and not in media from explants of contralateral control tissue or in media from the 10-22 iPS-like line cultured under pluripotency conditions. Total is 25 proteins. H-human specific peptide, M-mouse specific peptide, C-human and mouse peptide, B-bovine specific, U-species other than human/mouse/bovine.

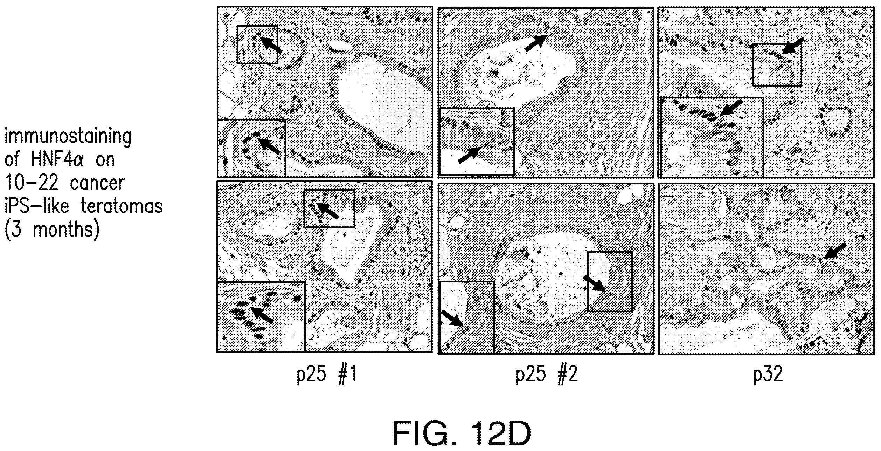

**HNF4α network activated in early to intermediate, but not late stage pancreatic cancer.** Further Ingenuity analysis revealed that another 25 of the secreted or released proteins were in a network observed to be centered on the transcription factor HNF4α, including numerous direct gene targets (Odom et al., 2004) (Figure 5F, Table 9 and 10). Nine of the proteins in the HNF4α network were also secreted into the plasma during the PanIN stage of a mouse PDAC model (Table 9) (Taguchi et al., 2011). Although HNF4α has not been reported in the development or progression of PDAC, by searching databases, it was noted the amplification of the HNF4α locus (Maser et al., 2007) and the up-regulation of HNF4α mRNA (Iacobuzio- Donahue et al., 2003; Logsdon et al., 2003) in human PDAC. Although some reports show HNF4α in pancreatic acinar cells as well as islets in adult mice (Gupta et al., 2007), HNF4a was found to be predominantly expressed in islets, with very low or no expression elsewhere in the normal pancreas (Figure 12C). Strikingly, while HNF4α was not expressed in normal human or mouse pancreatic ducts (Figure 6A; Figure 12C), it was expressed in the nucleus of 10-22 teratoma PanIN-like cells at 3 months and invasive stage cells at 9 months (Figure 6B,C; Figure 12D). HNF4α was detected cytoplasmically in moderately differentiated domains of the original tumor of the 10th patient, from which 10-22 cells were derived, but not in undifferentiated portions of the tumor (Figure 6D).

To more quantitatively assess HNF4α expression at different stages of human pancreatic cancer, a tissue microarray was used to assess multiple samples of human PanINs at different stages as well as samples of pancreatic cancer. HNF4α was barely detected in the nuclei of normal pancreatic ducts and very weakly in the samples of PanIN-1 cells, but exhibited a statistically significant increase in nuclear expression in the samples of PanIN-2 (p<0.05) and stronger and more uniform expression in PanIN-3 epithelia (p<0.05) (Figure 6E, G, J; Table 10). HNF4α was most frequently detected in well differentiated mucinous sections of PDAC (p<0.01) and barely or not detectable in undifferentiated or poorly differentiated epithelial structures of PDAC (Figure 6H-J), as seen in the original patient #10 tumor (Figure 6D). It was also note that in the human protein atlas (Uhlen et al., 2010), HNF4α appeared positive in well differentiated epithelial structures of PDAC but either cytoplasmic or not expressed in poorly differentiated or undifferentiated epithelial structures of PDAC, and not expressed in metastatic PDAC.

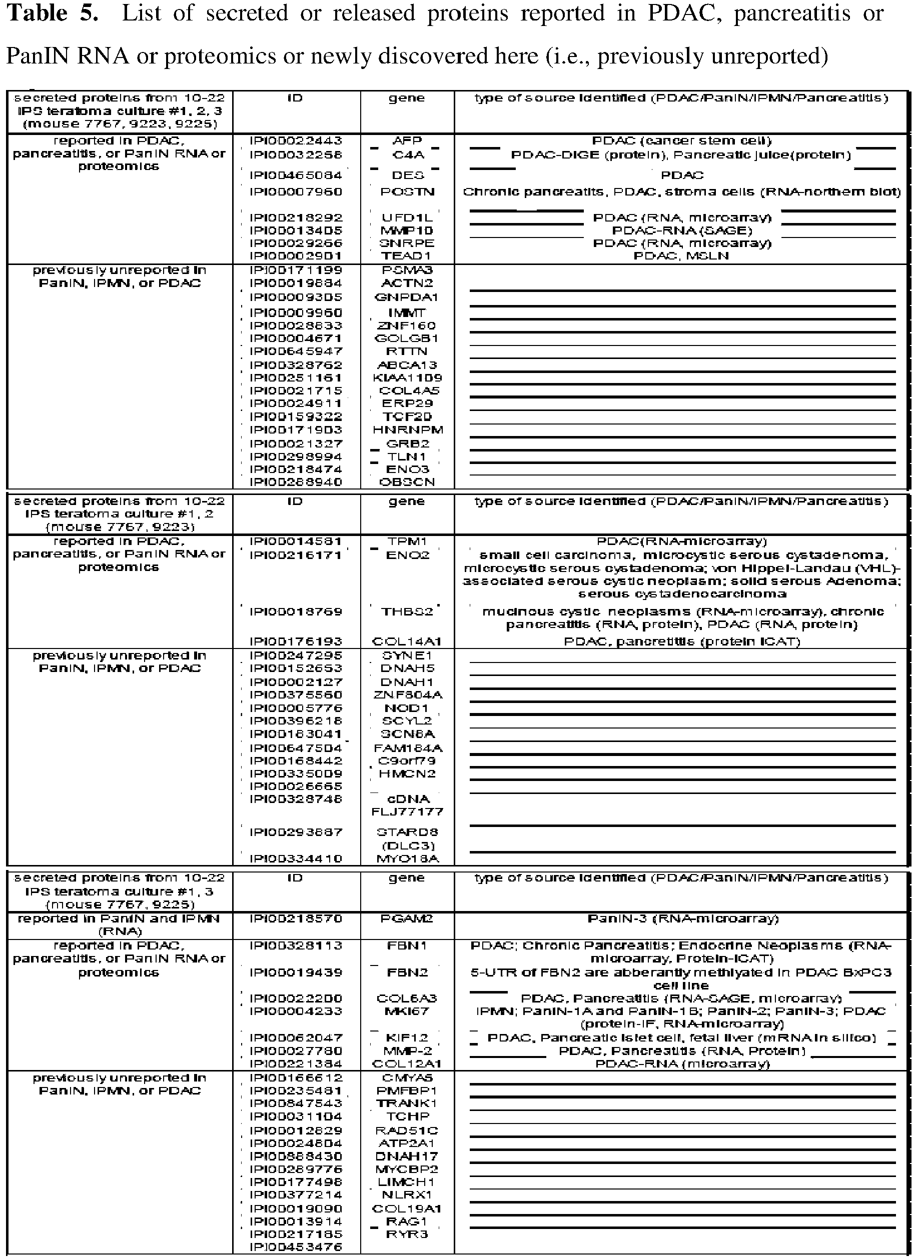

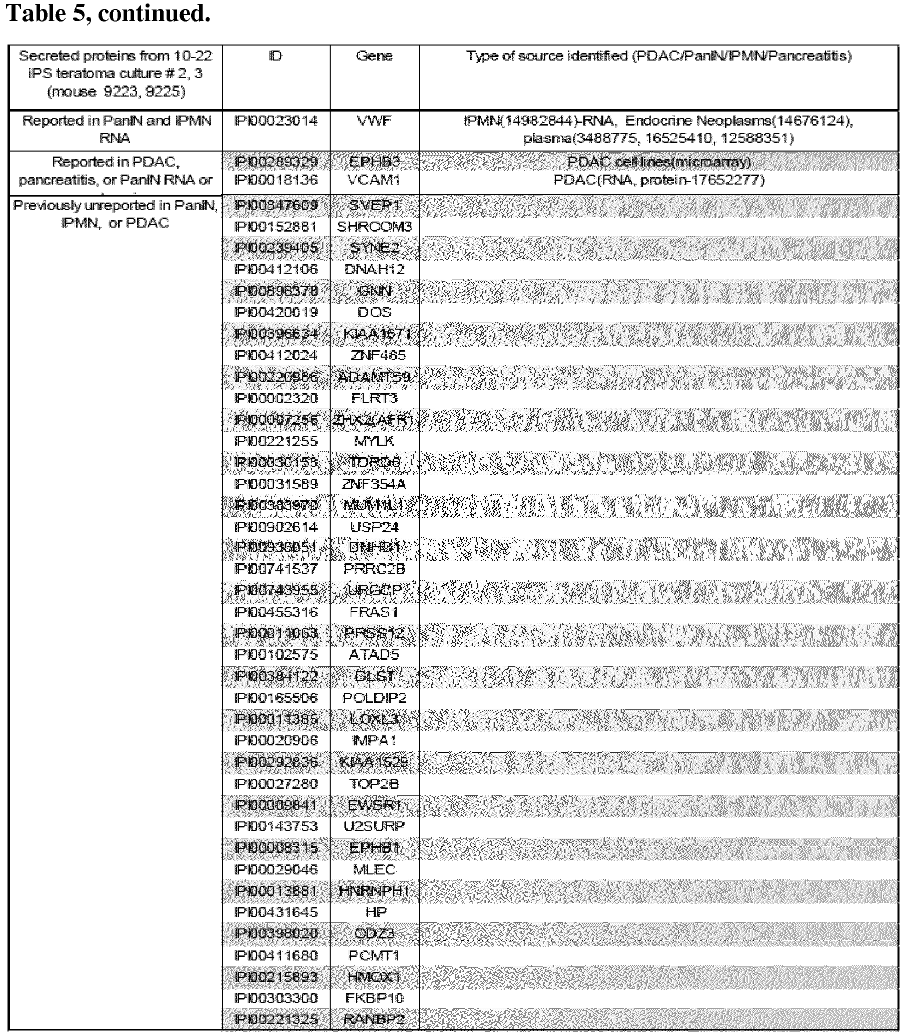

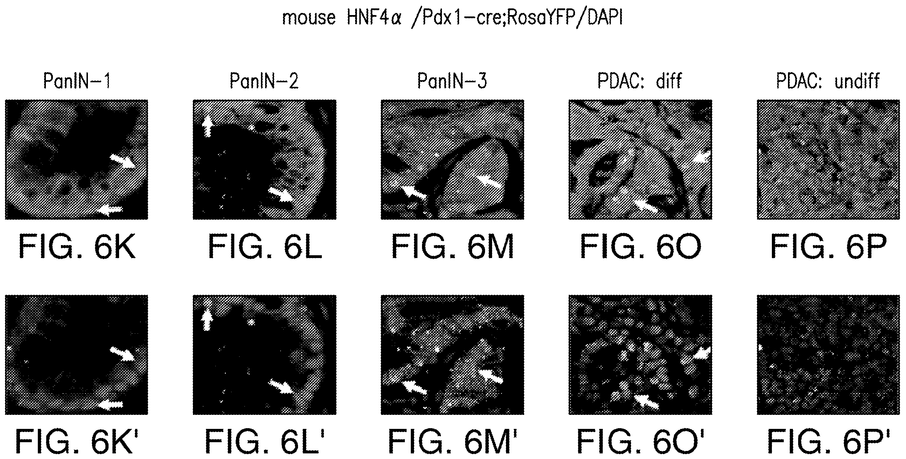

HNF4α was assessed in a mouse model of PDAC arising in a *KrasG12D;p53L*/+*; Pdx1- Cre; RosaLSL-YFP* background (Rhim et al., 2012). As in humans, HNF4α was sporadically expressed at the PanIN-1 stage (Figure 6K), it was expressed in most nuclei of PanIN-2 (Figure 6L) and PanIN-3 lesions (Figure 6M), and observed in nuclei of the more differentiated portions of the murine tumors but not in the undifferentiated portions (Figure 6O, P; white arrows). In conclusion, the ability of the 10-22 iPS-like cells to undergo early stages of human pancreatic cancer were validated by their morphology, histology, secreted or released proteins, and using them to discover a previously unappreciated network associated with early to invasive stage pathology in human clinical samples and a mouse model of the disease.

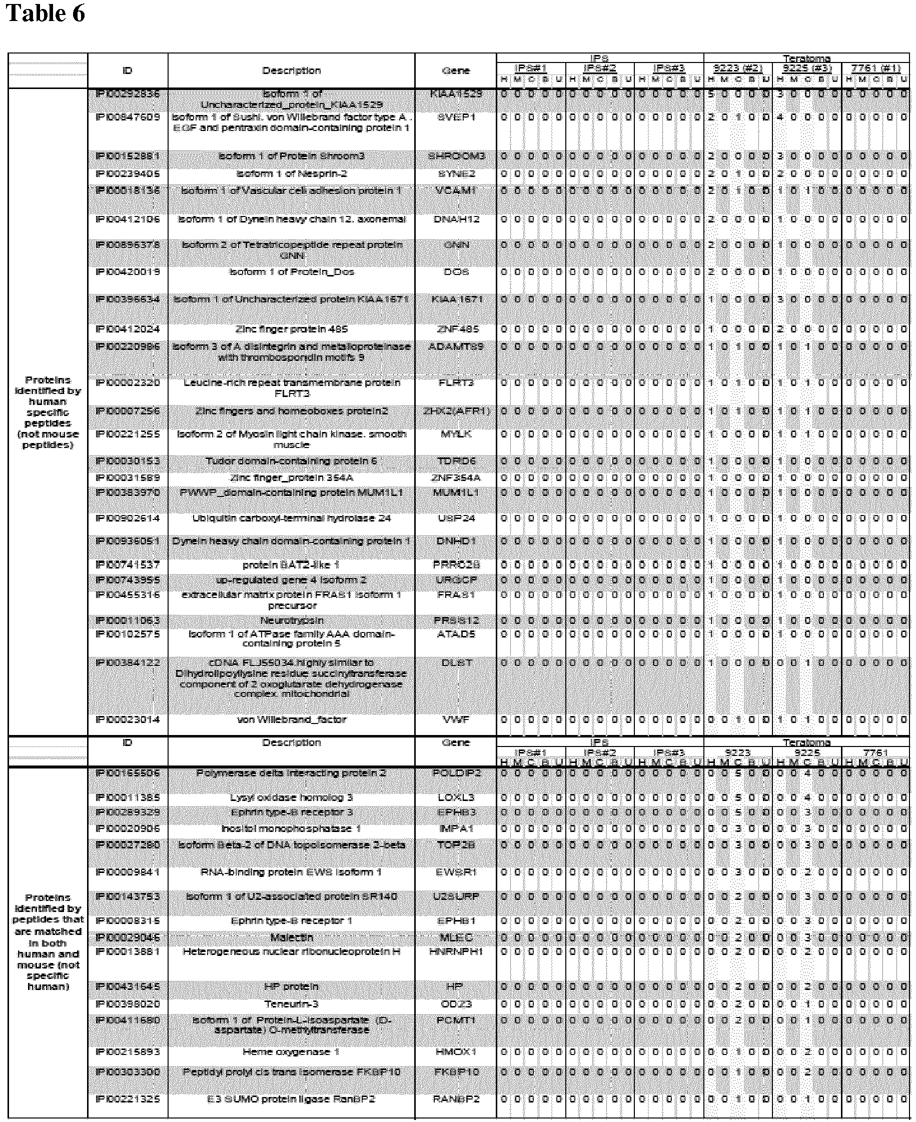

Numbers highlighted indicate the number of peptides providing a protein identification only in conditioned medium from explants cultured in serum-free medium from two independent tetratomas of 10-22 iPS-like cells (mouse #9223 and 9225), and not in media from explants of contralateral control tissue or in media from the 10-22 iPS-like line cultured under pluripotency conditions. Total is 42 proteins. H-human specific peptide, M-mouse specific peptide, C-human and mouse peptide, B-bovine specific, U-species other than human/mouse/bovine.

### DISCUSSION

There has been an absence of live human cell models of PDAC progression and consequently little information about proteins that could serve as released biomarkers and pathway indicators for early stages of the disease. When human PDAC or pancreatic cancer stem cells are grafted into immunodeficient mice, tumors rapidly arise that resemble the advanced PDAC stages from which the cells were derived and they do not undergo the slow growing phenotype of PDAC precursors. Based on the ability of certain cancer cells to be reprogrammed to pluripotency by nuclear transfer and then to undergo early mammalian development, it was hypothesized that pluripotent stem cell lines from human pancreatic tumors might have the capacity to progress through early developmental stages of the cancer. This would provide an opportunity for discovering intrinsic processes and secreted protein biomarkers of live, early-stage human cells for a devastating cancer. Indeed a rare, single pancreatic cancer iPS-like line, 10-22 cells, can provide novel insights into human cancer progression.

The ectopic expression of Oct4, Sox2, Klf4, and c-Myc and a pluripotent-like state suppressed the cancer phenotype. As previously observed, the pluripotency epigenetic environment can dominate over certain oncogenic states (Lonardo E, 2011). In nuclear transfer studies, only certain cancer cells are amenable to reprogramming (Blelloch et al., 2004; Hochedlinger et al., 2004; Li et al., 2003) and, similarly, one iPS-like line was obtained from pancreatic cancer harboring a *KRAS* mutation, the predominant driver of PDAC. While the *KRAS* mutation induces MAPK signaling, which can trigger mouse ES cells to differentiate (Kunath et al., 2007), in human ES cells, MAPK signaling can promote self-renewal (Eiselleova et al., 2009). Oncogenic *RAS* induces cellular senescence by the accumulation of p53 or CDKN2A (Serrano et al., 1997) and the expression of the four reprogramming factors also triggers senescence by inducing p53 and CDKN2A, thereby impairing reprogramming (Banito et al., 2009). Only patient #10 had a deletion in exon2 of CDKN2A, possibly explaining how the 10-22 cells could escape a senescent phenotype. Additional mutations could have arisen in the 10-22 cells that made the cells particularly amenable to iPS formation.

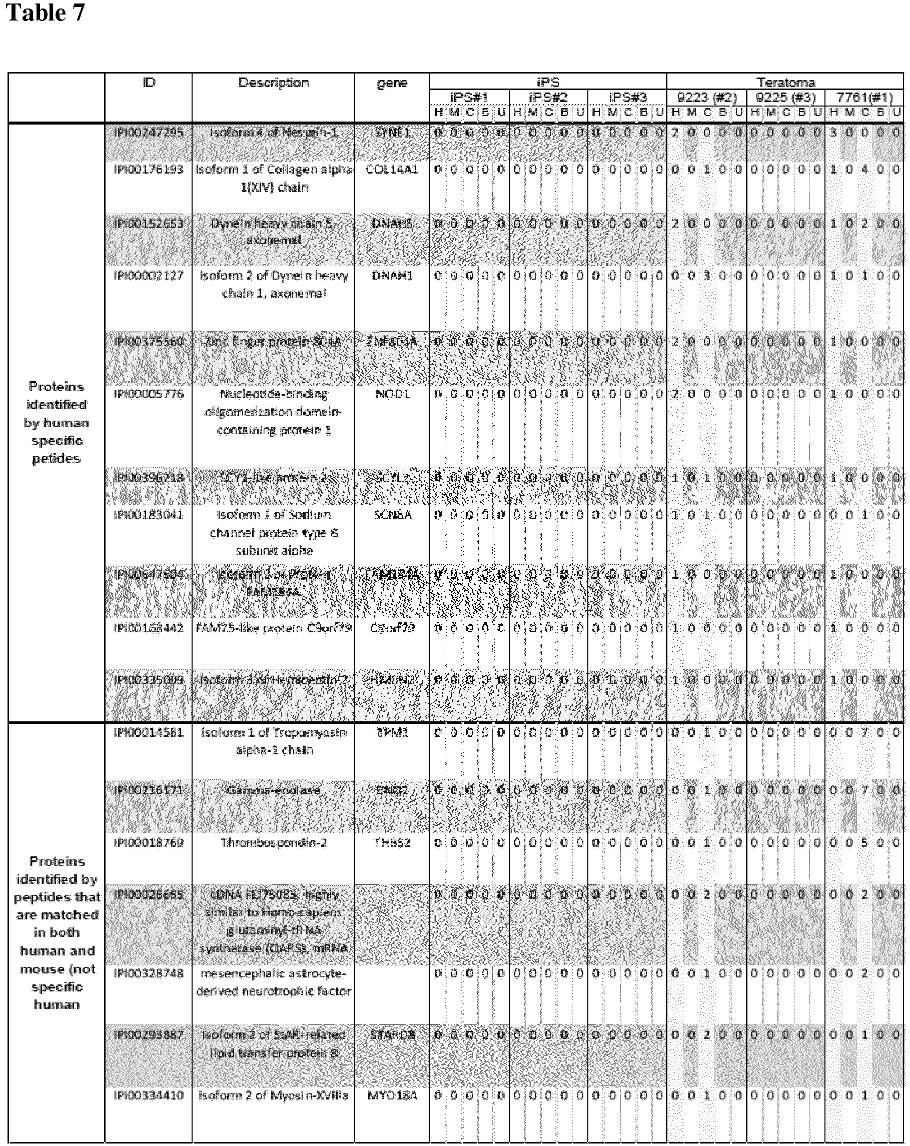

Numbers highlighted indicate the number of peptides providing a protein identification only in conditioned medium from explants cultured in serum-free medium from two independent tetratomas of 10-22 iPS-like cells (mouse #9223 and 7661), and not in media from explants of contralateral control tissue or in media from the 10-22 iPS-like line cultured under pluripotency conditions. Total is 18 proteins. H-human specific peptide, M-mouse specific peptide, C-human and mouse peptide, B-bovine specific, U-species other than human/mouse/bovine.

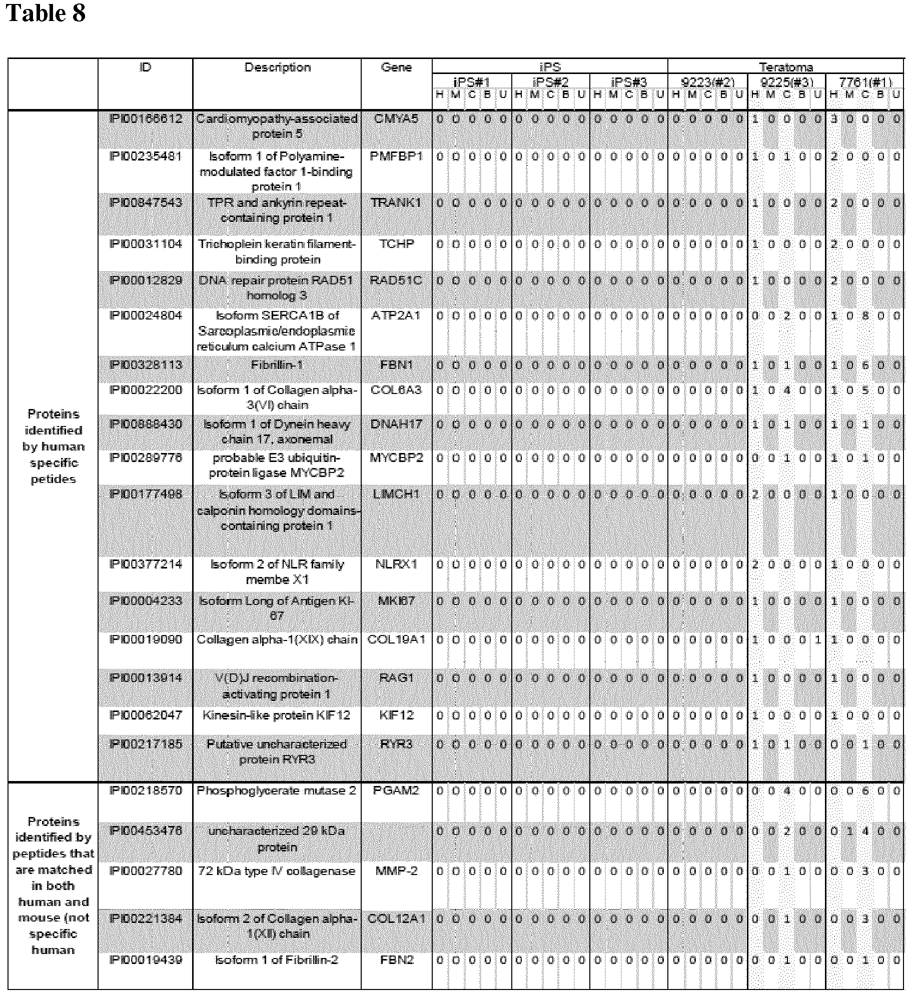

Numbers highlighted indicate the number of peptides providing a protein identification only in conditioned medium from explants cultured in serum-free medium from two independent tetratomas of 10-22 iPS-like cells (mouse #9223 and 7661), and not in media from explants of contralateral control tissue or in media from the 10-22 iPS-like line cultured under pluripotency conditions. Total is 22 proteins. H-human specific peptide, M-mouse specific peptide, C-human and mouse peptide, B-bovine specific, U-species other than human/mouse/bovine.

The release from pluripotency allowed the cancer genome to be expressed in a stage-specific fashion, as opposed to undergoing an immediate regression to the late stage phenotype. Release from pluripotency is normally accompanied by the development of germ layer cells and then specialized tissues, which may continue to dominate, epigenetically, over the resident cancer genome (Blelloch et al., 2004; Hochedlinger et al., 2004; Li et al., 2003). The 10-22 cells from PDAC generated diverse tissue types in teratomas as well as pancreatic ductal tissue that exhibited PanIN lesions and later progression. The apparent preference for pluripotent cells to regenerate the cancer type from which they were derived reflects the tendency of iPS cell lines in general to preferentially differentiate into their lineages of origin (Bar-Nur et al., 2011; Kim et al., 2011). Several lines of evidence indicate that the 10-22 iPS-like line is derived from PDAC. First, the pathology of the original, recurrent tumor was that of PDAC and the CGH profile of the bulk population of cultured cells, which had a highly disrupted genome, was represented in the CGH profile of the 10-22 iPS-like line (Figs. 1I, 9B). While the tumor harbored pockets of more differentiated epithelial cells amidst a vast majority of undifferentiated cells, and therefore it is not certain which type of cell was immortalized in the 10-22 line, the 10-22 cells' disrupted genome does reflect that of a typical epithelial cell in the recurrent tumor. Second, the PanIN-like structures from the 10-22 cells' teratomas expressed SOX9 (Figure 11B-E), which is required for early, *KrasG12D*-dependent pancreatic precursor lesions in a mouse model (Kopp et al., 2012), as well as PDX1, a definitive pancreatic cancer epithelial marker. Thus the ductal lesions from the 10-22 cells are of a pancreatic type. Third, teratomas at 9 months from 10-22 cells progressed to the histology and locally invasive characteristics of later stage PDAC (Fig. 4). Thus, the 10-22 line was not from an early stage cell that would solely undergo an early stage phenotype. Not limited to a particular theory, the evidence indicates that the 10-22 iPS-like line is from PDAC cells in the original tumor and that, upon re-differentiation in teratomas, it undergoes progression of the disease. This is unlike other human PDAC lines, which exhibit late stages of cancer (Lieber et al., 1975; Yunis et al., 1977).

Pluripotency genes such as *NANOG* are expressed in sphere cultures of pancreatic cancer stem cells (CSCs), suggesting that such cells might be more susceptible to reprogramming (Lonardo et al., 2011). However, CD133+CXCR4+ pancreatic CSCs are not enriched and the expression of pluripotent genes is not observed in the adherent culture conditions used to derive the 10-22 cells (Hermann et al., 2007). Also, the OCT4 and NANOG pluripotency genes were highly methylated in the parental tumor #10 epithelium cultures, in contrast to the 10-22 cell line and the huES H1 control, and NANOG itself was not expressed in the primary tumor, although OCT4 was expressed sporadically (Fig. 8B, C). Not limited to a particular theory, it seems unlikely that 10-22 cells were derived from pancreatic CSCs. In addition, pancreatic CSCs (Hermann et al., 2007; Ishizawa et al., 2010; Li et al., 2007) rapidly generate aggressive tumors that represent the primary tumors; whereas the 10-22 cells generate slow growing PanINs (Figs. 3, 10). Finally, tumors generated with pancreatic CSCs give rise to both cytokeratin negative and positive cells in the resultant tumors, in contrast to the homogenous K19 positive staining in PanIN-like ducts at 3 month teratomas (Figs. 3, 8A). Thus, 10-22 cells appear not to exhibit properties of pancreatic cancer stem cells.

The proteins released or secreted from the PanIN-like teratomas fell into at least 3 major networks, including inter-connected networks for TGFβ and integrin signaling that suppress PDAC progression (Hezel et al., 2012). For the first time, the activation of an HNF4α network distinctive for the late PanIN to invasive stages was discovered. HNF4α is not or barely expressed in normal pancreatic ductal cells, poorly expressed in the PanIN1 stage, but is activated in PanIN2 and PanIN3 stages, invasive stages, and in early well-differentiated human pancreatic cancer. HNF4α levels then decrease markedly in advanced or undifferentiated PDAC. It was found that these expression states also occur in a mouse model of PDAC progression. Dynamics in HNF4α expression affect the oncogenic transformation of liver cells (Hatziapostolou et al., 2011). It remains to be determined whether the expression of HNF4α and its target genes is a cause or consequence of pancreatic cancer progression. Yet considering that pancreatic cancer is typically discovered in advanced or metastatic stages, activation of HNF4α and the release or secretion of proteins from the factor's target genes specifically in the late PanIN stages should provide useful diagnostics.

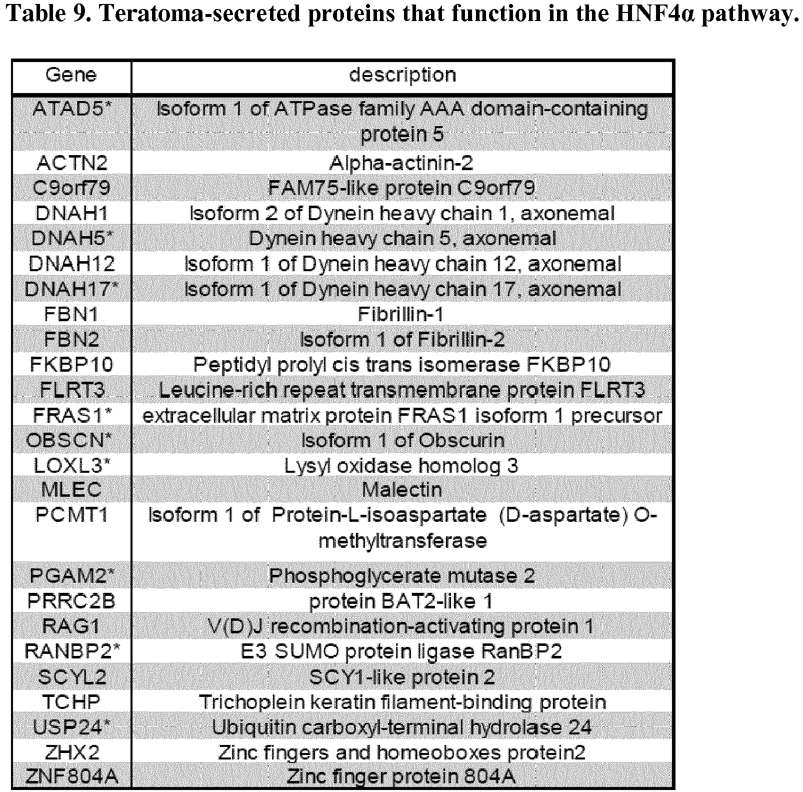

Proteins that are directly or indirectly regulated by HNF4α, out of the secreted proteins from at least two 10-22 teratoma explants embedded into Matrigel.

Of 107 proteins reproducibly released or secreted from PanIN-like cells derived from the 10-22 line, a total of 68 proteins overlap with genes, proteins and networks expressed in human PanIN and PDAC (Tables 10, 11, 12 and 13), further validating the origin of the cells. In addition, it is shown that such proteins are released from the cells and stable, thus serving as biomarkers of early PDAC. A subset of the secreted proteins could be from locally activated stromal cells in the explants. Not limited to a particular theory, the combined detection of released proteins that are the products of the HNF4α, TGFβ and integrin networks within PanIN and invasive PDAC cells provide means for noninvasively detecting the progression of pancreatic cancer in humans.

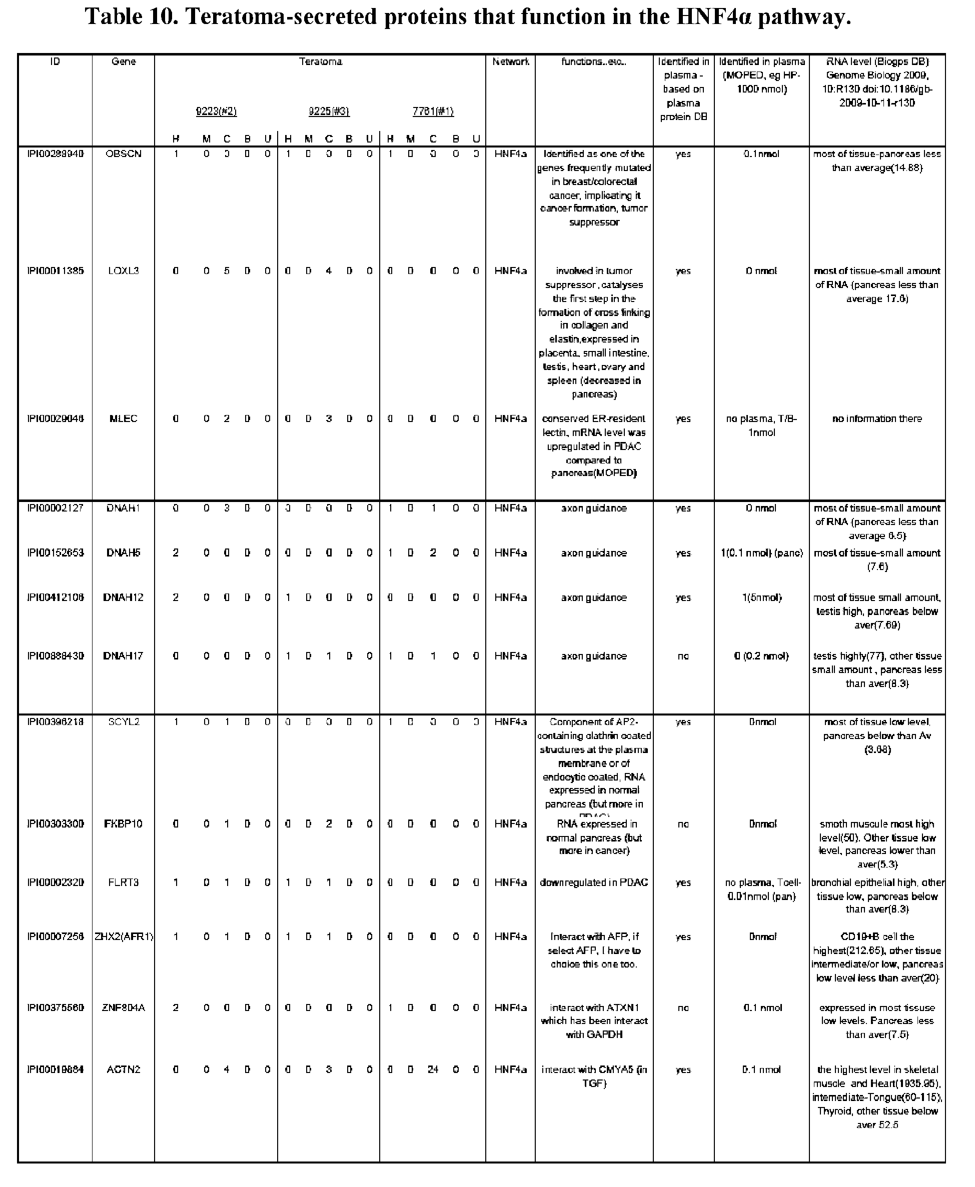

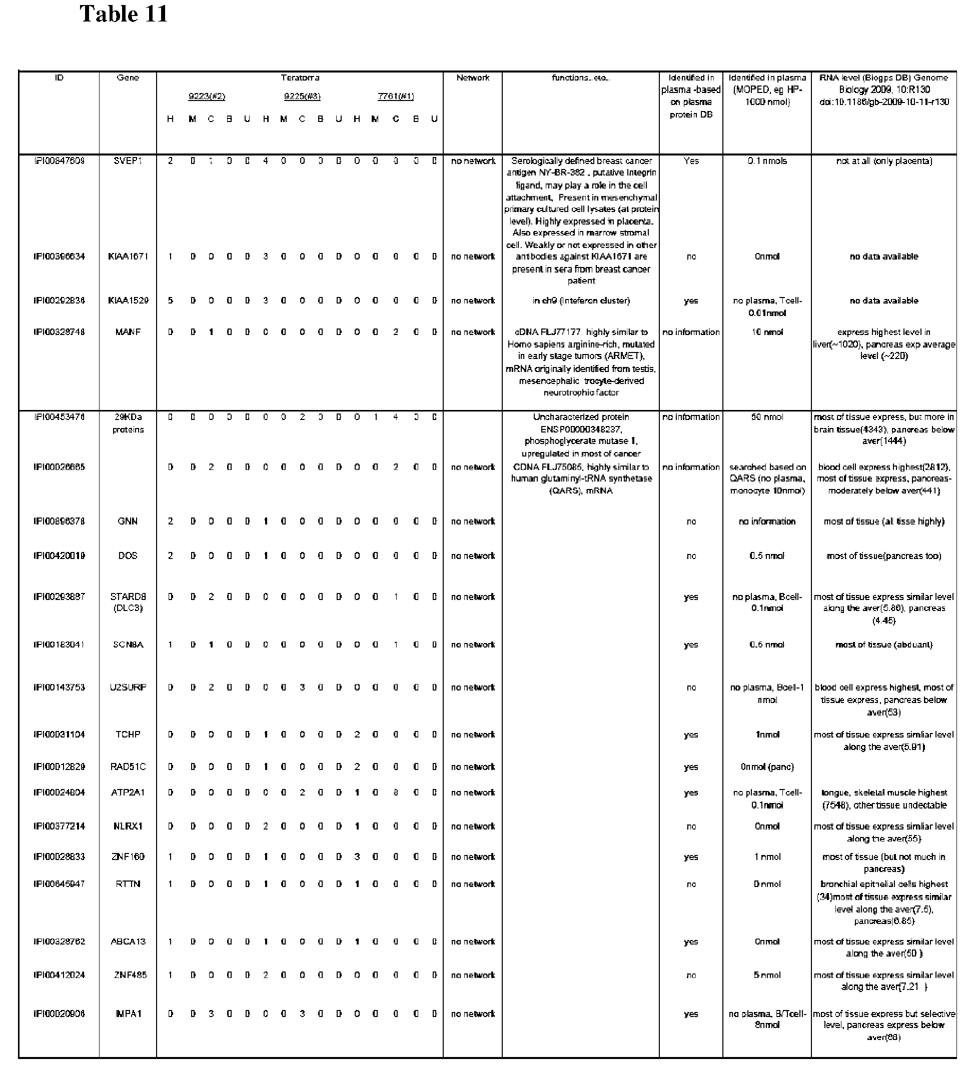

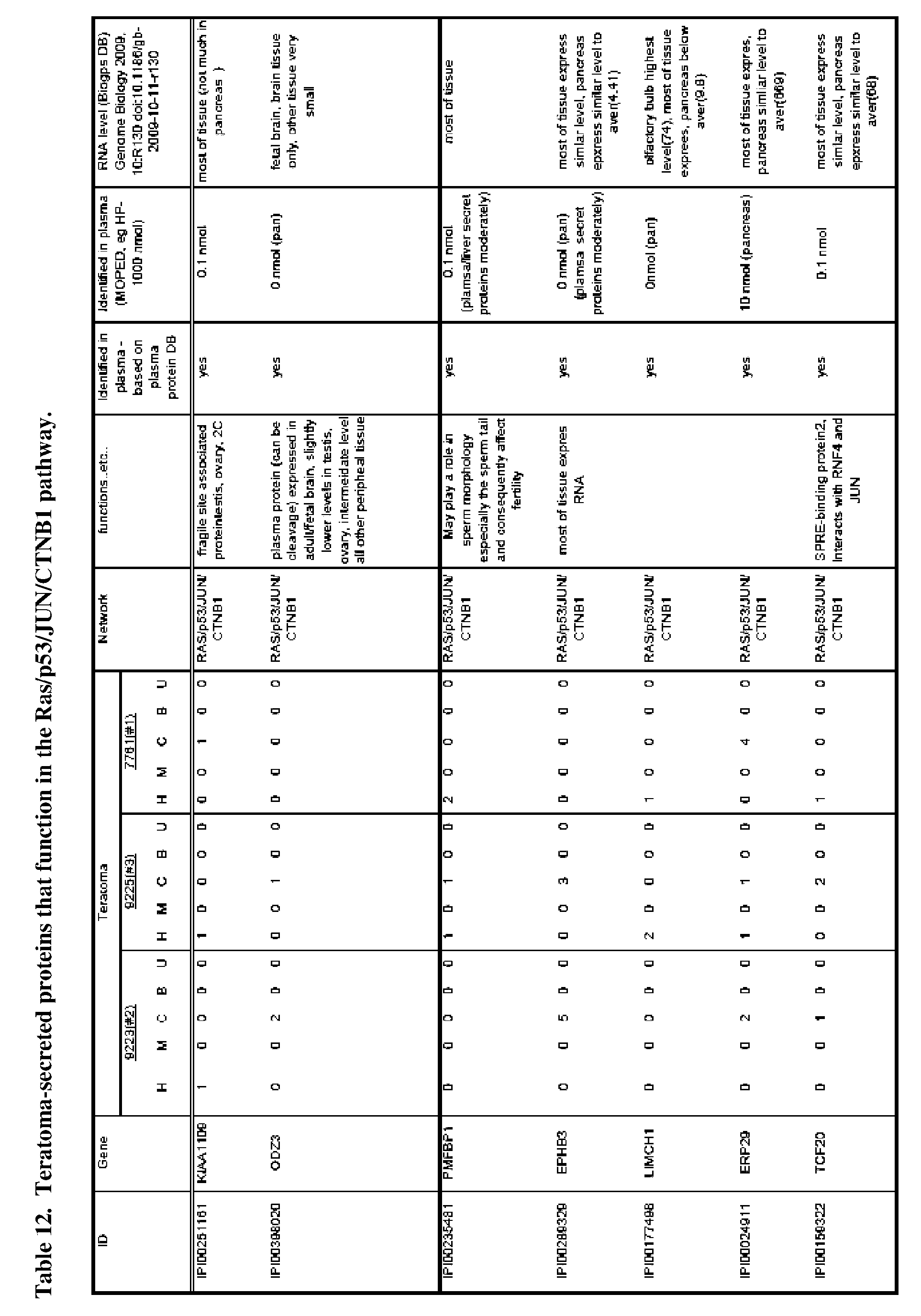

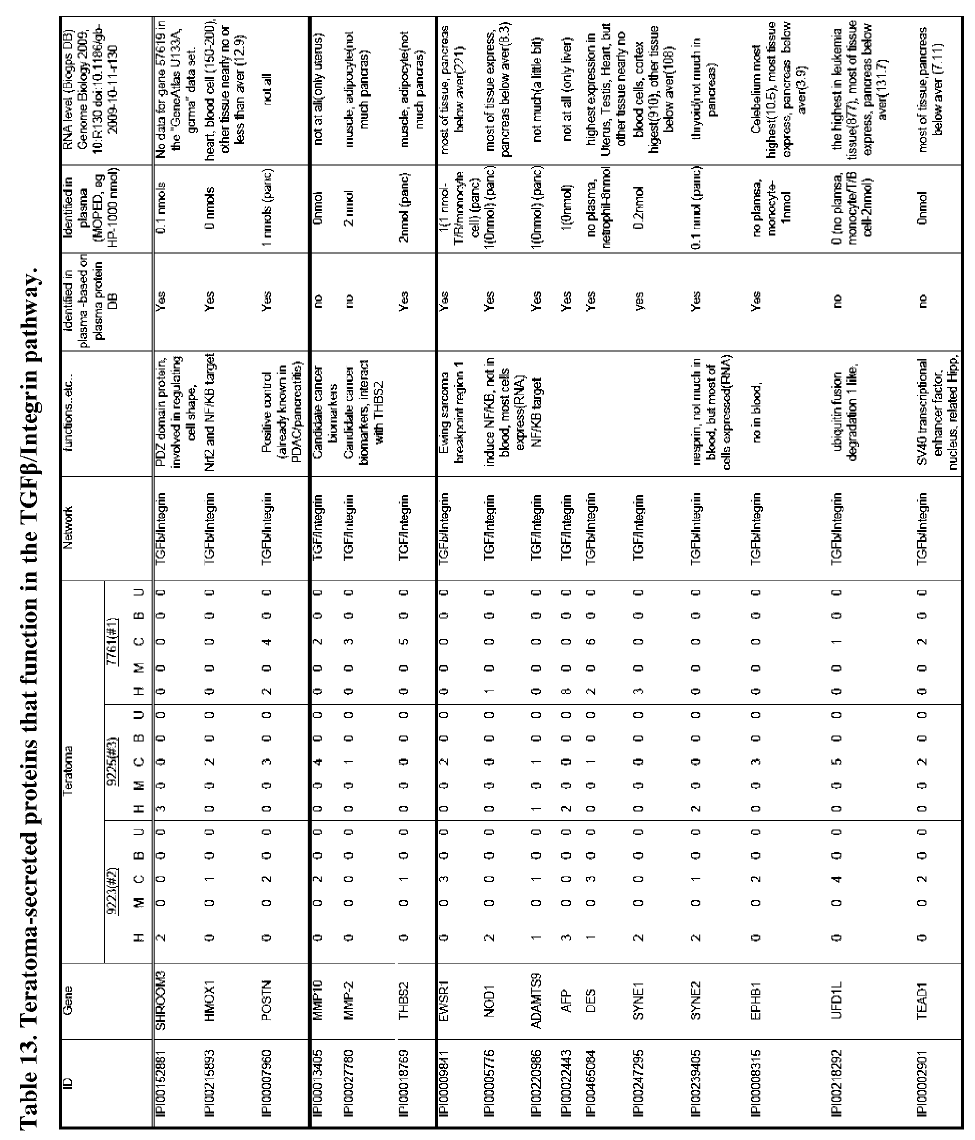

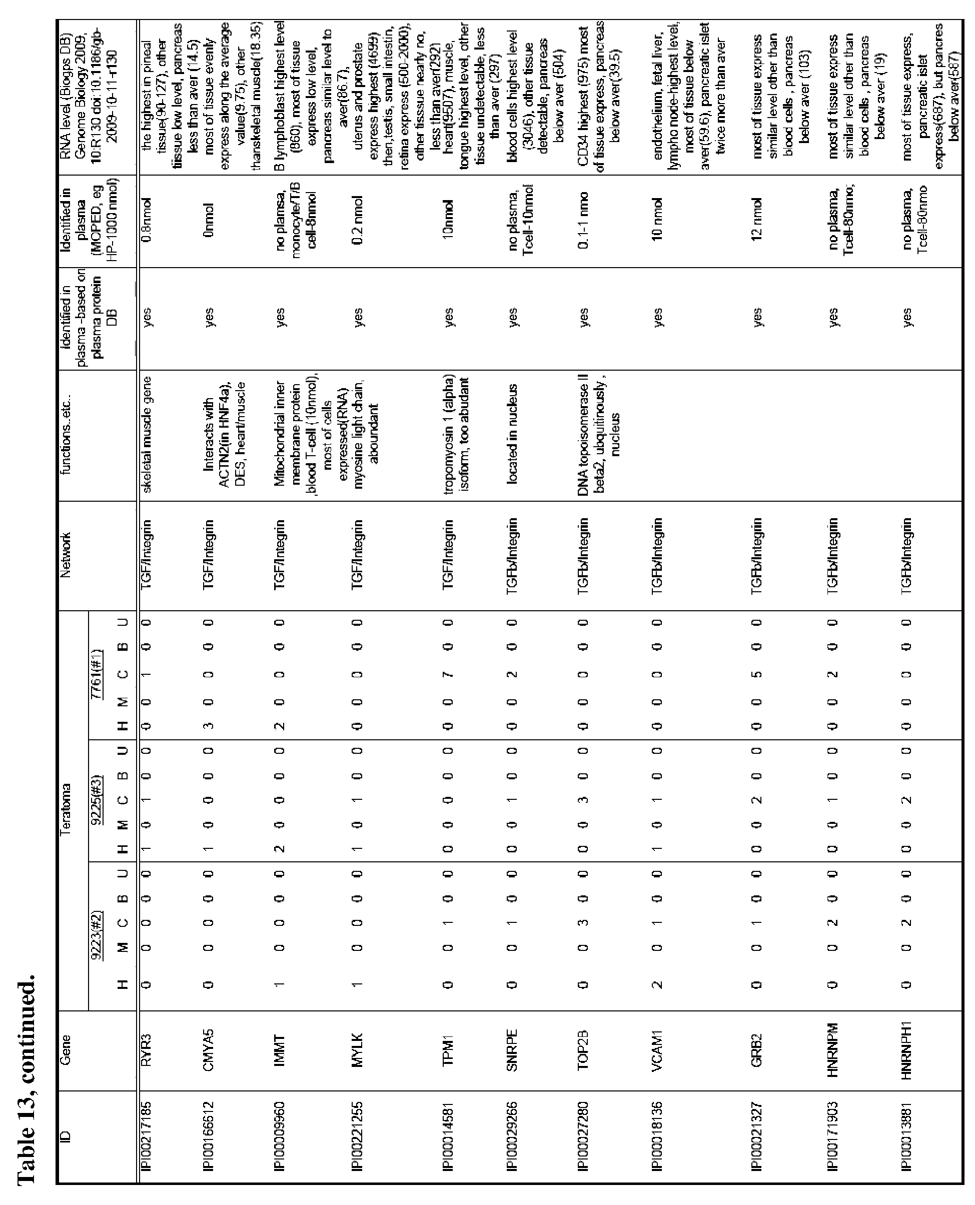

**Table 14. Summary of HNF4a immunohistochemistry on human tissues.**

| | margin of tumor | PanIN-1 | PanIN-2 | PanIN-3 | well diff. PDAC | poorly diff. PDAC |
|---|---|---|---|---|---|---|
| # samples | 4 | 8 | 7 | 3 | 8 | 8 |
| sections analyzed | 9 | 18 | 17 | 17 | 25 | 21 |
| HNF4α + nuclei | 11 | 248 | 867 | 1107 | 3099 | 60 |
| total nuclei counted | 209 | 1271 | 2008 | 1981 | 4585 | 1,677 |
| %HNF4α + nuclei | 4.91 | 16.32 | 38.64 | 59.32 | 71.22 | 3.76 |

### EXAMPLE 2: Detection of biomarkers in subjects with pancreatic cancer by ELISA.

In this Example, validation of the disclosed biomarkers for pancreatic cancer was performed in plasma samples of subjects that had pancreatic cancer using enzyme-linked immunosorbent assays (ELISA).

Validation of the disclosed biomarkers was performed by analyzing plasma samples of 10 patients with pancreatic cancer at various stages and analyzing plasma samples of 10 control subjects that did not have pancreatic cancer. Of the 10 subjects with pancreatic cancer, 7 had resectable and locally advanced cancer and 5 had resectable pancreatic cancer that was not as far advanced (Table 15).

Thirty three of the identified biomarkers described in Example 1, and one control, were analyzed in the plasma of the subjects by ELISA (Table 15). Human plasma samples were prepared from blood drawn from patients or controls by treating with EDTA or heparin as an anticoagulant. The mixture was centrifuged for 15 min at 1000 x g at 2-8 degrees centrigrade within 30 min of blood collection. The material was frozen in aliquots and repeated freeze-thaw cycles were avoided. ELISA assays were performed per the instructions of the manufacturer of the ELISA kit. In brief, 100µl of a standard or 10µl of a sample were added per well of the microtiter plate; 90 ul of diluent was added to the sample well. The wells were covered with the provided adhesive strip and incubated for 2 hours at 37°C. The liquid present in each well was removed and 100µl of manufacturer's Biotin-antibody (1x) was added to each well and covered with a new adhesive strip, followed by incubation at 37°C for 1 hour. Each well was aspirated and washed with 200µl of the manufacturer's wash buffer repeatedly for a total of three washes. After the last wash, any remaining wash buffer was removed by aspirating or decanting and the microtiter plate was inverted and blotted to further remove any remaining fluid in each well. 100µl of HRP-avidin (1x) was added to each well, and the microtiter plate was covered with a new adhesive strip and incubated for 1 hour at 37°C. The aspiration/wash process was repeated for five times as performed in the previous wash step, followed by the addition of 90µl of the manufacturer's TMB Substrate to each well and incubation for 15-30 minutes at 37°C. After the incubation at 37°C, 50µl of manufacturer's Stop Solution was added to each well and the plate was gently tapped to ensure thorough mixing. The optical density of each well was determined within 5 minutes using a microplate reader set to 450 nm. The signals in each well were analyzed by a 4 parameter logistic nonlinear regression model for curve-fitting to the standard curve, using Soft Max Pro, allowing the determination of antigen concentration in each plasma sample.

To compare antigen concentrations between samples and between patient (cases) plasma and plasma of control individuals, a logistic regression analysis was performed to estimate the area under the receiver operating characteristic (ROC) curve (*i.e.,* c-statistic). The ROC curve was estimated by plotting the true positive rate (sensitivity) vs. the false positive rate (1-specificity) across the range of values observed for the biomarker of interest in the dataset. A c-statistic of 0.5 coincides with the marker predicting case/control status correctly 50% of the time. A c-statistic higher than 0.7 would indicate that the biomarker is reliable as a diagnostic for pancreatic cancer.

**Table 15**

| | | **c-statistic from logistic regression (area under ROC curve)** | | |
|---|---|---|---|---|
| **Biomarker** | **Network** | **All Cases (N=10)** | **Resectable & Locally Advanced (N=7)** | **Resectable (N=5)** |
| AFP | TGFβ/integrin | 0.480 | 0.464 | 0.420 |
| RTTN | No network | 0.595 | 0.579 | **0.730** |
| NLRX1 | No network | 0.490 | 0.529 | 0.580 |
| DNAH12 | HNF4a | **1.00** | **1.00** | **1.00** |
| ODZ3 | Ras/p53/JUN/CTN B1 | 0.560 | 0.529 | 0.560 |
| ADAMST9 | TGFβ/integrin | 0.610 | 0.600 | 0.540 |
| TPM1 | TGFβ/integrin | 0.645 | 0.686 | **0.800** |
| DNAH1 | HNF4a | **0.740** | **0.764** | **0.780** |
| PMFBP1 | Ras/p53/JUN/CTN B1 | 0.550 | 0.55 | 0.550 |
| DNAH17 | HNF4a | 0.630 | 0.486 | 0.550 |
| EPHB1 | TGFβ/integrin | 0.330 | 0.357 | 0.430 |
| DOS | No network | 0.545 | 0.543 | 0.640 |
| MMP2 | TGFβ/integrin | 0.570 | 0.557 | 0.600 |
| STARD8 | no network | **0.700** | **0.729** | **0.800** |
| ATP2A1 | no network | **0.725** | **0.736** | **0.700** |
| FKBP10 | HNF4a | 0.700 | 0.657 | 0.640 |
| TCHP | no network | 0.605 | 0.393 | 0.470 |
| TCF20 | Ras/p53/JUN/CTN B1 | 0.630 | 0.507 | 0.500 |
| ABCA13 | no network | 0.550 | 0.550 | 0.550 |
| SCN8A | no network | 0.550 | 0.550 | 0.550 |
| TOP2B | TGFβ/integrin | **0.860** | **0.829** | **0.760** |
| LIMCH1 | Ras/p53/JUN/CTN B1 | **0.725** | 0.607 | 0.560 |
| UFD1L | TGFβ/integrin | 0.695 | 0.607 | 0.500 |
| FLRT3 | HNF4a | 0.400 | 0.386 | 0.400 |
| ZHX2 | HNF4a | 0.570 | 0.514 | 0.610 |
| SYNE1 | TGFβ/integrin | 0.560 | 0.586 | **0.780** |
| THBS2 | TGFβ/integrin | **0.760** | **0.886** | **0.840** |
| HMOX1 | TGFβ/integrin | 0.610 | 0.600 | 0.620 |
| Obscurin | HNF4a | 0.550 | 0.486 | 0.550 |
| DNAH5 | HNF4a | 0.550 | 0.593 | 0.540 |
| Shroom3 | TGFβ/integrin | 0.450 | 0.471 | 0.500 |
| LOXL3 | HNF4a | 0.480 | 0.614 | **0.700** |
| Malectin | HNF4a | 0.510 | 0.557 | 0.460 |
| Ca199 | N/A | 1.000 | 1.000 | 1.000 |

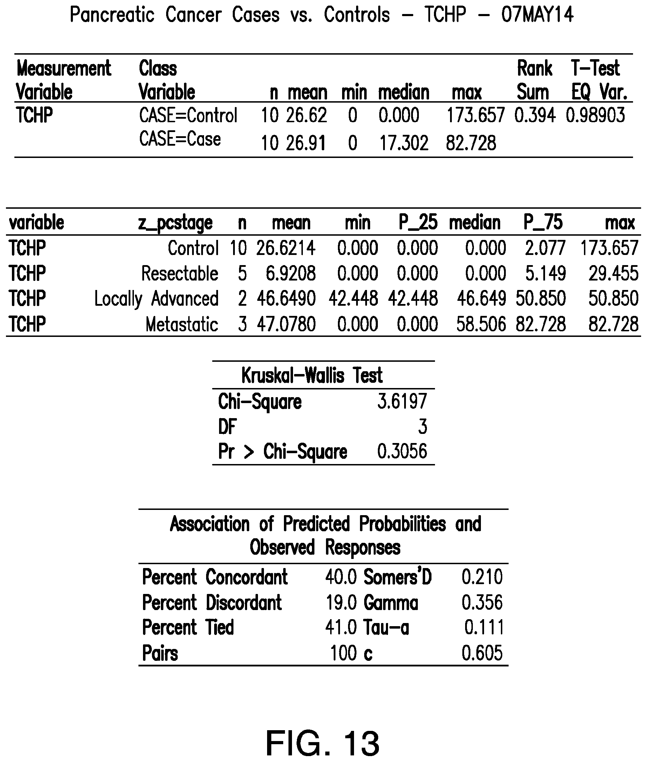

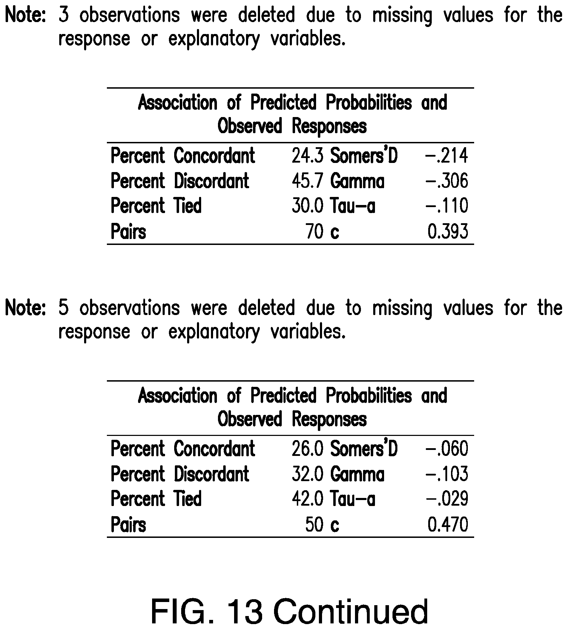

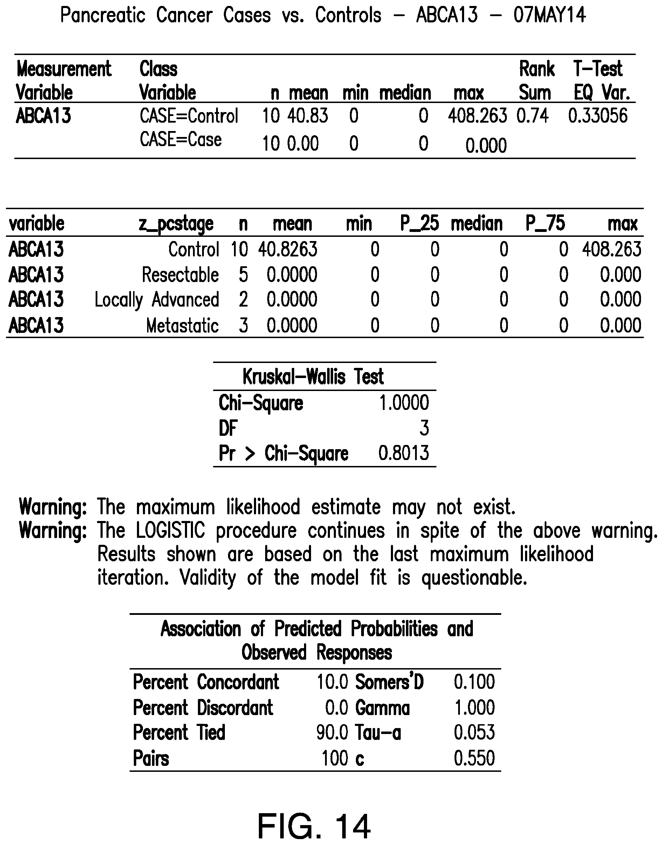

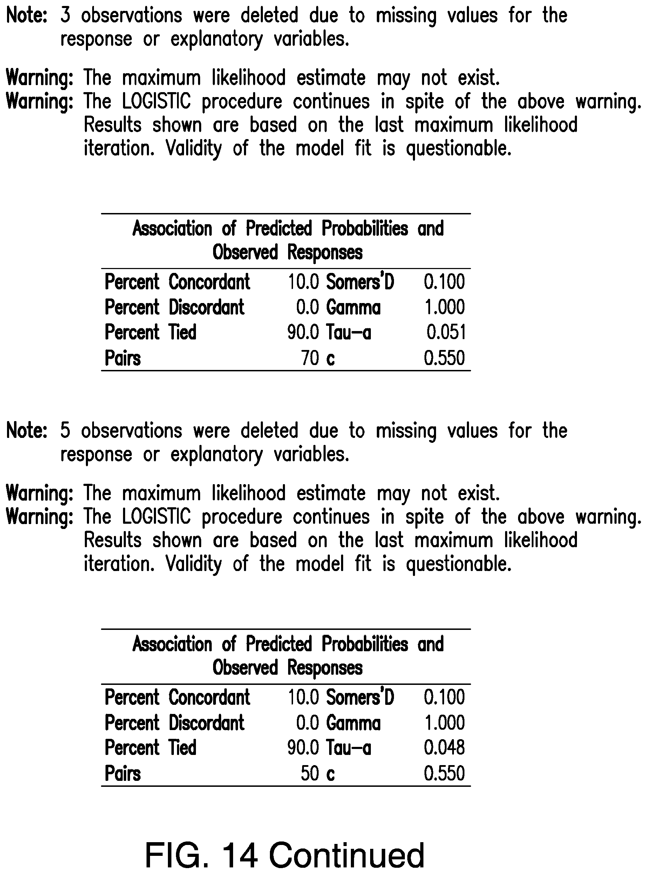

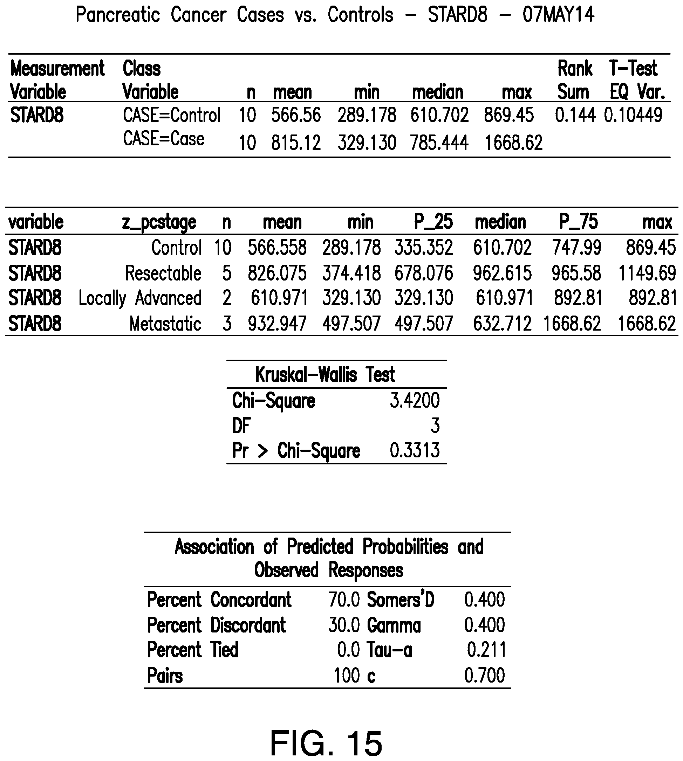

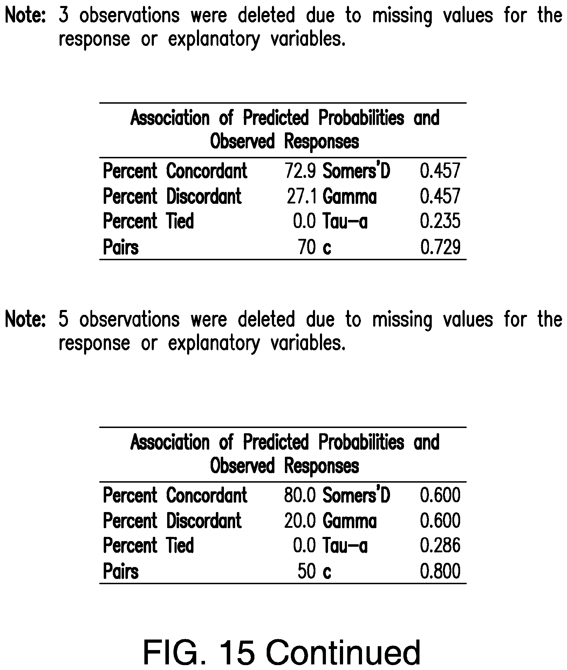

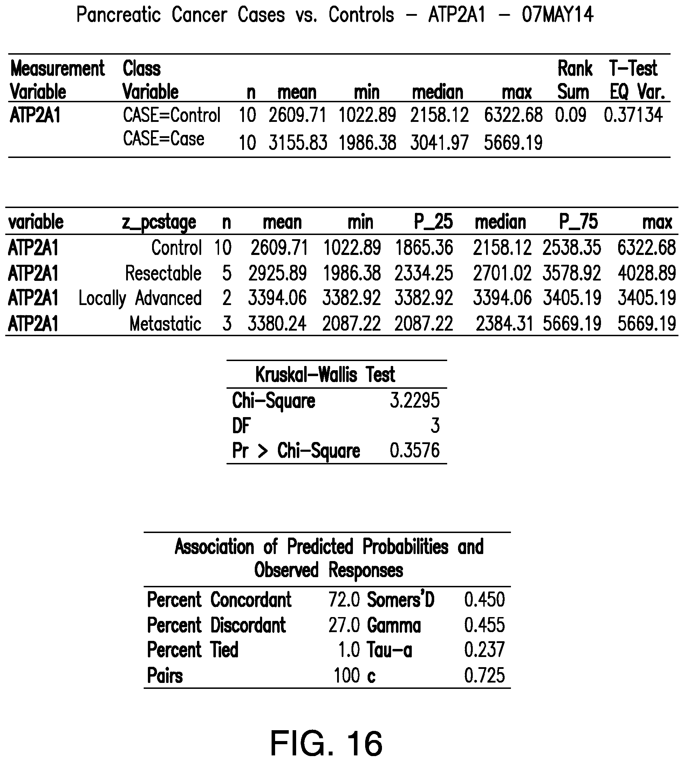

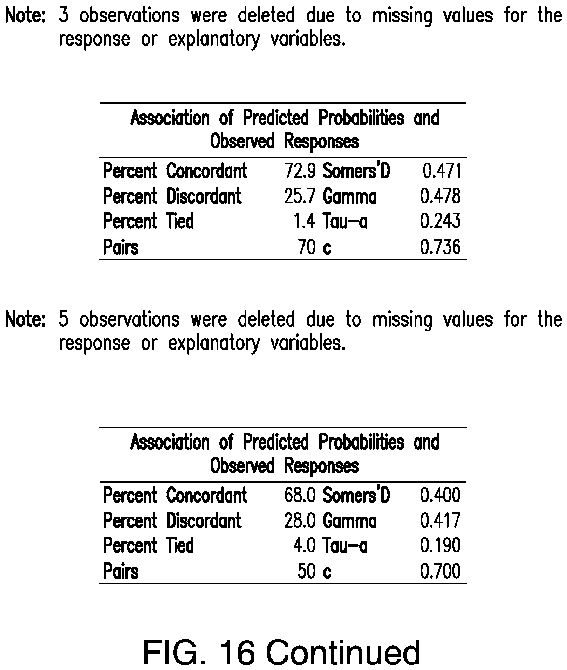

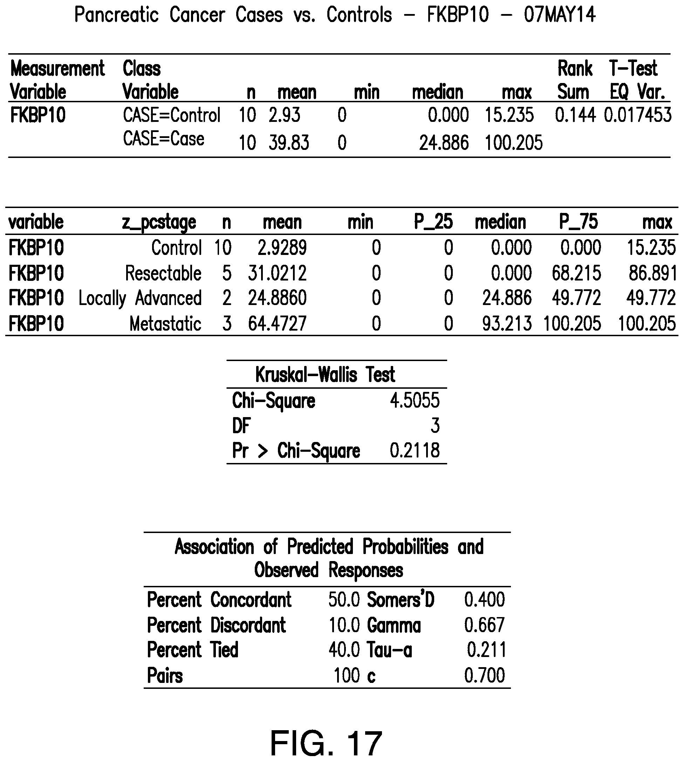

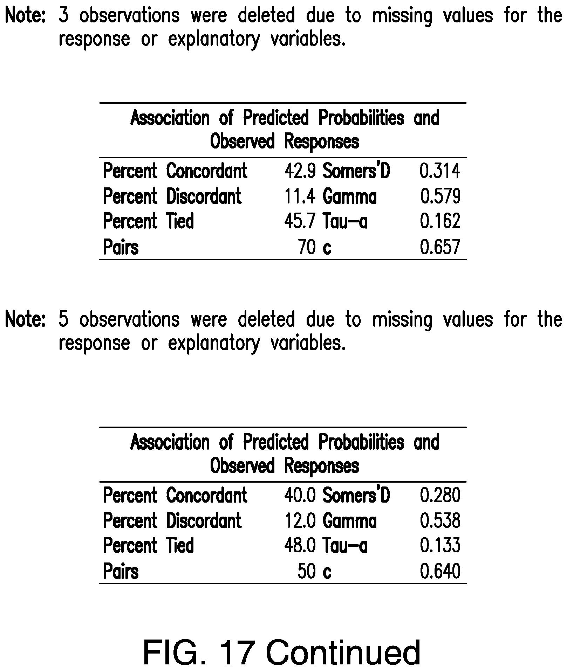

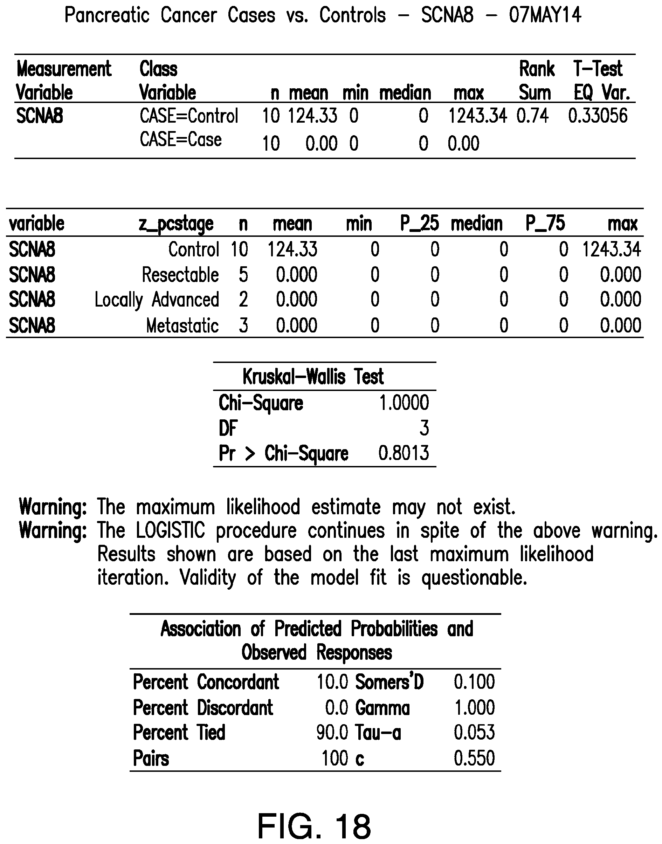

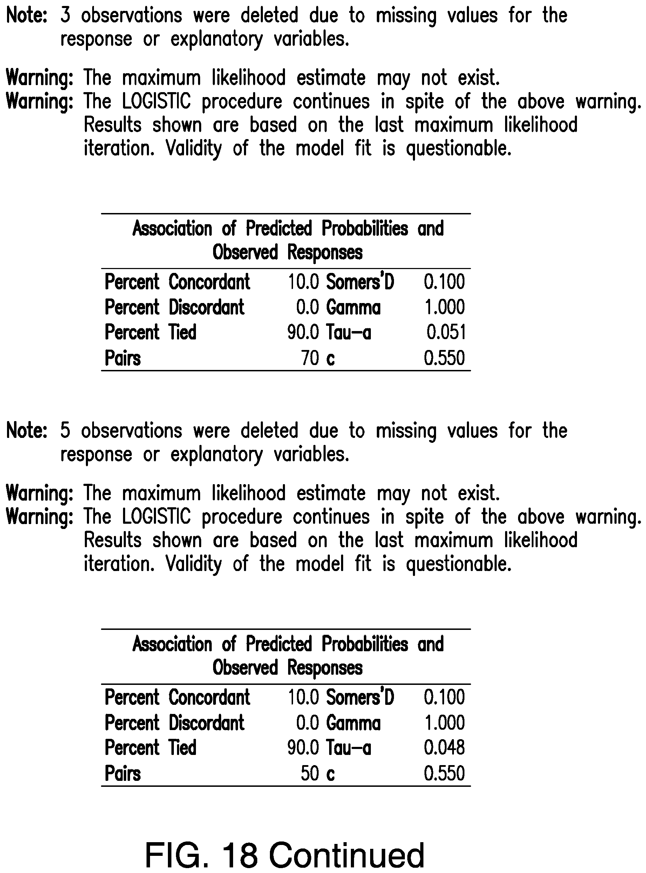

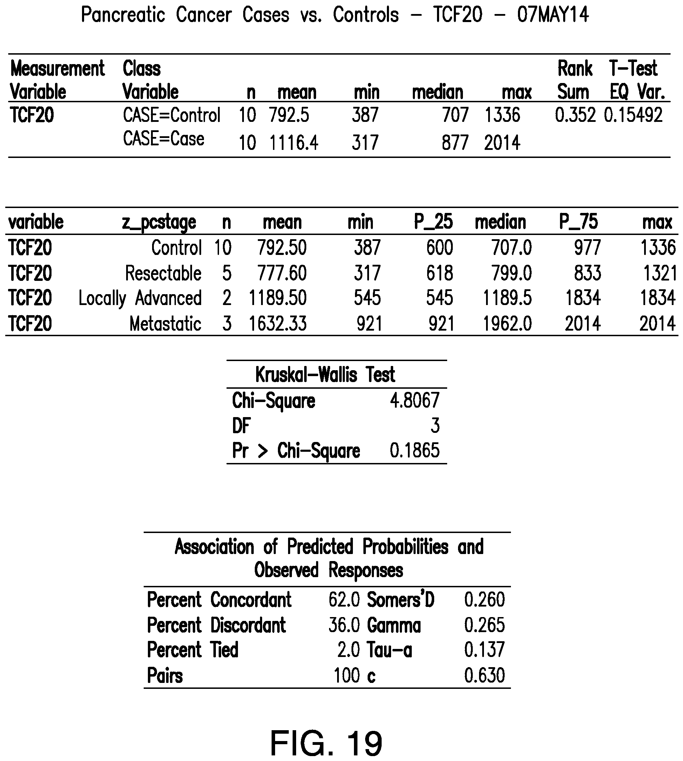

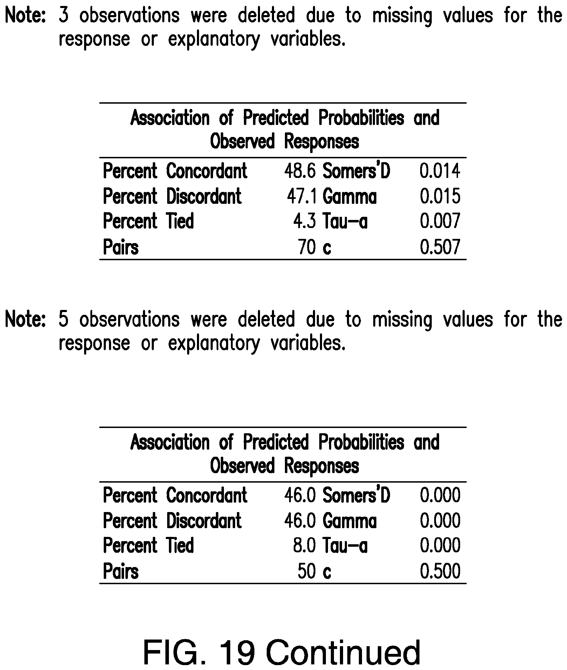

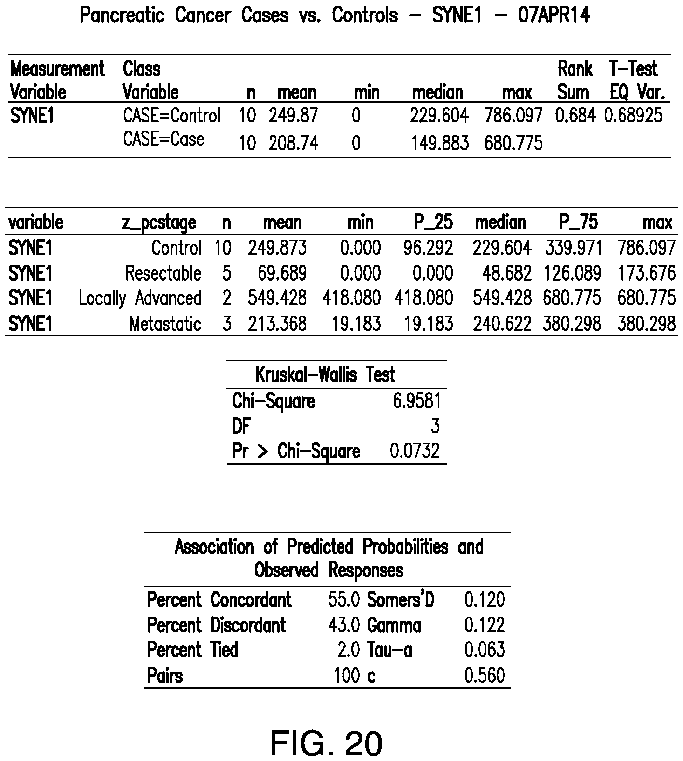

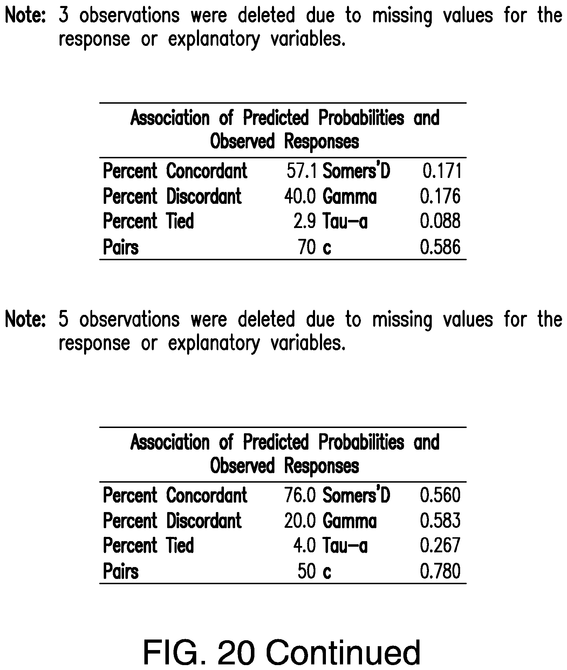

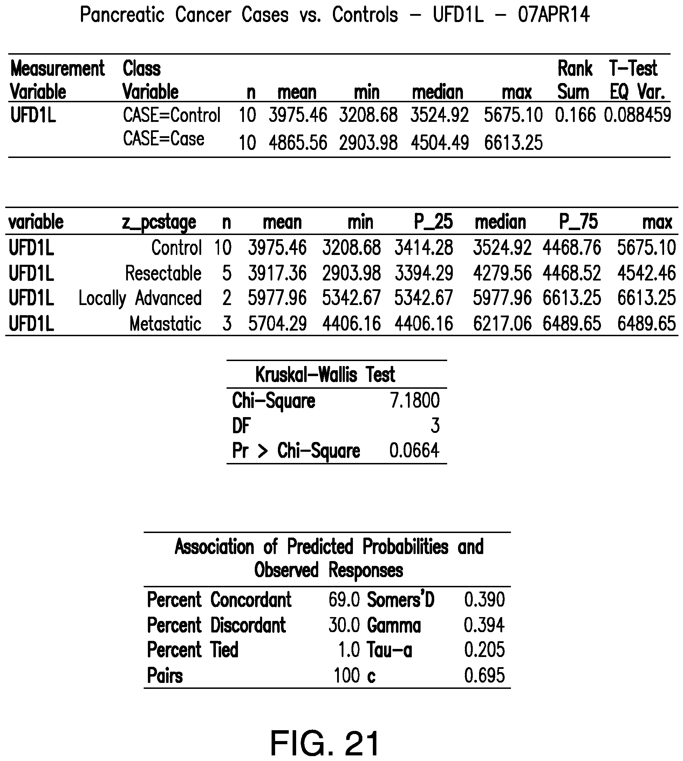

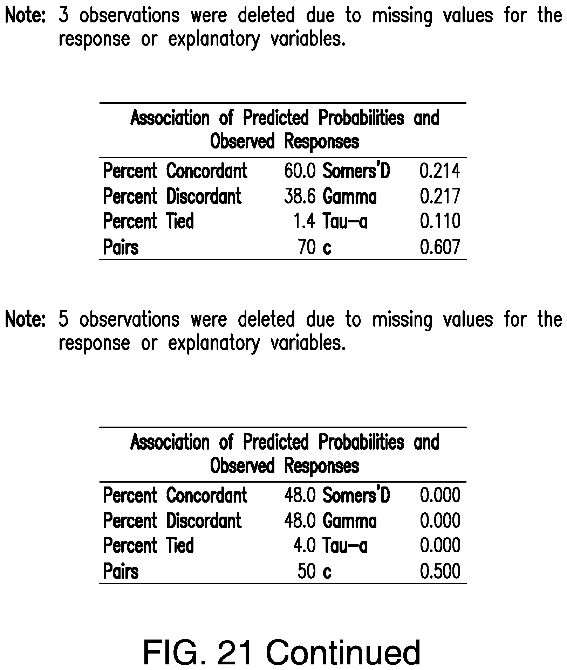

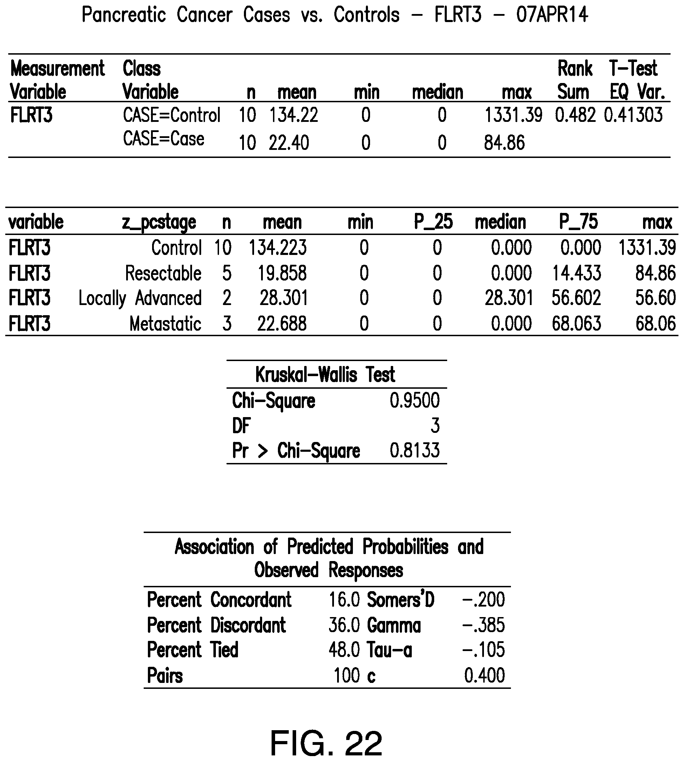

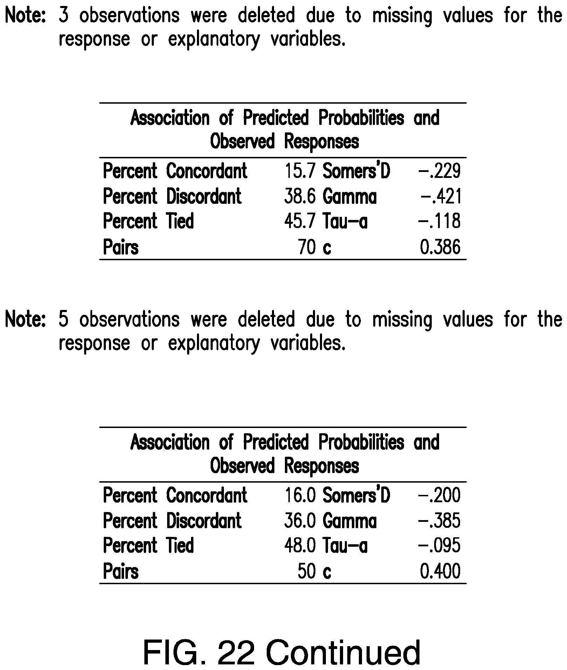

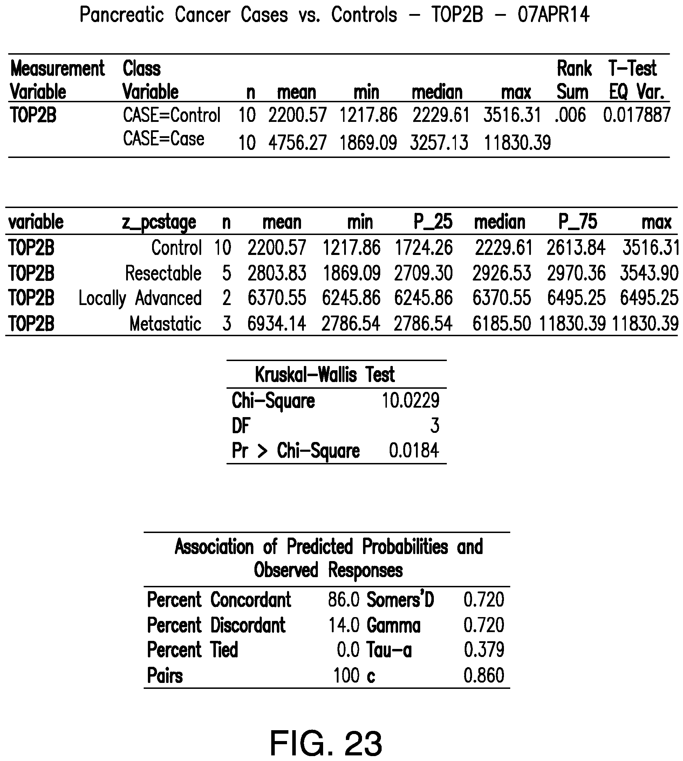

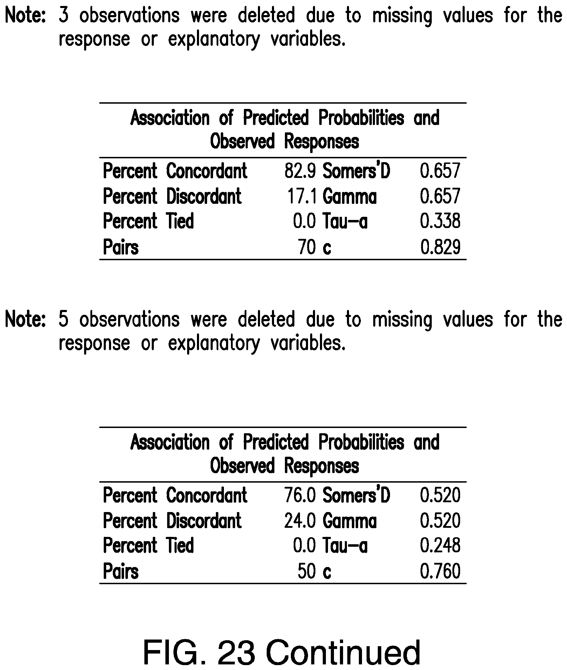

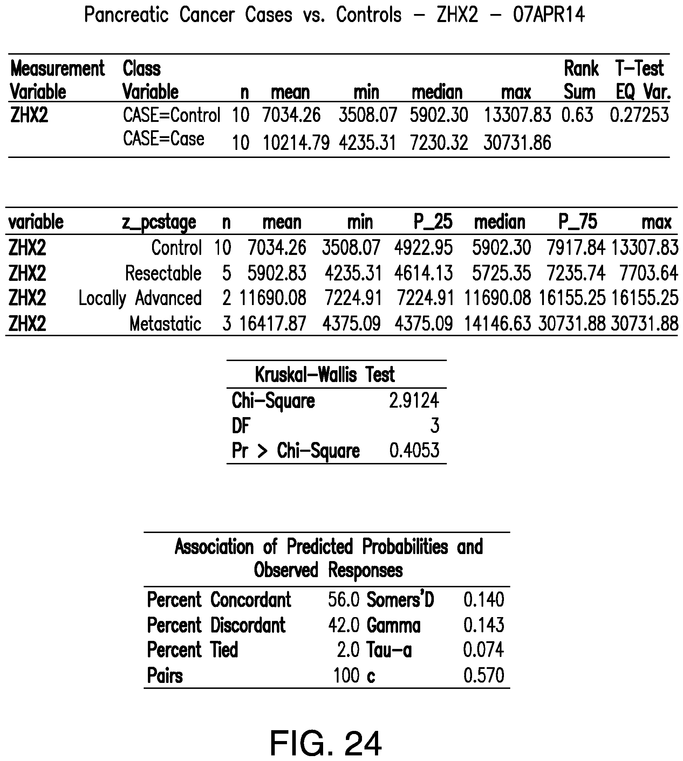

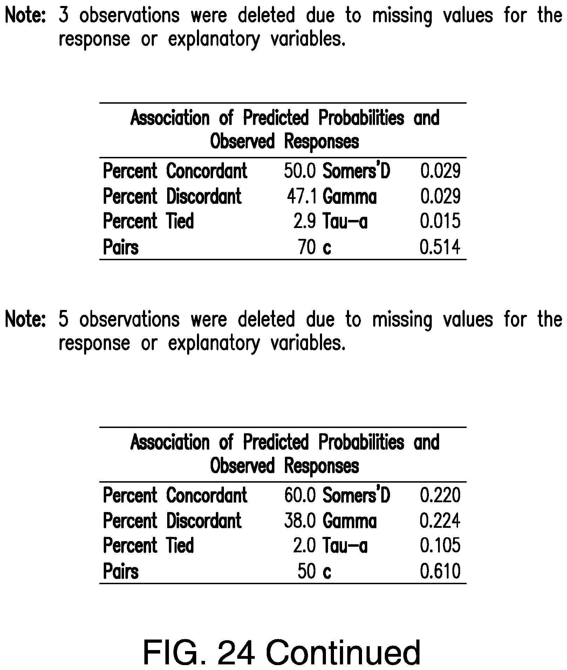

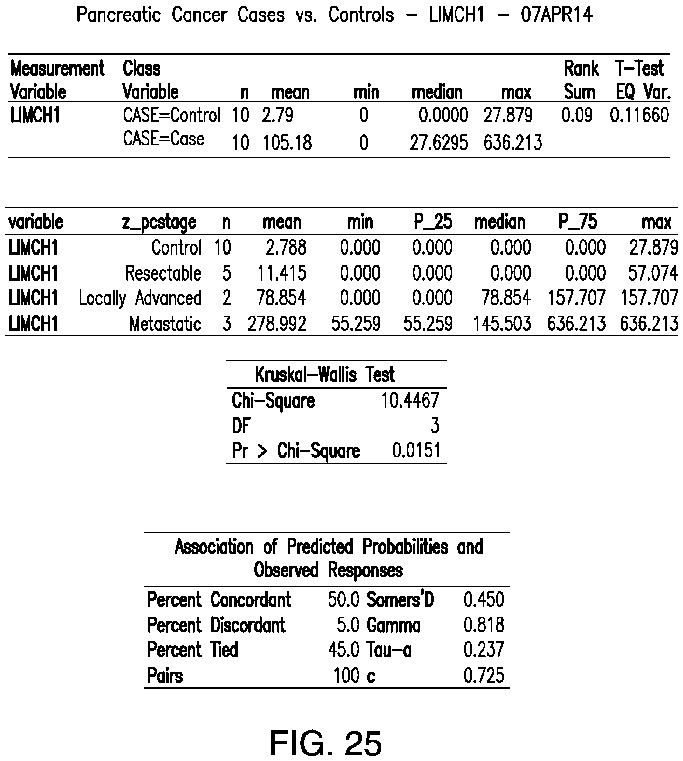

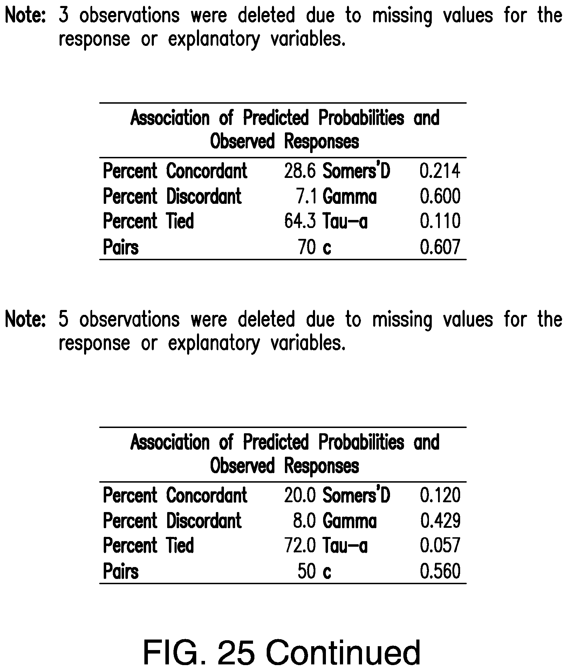

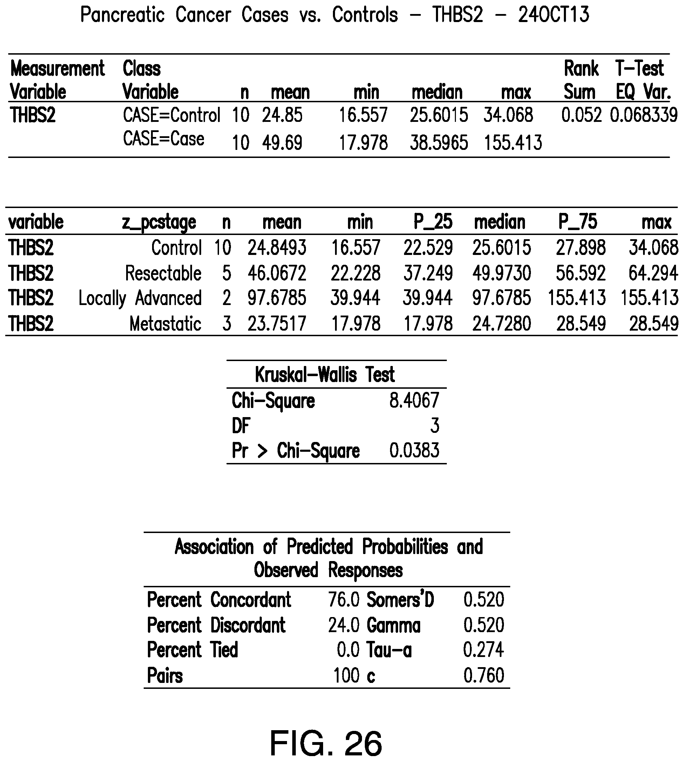

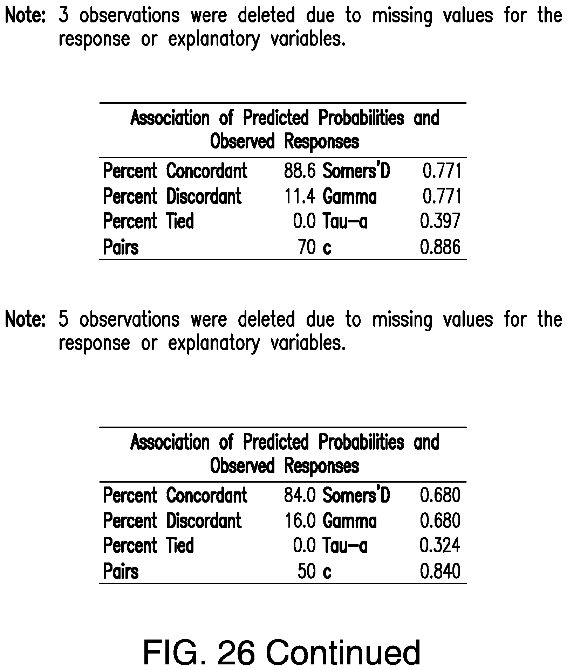

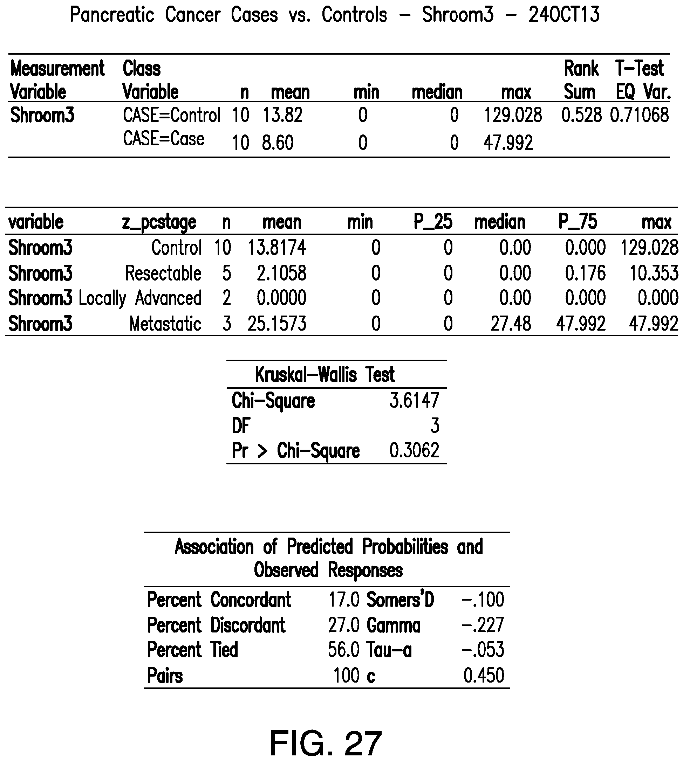

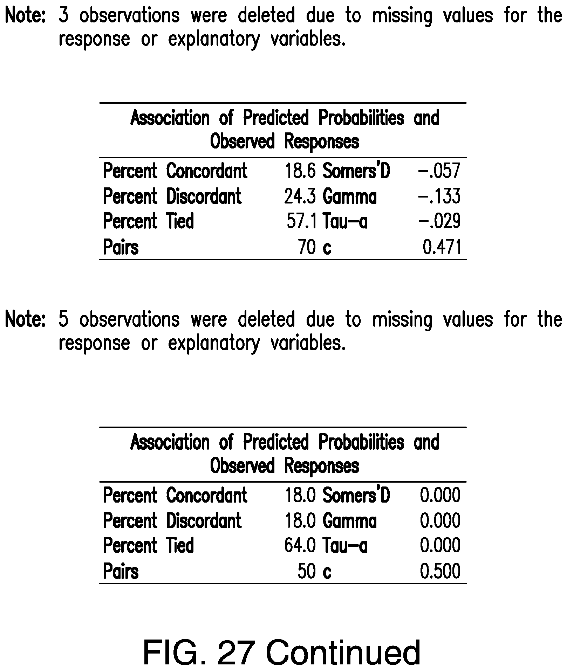

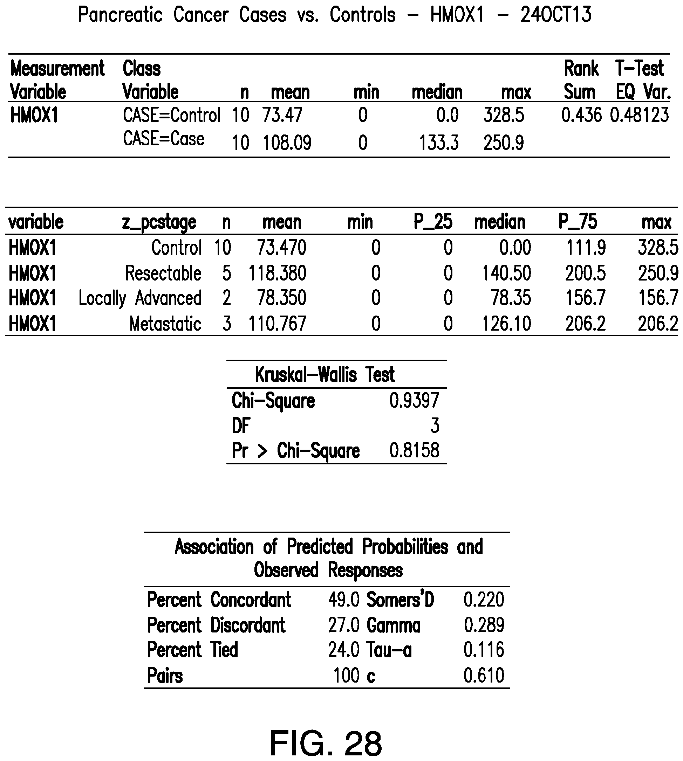

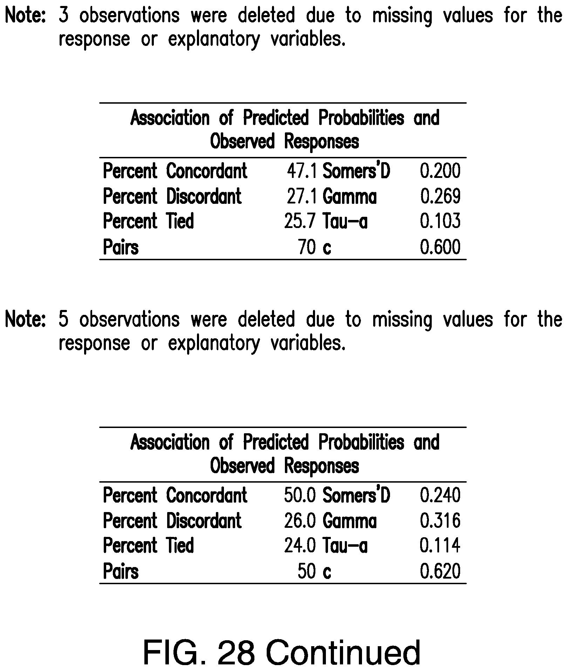

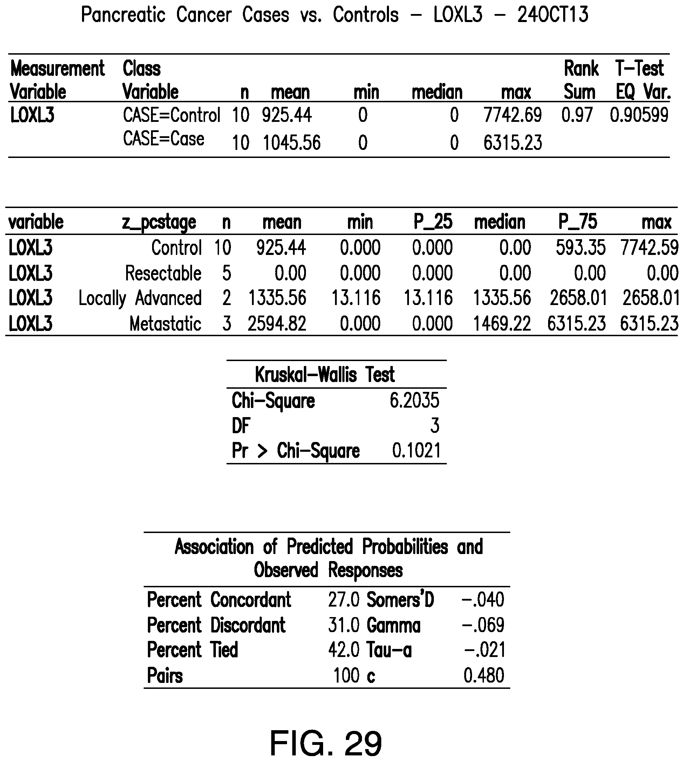

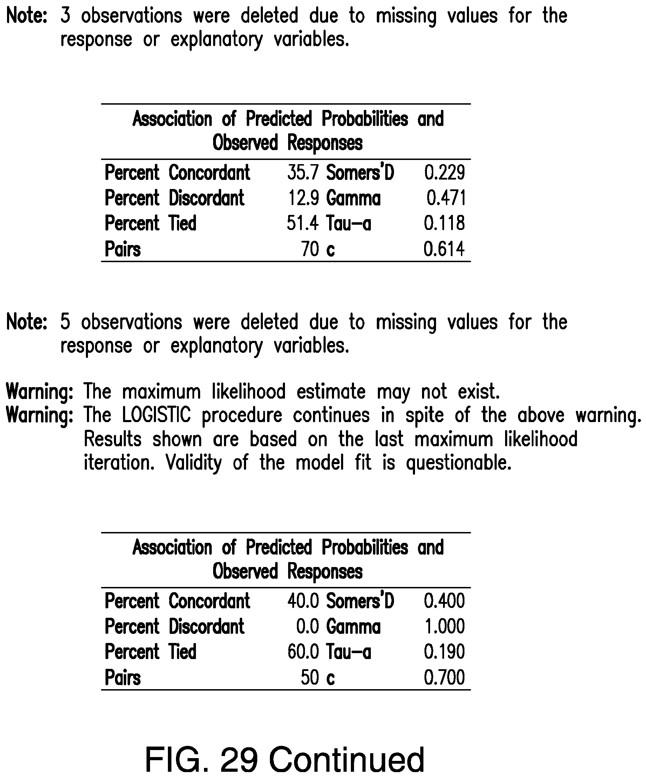

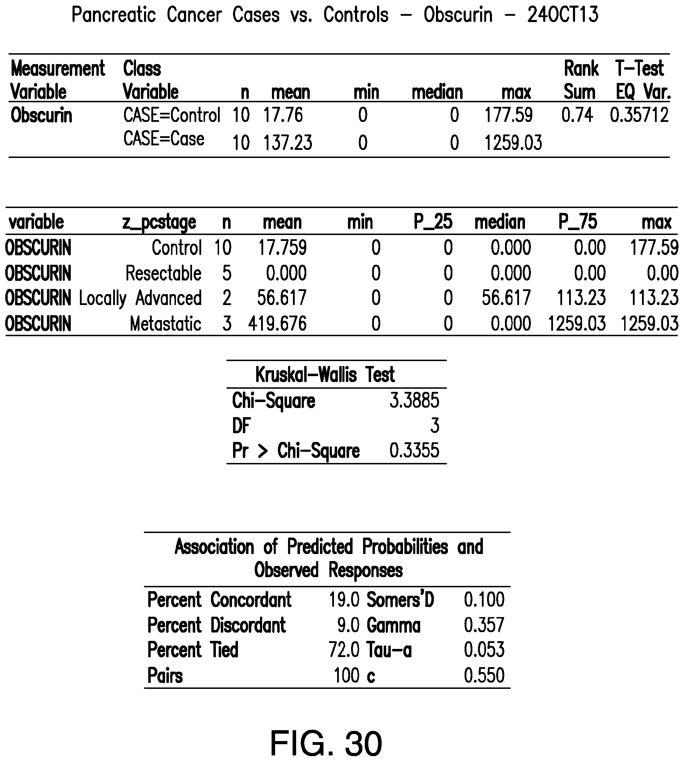

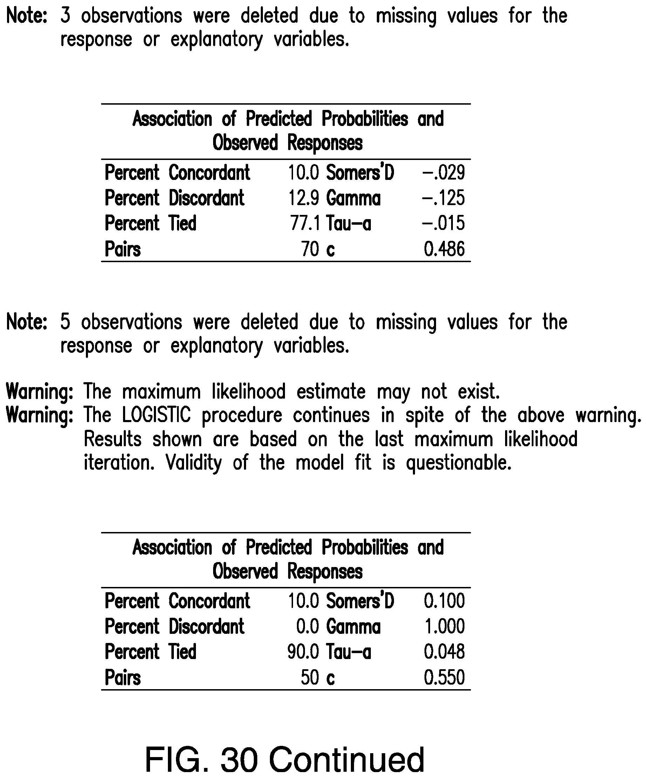

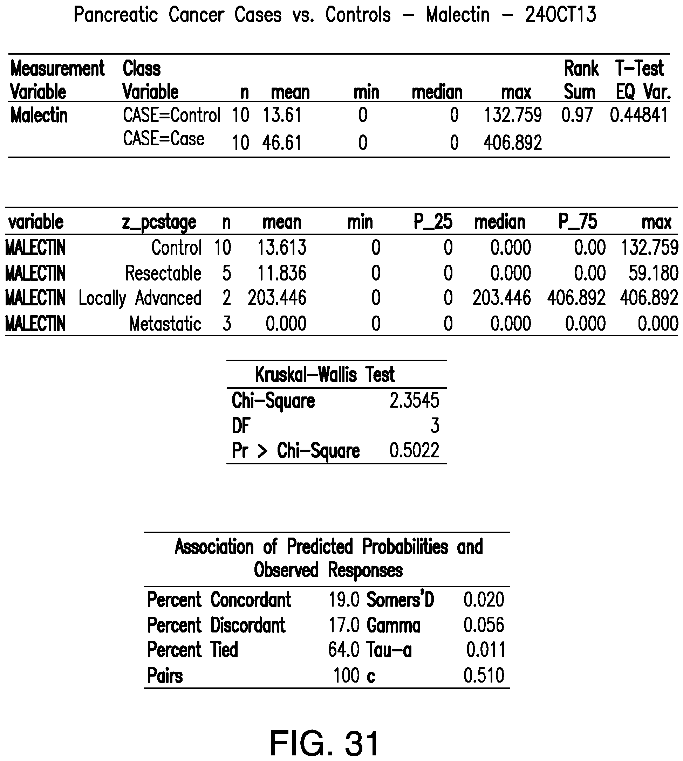

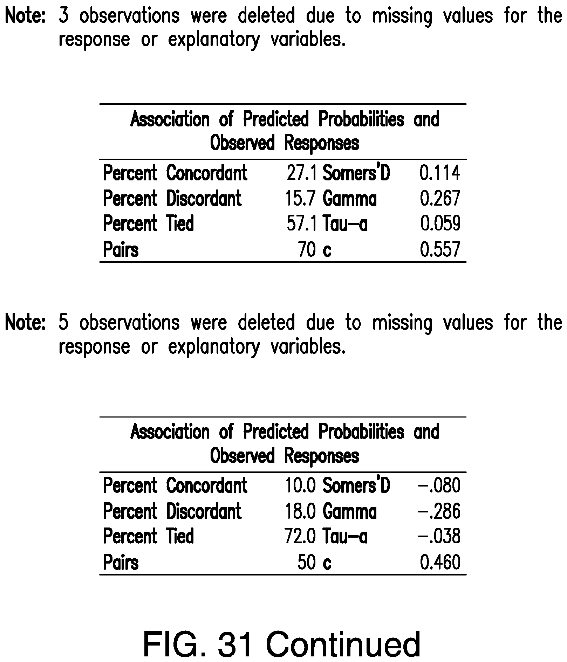

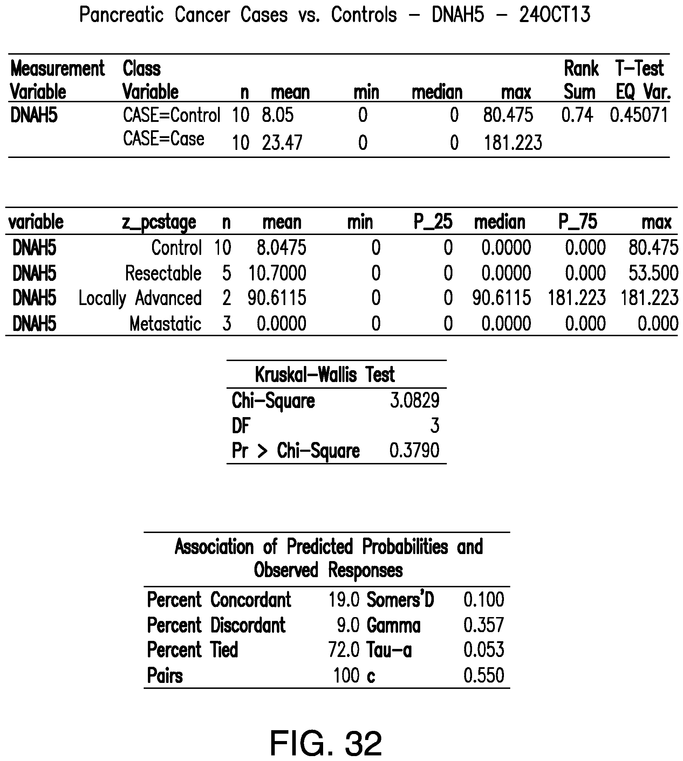

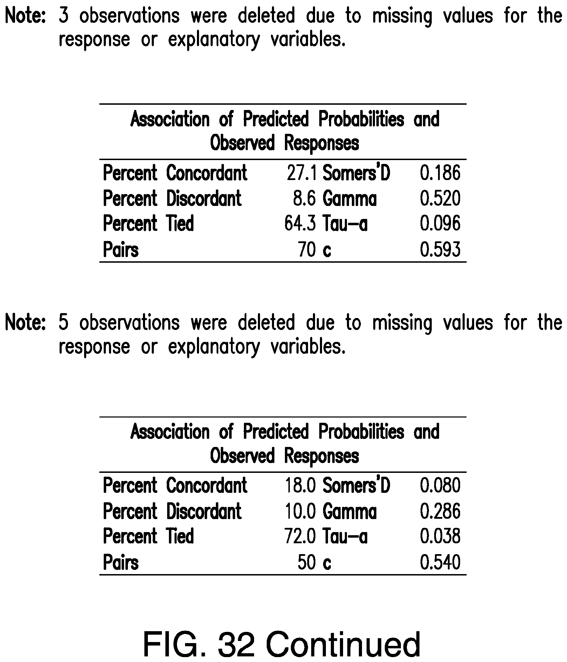

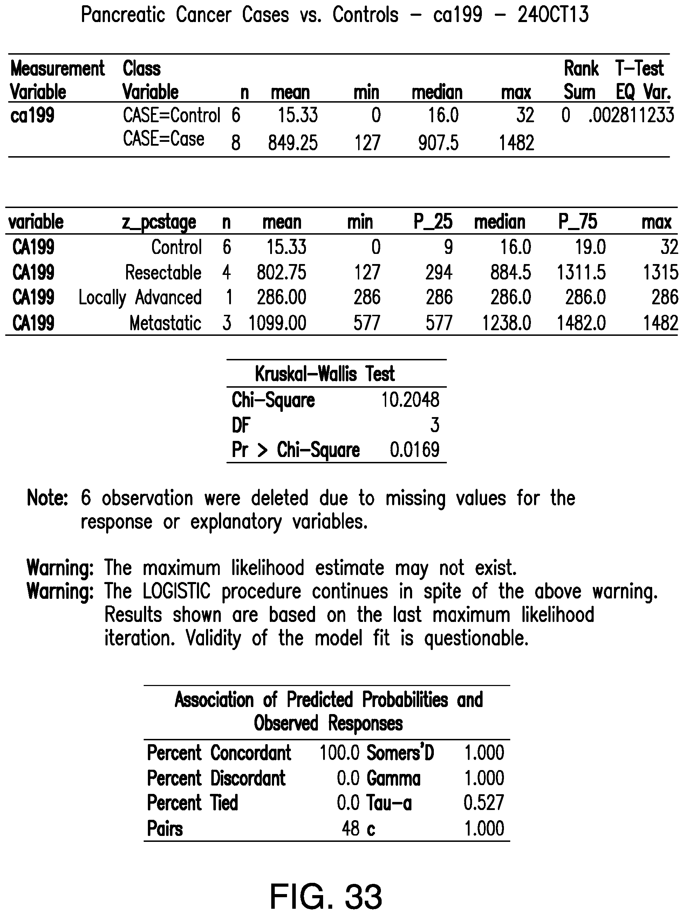

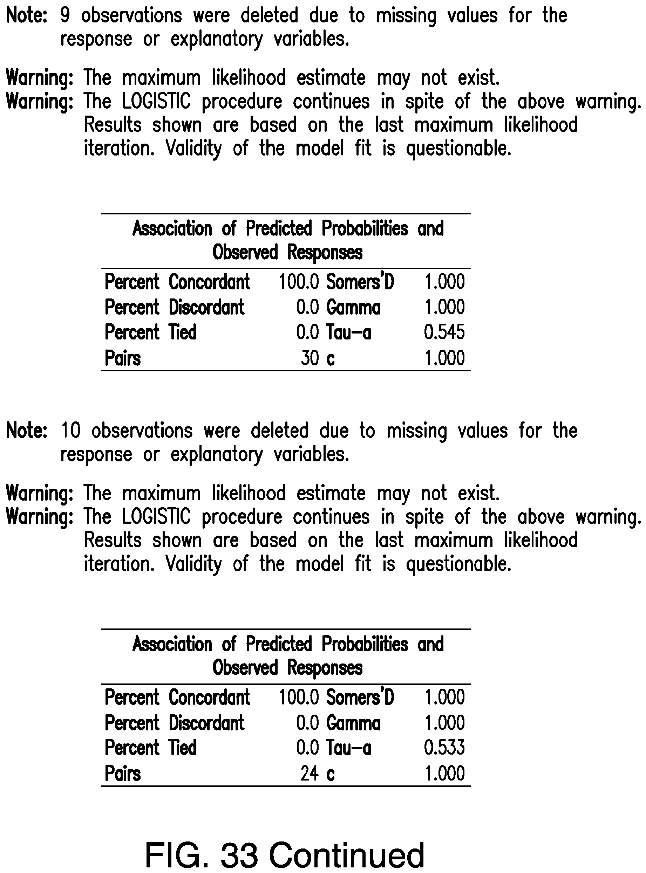

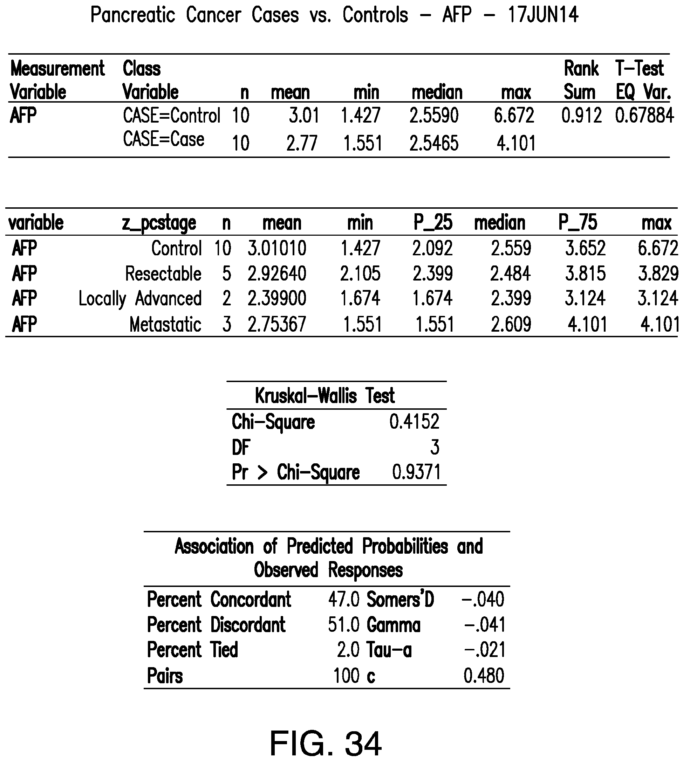

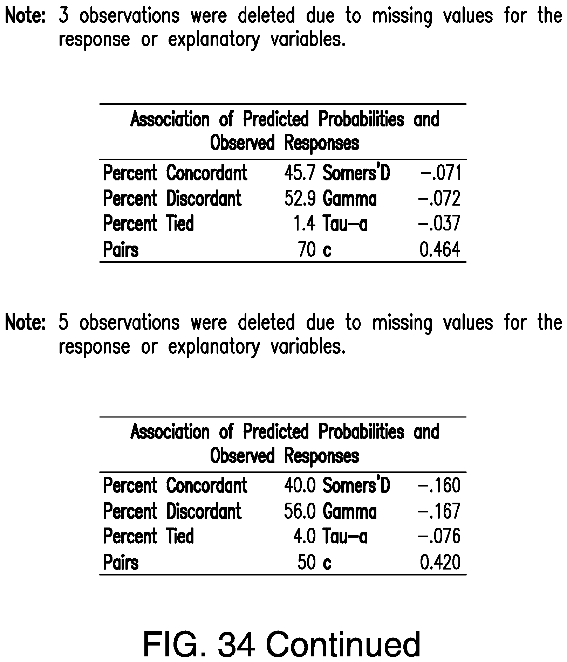

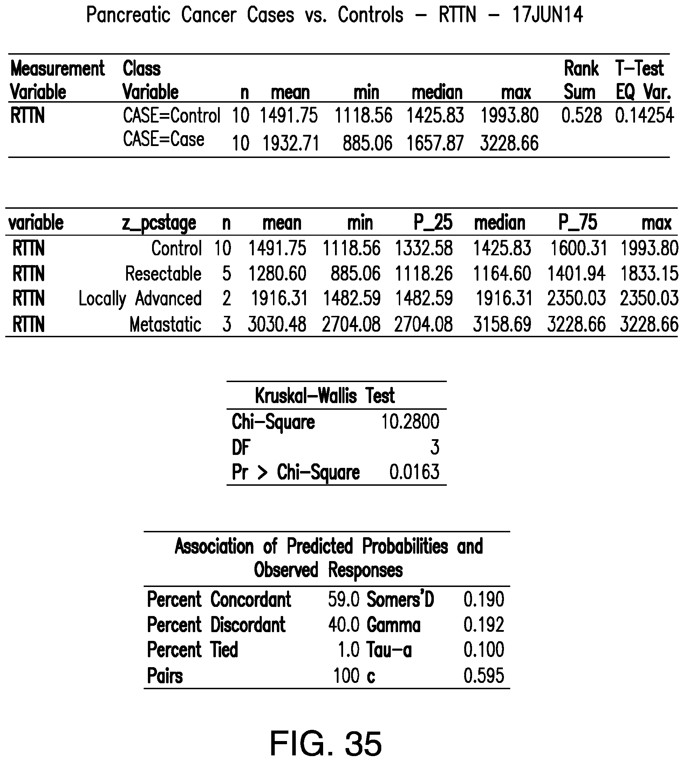

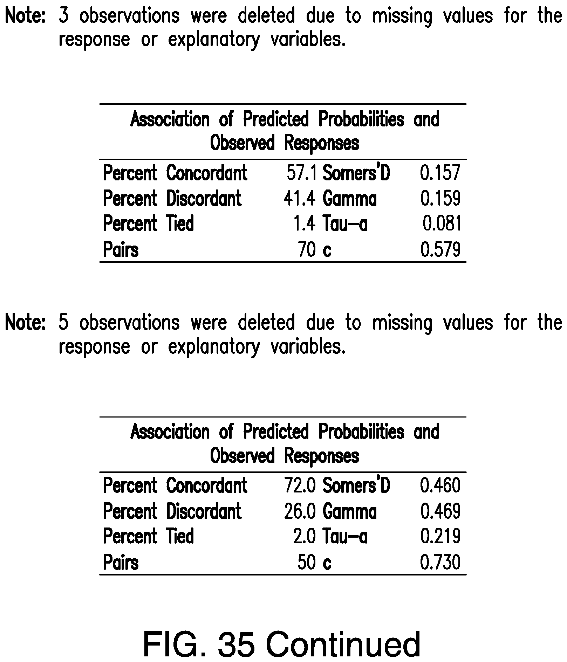

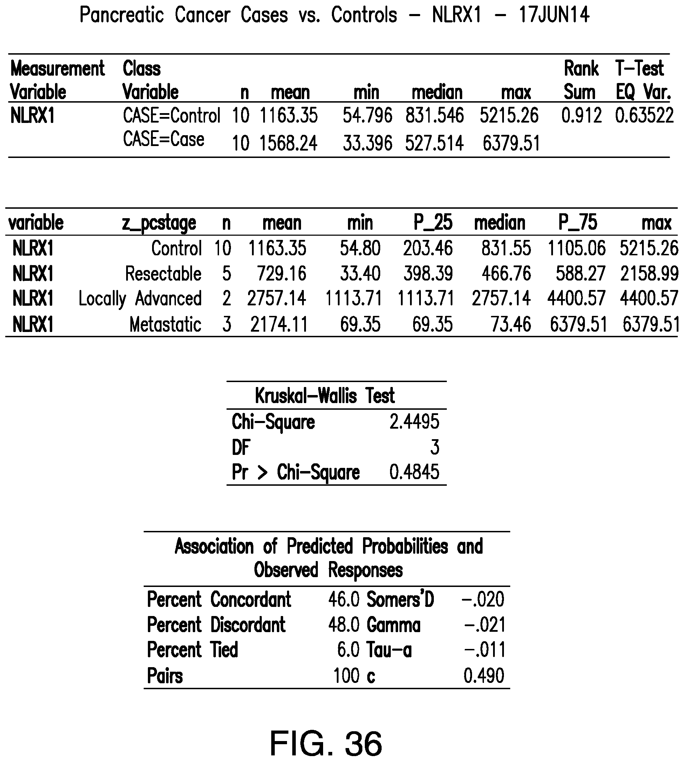

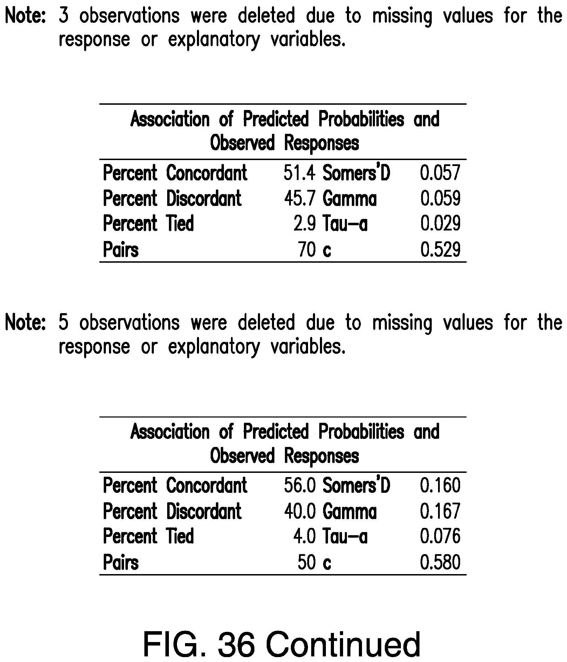

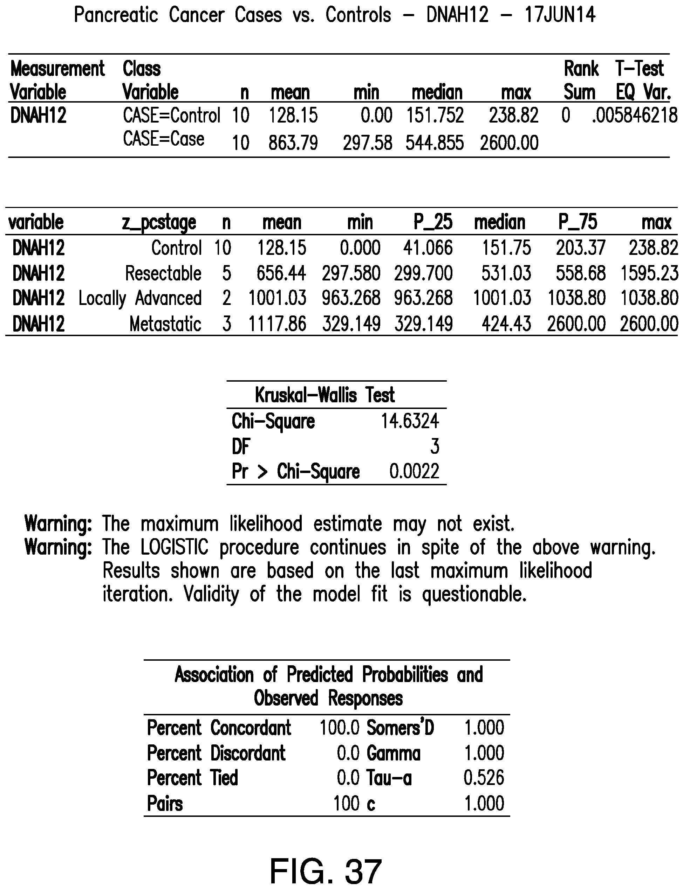

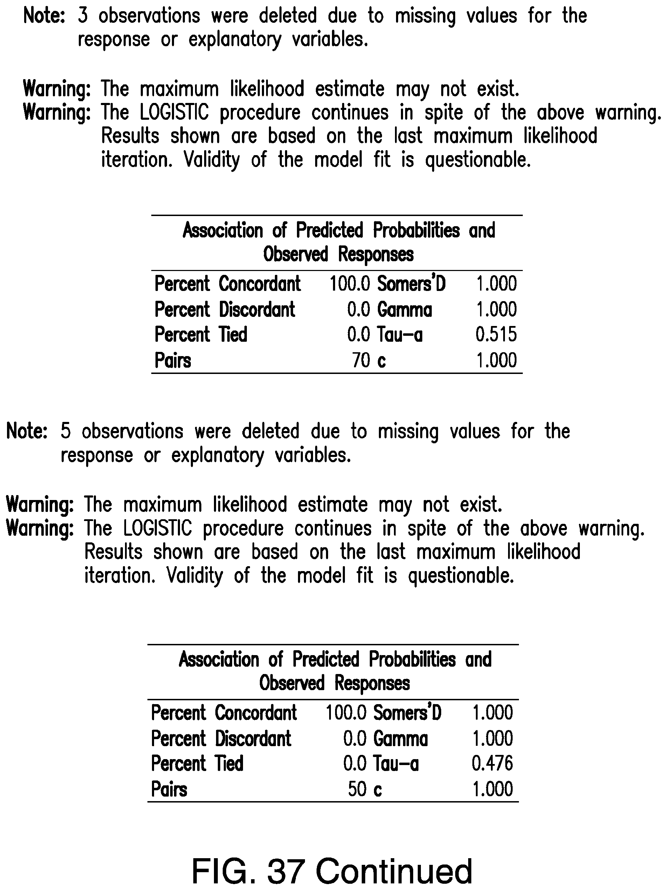

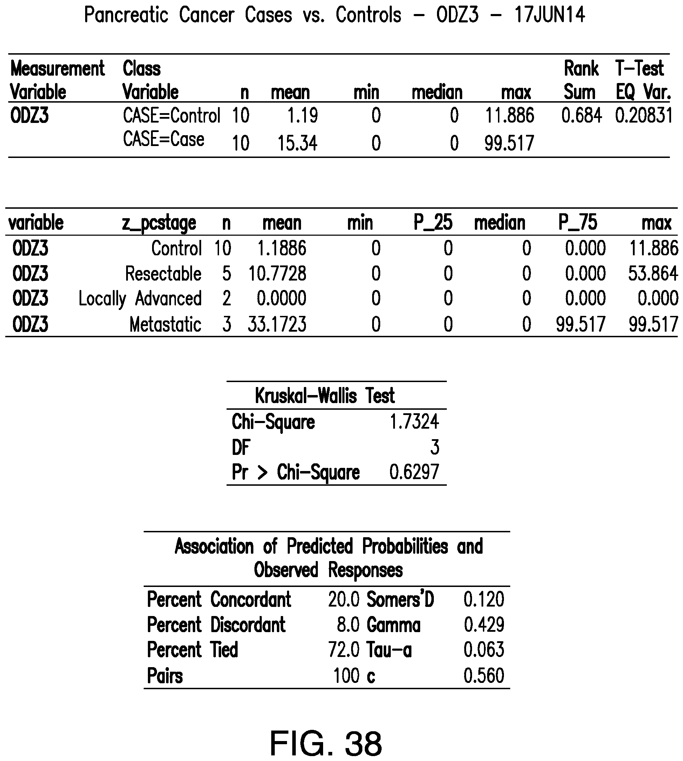

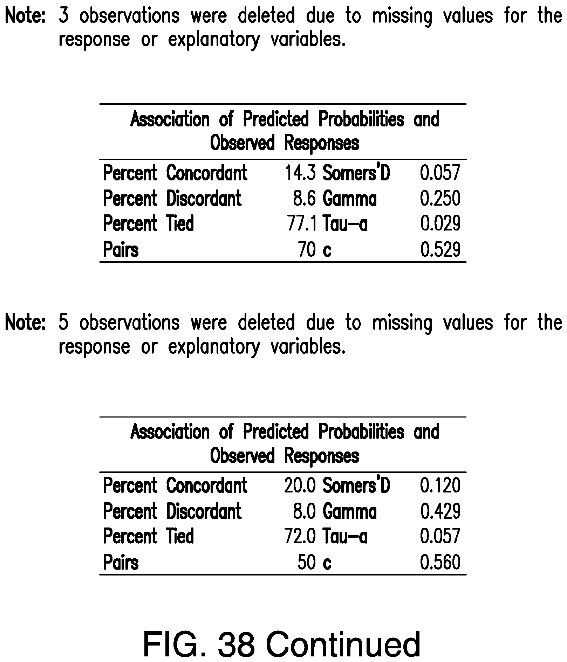

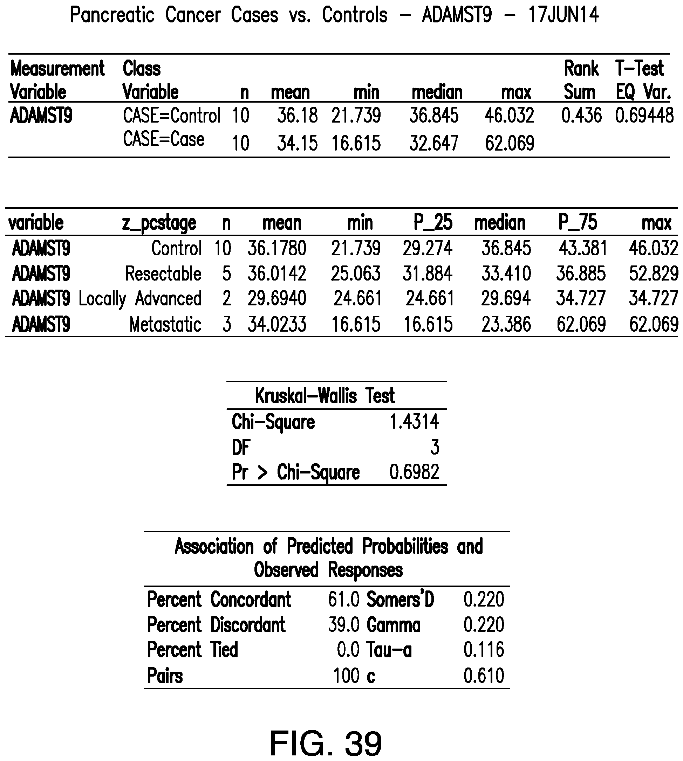

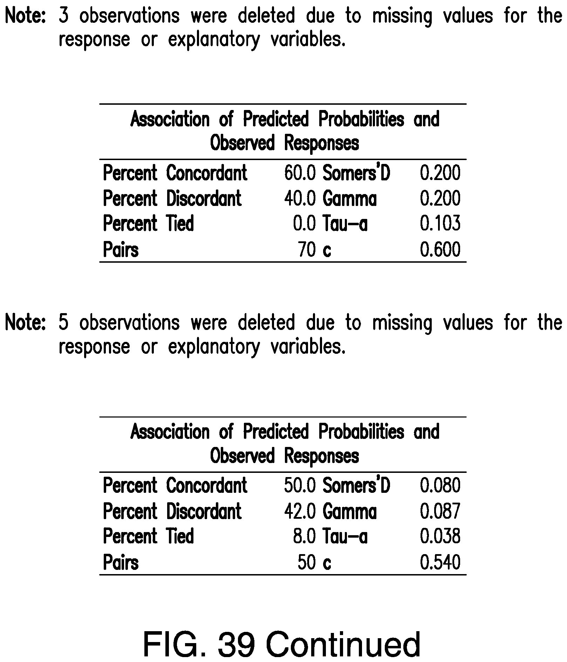

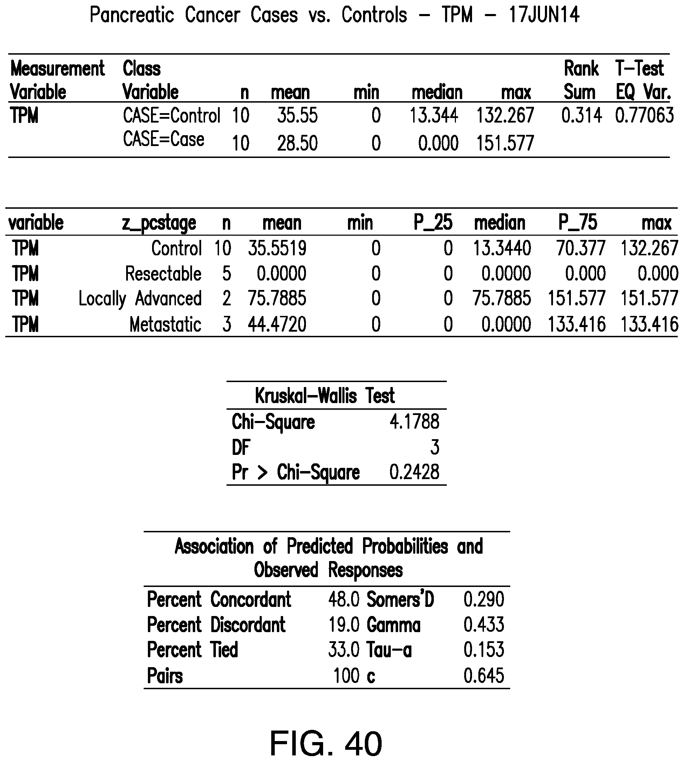

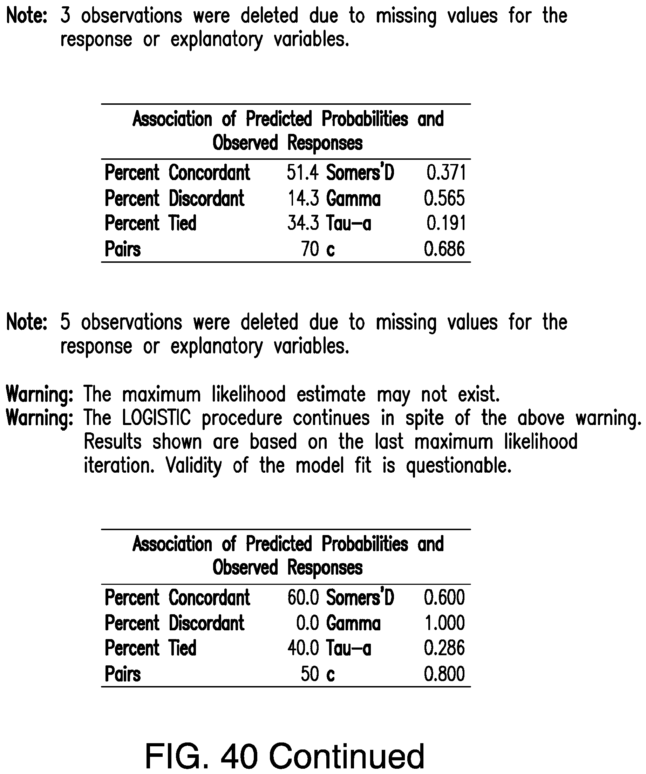

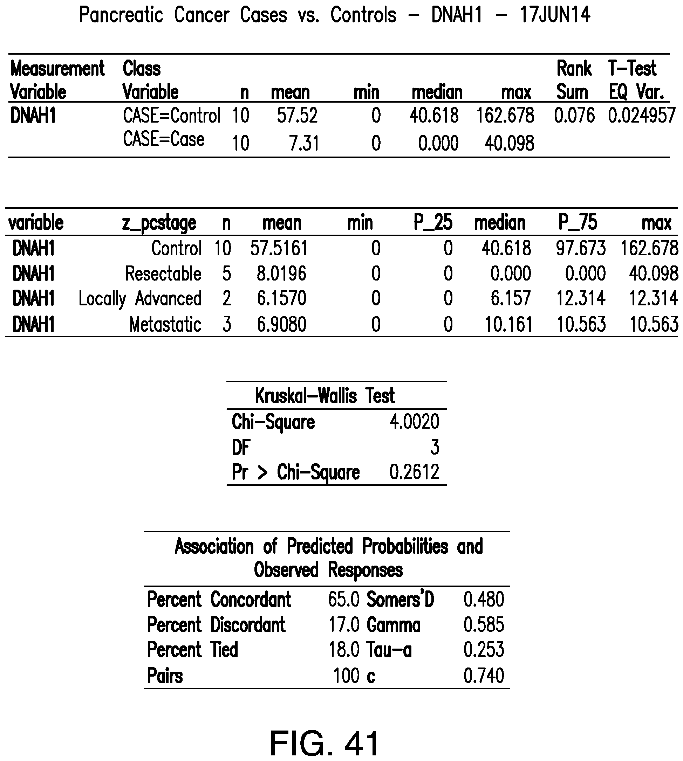

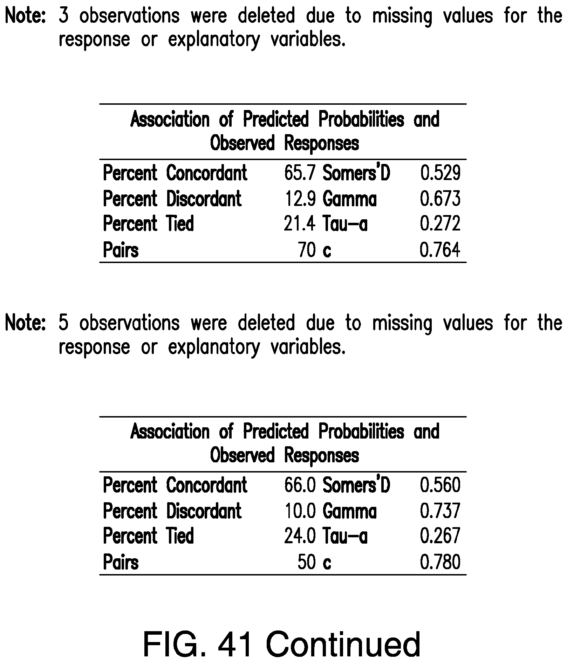

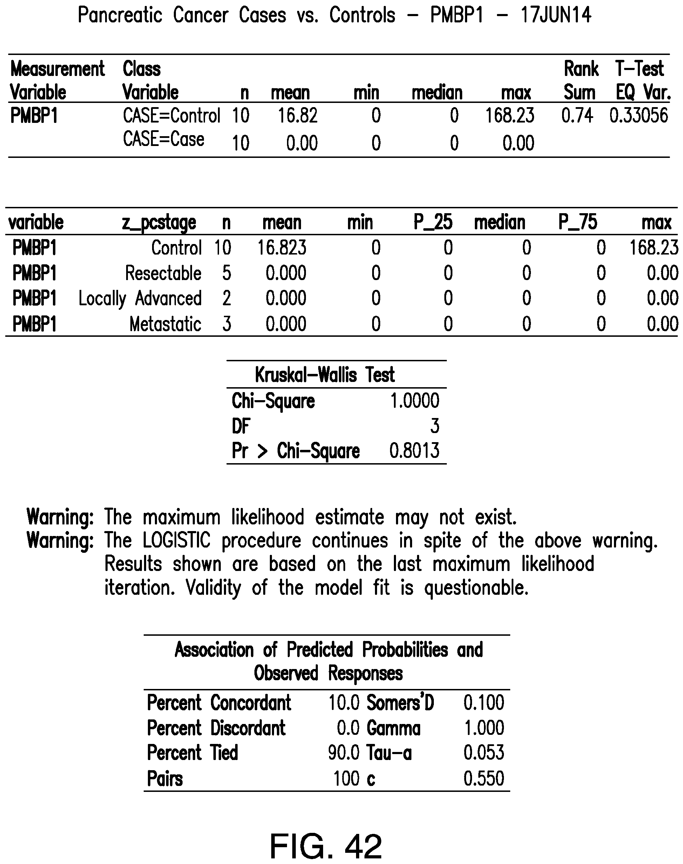

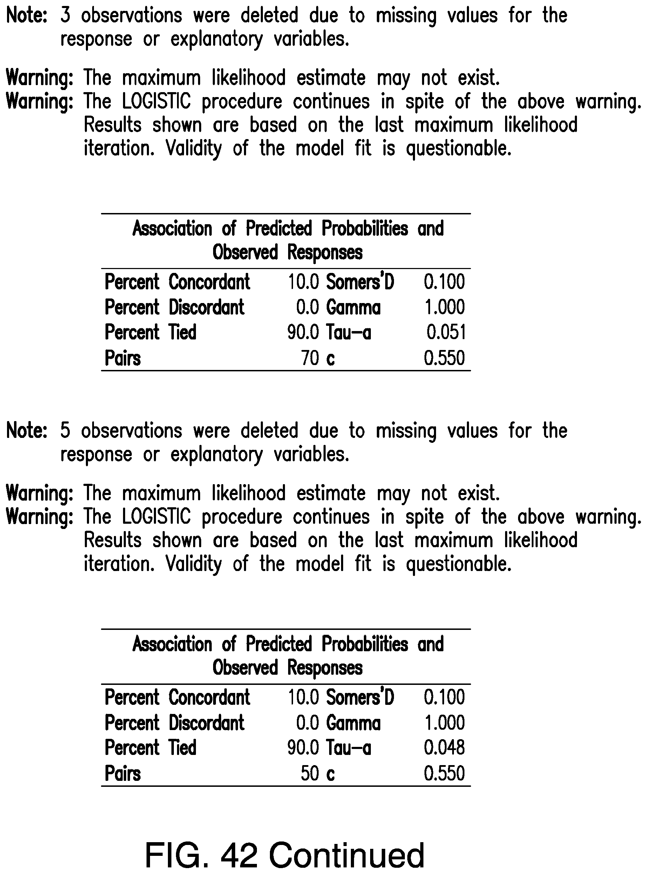

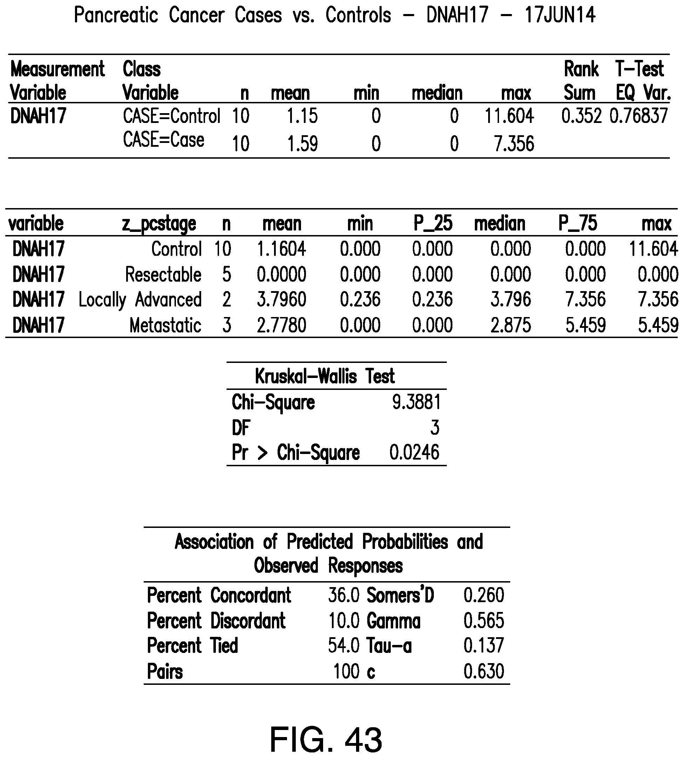

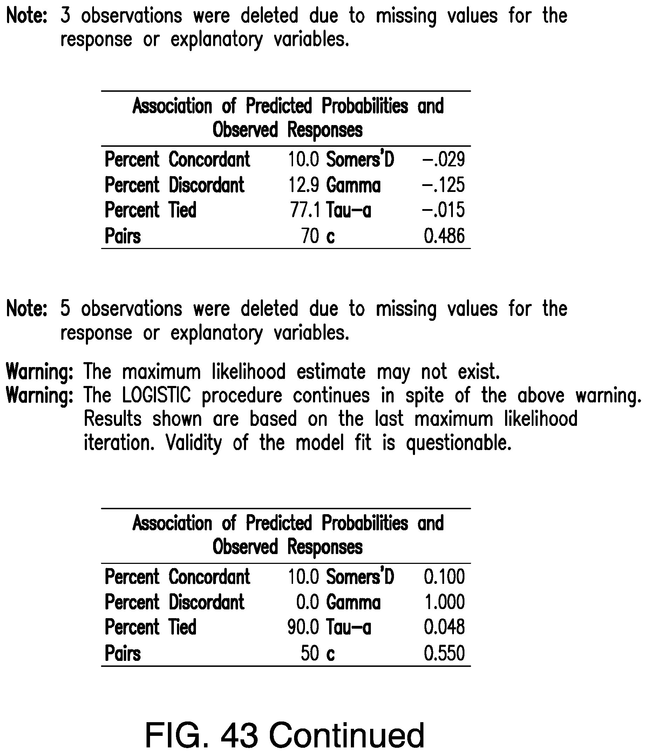

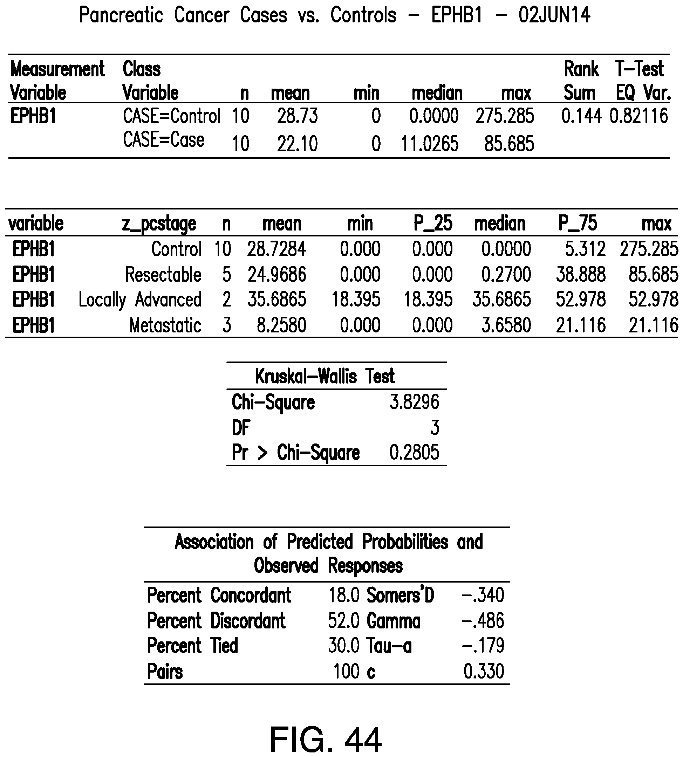

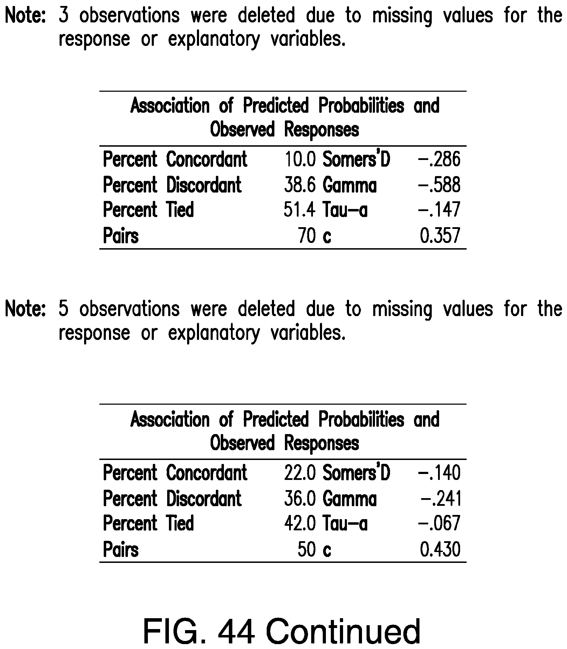

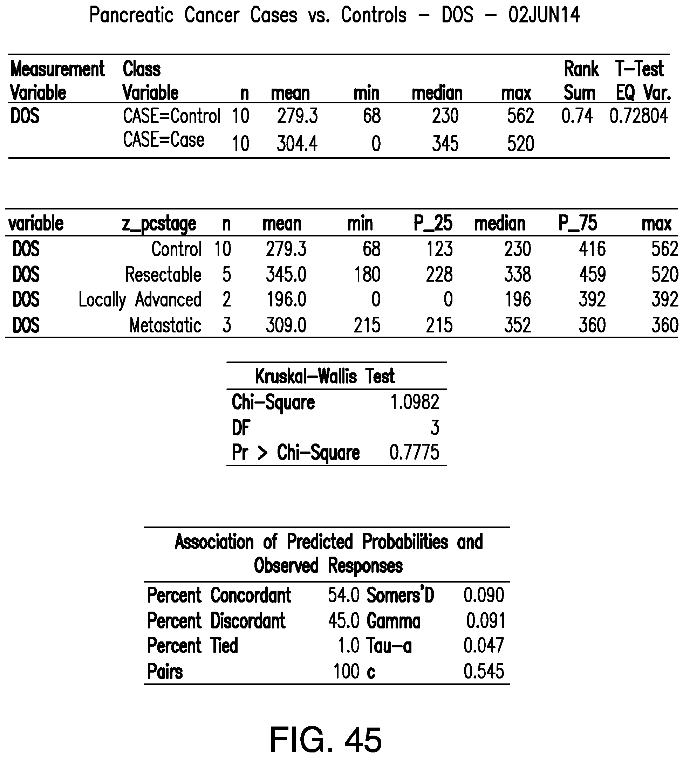

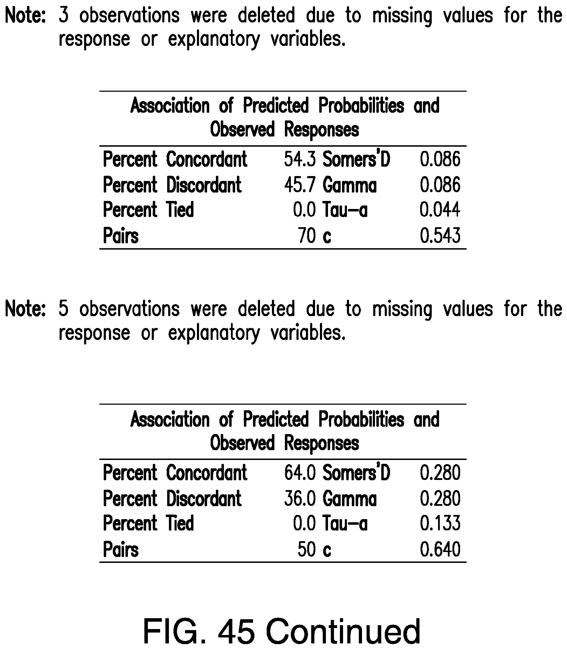

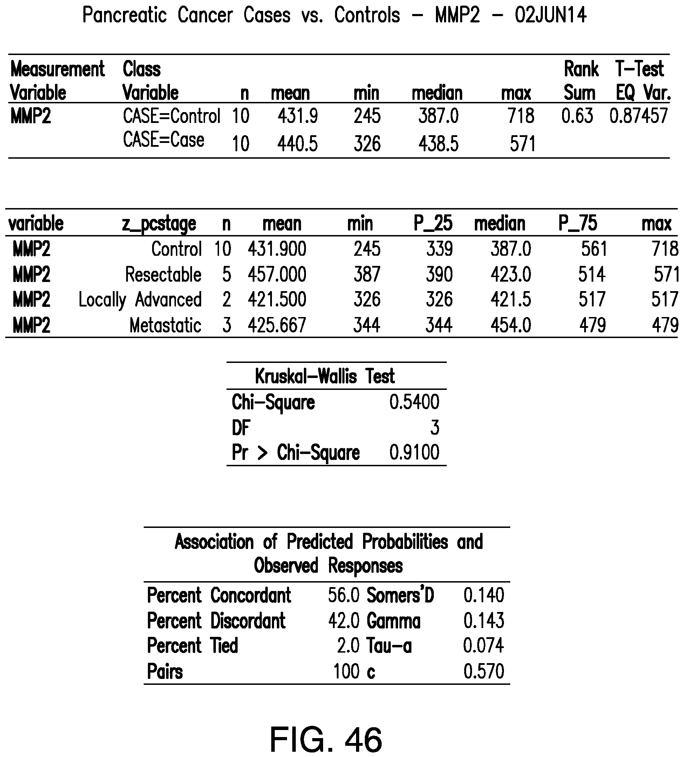

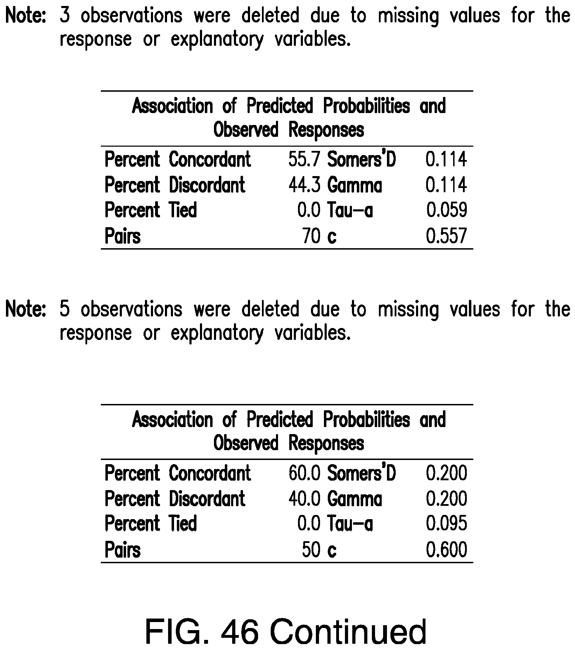

The thirty three biomarkers tested were AFP, RTTN, NLRX1, DNAH12, ODZ3, ADAMST9, TPM1, DNAH1, PMFBP1, DNAH17, EPHB1, DOS, MMP2, STARD8, ATP2A1, FKBP10, TCHP, TCF20, ABCA13, SCN8A, TOP2B, LIMCH1, UFD1L, FLRT3, ZHX2, SYNE1, THBS2, HMOX1, Obscurin, DNAH5, Shroom3, LOXL3, Malectin (Table 15 and Figures 13-46). Cancer antigen 19-9 (Ca199), which is a reliable tumor marker for pancreatic cancer, was used as positive control and resulted in a c-statistic value of 1.0 (Table 15 and Figure 33). Of the thirty three biomarkers tested, RTTN, DNAH12, TPM1, DNAH1, STARD8, AP2A1, TOP2B, LIMCH1, SYNE1, THBS2 and LOXL3 were identified as reliable biomarkers for indicating the presence of pancreatic cancer in a subject as they exhibited a c-statistic value greater than 0.7 (Table 15 and Figures 13-46). For example, TOP2B, a member of the TGFβ/integrin pathway, exhibited a c-statistic value of 0.860 in all pancreatic cancers, indicating that TOP2B is a reliable biomarker for early and advanced stage pancreatic cancer (Table 15 and Figure 23). Similar results were observed for DNAH1, STARD8, ATP2A1, **LIMCH1** and THBS2 (Table 15 and Figures 15, 16, 25, 26 and 41). RTTN, TPM1, LOXL3 and SYNE1 exhibited a c-statistic value greater than 0.7 in the plasma samples of subjects with resectable cancer indicating that RTTN, TPM1, LOXL3 and SYNE1 are reliable biomarkers for early stage, resectable pancreatic cancer (Table 15 and Figures 20, 29, 35 and 40). DNAH12, a member of the HNFa pathway exhibited a c-statistic value of 1.0 in plasma samples of all pancreatic cancer patients, *i.e*., patients with locally advanced and resectable pancreatic cancers, indicating that DNAH12 can be a reliable biomarker for pancreatic cancer (Table 15 and Figure 37).

### EXAMPLE 3: Detection of biomarkers in subjects with pancreatic cancer by mass spectrometry.

In this Example, detection of the disclosed biomarkers for pancreatic cancer was performed in plasma samples of subjects that had pancreatic cancer using mass spectrometry.

Two control plasmas (M1, M2) and two metastatic PDAC case plasmas (M3, M4) were selected and coded to allow blind testing. Each plasma sample was subjected to a serum albumin removal gel ("pull-down"), and the superntants were collected. The supernatant of each plasma sample was subjected to a mixture of protein A beads and protein G beads to remove IgG, and adjusted with 6M Urea, 10 mM DTT and 50 mM IAA to block free sulfhydryl groups. The supernatants were subsequently treated with trypsin at 1:50 and subjected to a C18 cartridge desalting step. 30 µg were aliquoted from each supernatant sample for each LC-MS/MS run. pFind was used to analyze the data and to identify peptide IDs. The reverse peptide decoys were compared with the forward peptides, a 5% FDR filter was applied and a list was generated that provided the peptide hits. Each plasma sample gave about 22,000 peptide hits (MS-1: 21294 peptide hits; MS-2: 23621 peptide hits; MS-3: 22342 peptide hits; and MS-4: 21991 peptide hits), which allowed comparisons between samples. As shown in Table 16, peptide hits for some of the 64 biomarker candidates were observed in the PDAC samples.

The most abundant proteins that were identified from the samples were Alpha-2-macroglobulin with 301 peptide hits, A2M with 2700 peptides hits, Ceruloplasmin with 1495 peptides hits and Albumin with 1398 peptides hits. These results indicate that there were a number of proteins received the greatest number of peptide hits, as noted above, and dominated the spectrum leading to the masking of low abundance proteins in the samples. This can be due to the insufficient depletion of plasma proteins from the plasma samples.

Of the 64 candidate proteins, the following were detected in the two tested PDAC samples (MS3 and MS4): DNAH1, VCAM1 and MYLK (Table 16). Of the 64 candidate proteins, the following were detected in one of the PDAC case samples (MS3 or MS4): ACTN2, ATP2A1, DNAH5, TCF20, SYNE2, SYNE1, KIAA1109, ABCA13, ZNF804A, SCYL2 and KIAA1671 (Table 16). Of the four candidates that were identified to have c-statistics > 0.7 by ELISA, as described in Example 2 and Table 15, DNAH1, ATP2A1, SYNE1 were detected in at least one of the PDAC plasma samples (MS3 or MS4) (Table 16). These data provide independent experimental results indicating that the identified proteins can serve as biomarkers for pancreatic cancer.

**Table 16**

| | | **Proteins** | **Peptide hits** | | | | | | **not** |
|---|---|---|---|---|---|---|---|---|---|
| **Gene** | **Pathway** | | **MS1** | **MS2** | **MS3** | **MS4** | **c ≥ 0.7** | **c < 0.7** | **tested** |
| DNAH1 | HNF4a | IPI00002127 | 0 | 1 | 2 | 9 | X | | |
| VCAM1 | TGFb/integrin | IPI00018136 | 3 | 5 | 2 | 1 | | | X |
| ACTN2 | HNF4a | IPI00019884 | 0 | 0 | 0 | 1 | | | X |
| ATP2A1 | no network | IPI00024804 | 1 | 0 | 1 | 0 | X | | |
| DNAH5 | HNF4a | IPID0152653 | 2 | 0 | 1 | 0 | | X | |
| TCF20 | RAS/P53/JUN | IP100159322 | 0 | 0 | 1 | 0 | | X | |
| MYLK | TGFb/integrin | IPI00221255 | 1 | 0 | 1 | 1 | | | X |
| PMFBP1 | RAS/P53/JUN | IPI00235481 | 1 | 0 | 0 | 0 | | X | |
| SYNE2 | TGFb/integrin | IPI00239405 | 0 | 1 | 0 | 2 | | | X |
| SYNE1 | TGFb/integrin | IPI00247295 | 2 | 2 | 0 | 1 | X | | |
| KIAA1109 | RAS/P53/JUN | IPI00251161 | 0 | 0 | 0 | 1 | | | X |
| ABCA13 | no network | IPI00328762 | 1 | 0 | 0 | 1 | | | X |
| ZNF804A | HNF4a | IPI00375560 | 2 | 0 | 0 | 1 | | | X |
| SCYL2 | HNF4a | IPI00396218 | 0 | 3 | 2 | 0 | | | X |
| KIAA1671 | no network | IPI00396634 | 0 | 0 | 0 | 1 | | | X |
| ODZ3 | RAS/P53/JUN | IPI00398020 | 1 | 0 | 0 | 0 | | X | |
| DNAH12 | HNF4a | IPI00412106 | 0 | 1 | 0 | 0 | X | | |
| DES | TGFb/integrin | IPI00465084 | 2 | 0 | 0 | 0 | | | X |
| | | | | | | | | | |
| CA19-9 | | IPI00007671 | 0 | 0 | 0 | 0 | | | |
| CA19-9 | | IPI00910296 | 0 | 0 | 0 | 0 | | | |

### REFERENCES

Banito, A., Rashid, S.T., Acosta, J.C., Li, S., Pereira, C.F., Geti, I., Pinho, S., Silva, J.C., Azuara, V., Walsh, M., et al. (2009). Senescence impairs successful reprogramming to pluripotent stem cells. Genes Dev 23, 2134-2139.
Bar-Nur, O., Russ, H.A., Efrat, S., and Benvenisty, N. (2011). Epigenetic memory and preferential lineage-specific differentiation in induced pluripotent stem cells derived from human pancreatic islet Beta cells. Cell Stem Cell 9, 17-23.
Bardeesy, N., Cheng, K.H., Berger, J.H., Chu, G.C., Pahler, J., Olson, P., Hezel, A.F., Horner, J., Lauwers, G.Y., Hanahan, D., et al. (2006). Smad4 is dispensable for normal pancreas development yet critical in progression and tumor biology of pancreas cancer. Genes Dev 20, 3130-3146.
Blelloch, R.H., Hochedlinger, K., Yamada, Y., Brennan, C., Kim, M., Mintz, B., Chin, L., and Jaenisch, R. (2004). Nuclear cloning of embryonal carcinoma cells. Proc Natl Acad Sci U S A 101, 13985-13990.
Bock, C., Kiskinis, E., Verstappen, G., Gu, H., Boulting, G., Smith, Z.D., Ziller, M., Croft, G.F., Amoroso, M.W., Oakley, D.H., et al. (2011). Reference Maps of human ES and iPS cell variation enable high-throughput characterization of pluripotent cell lines. Cell 144, 439-452.
Brambrink et al.(2008) Cell Stem Cell 2, 151.
Brat, D.J., Lillemoe, K.D., Yeo, C.J., Warfield, P.B., and Hruban, R.H. (1998). Progression of pancreatic intraductal neoplasias to infiltrating adenocarcinoma of the pancreas. Am J Surg Pathol 22, 163-169.
Buchholz, M., Braun, M., Heidenblut, A., Kestler, H.A., Kloppel, G., Schmiegel, W., Hahn, S.A., Luttges, J., and Gress, T.M. (2005). Transcriptome analysis of microdissected pancreatic intraepithelial neoplastic lesions. Oncogene 24, 6626-6636.
Carette, J.E., Pruszak, J., Varadarajan, M., Blomen, V.A., Gokhale, S., Camargo, F.D., Wernig, M., Jaenisch, R., and Brummelkamp, T.R. (2010). Generation of iPSCs from cultured human malignant cells. Blood 115, 4039-4042.
Ding, Y., Cravero, J.D., Adrian, K., and Grippo, P. (2010). Modeling pancreatic cancer in vivo: from xenograft and carcinogen-induced systems to genetically engineered mice. Pancreas 39, 283-292.
Eiselleova, L., Matulka, K., Kriz, V., Kunova, M., Schmidtova, Z., Neradil, J., Tichy, B., Dvorakova, D., Pospisilova, S., Hampl, A., et al. (2009). A complex role for FGF-2 in self renewal, survival, and adhesion of human embryonic stem cells. Stem Cells 27, 1847-1857.
Esteller, M. (2007). Cancer epigenomics: DNA methylomes and histone-modification maps. Nat Rev Genet 8, 286-298.
Gattani A M, Mandeli J, Bruckner H W. (1996). Tumor markers in patients with pancreatic carcinoma. Cancer 78: 57-62.
Gupta, R.K., Gao, N., Gorski, R.K., White, P., Hardy, O.T., Rafiq, K., Brestelli, J.E., Chen, G., Stoeckert, C.J., Jr., and Kaestner, K.H. (2007). Expansion of adult beta-cell mass in response to increased metabolic demand is dependent on HNF-4alpha. Genes Dev 21, 756-769.
Hahn, S.A., Schutte, M., Hoque, A.T., Moskaluk, C.A., da Costa, L.T., Rozenblum, E., Weinstein, C.L., Fischer, A., Yeo, C.J., Hruban, R.H., et al. (1996). DPC4, a candidate tumor suppressor gene at human chromosome 18q21.1. Science 271, 350-353.
Hahn, S.A., Seymour, A.B., Hoque, A.T., Schutte, M., da Costa, L.T., Redston, M.S., Caldas, C., Weinstein, C.L., Fischer, A., Yeo, C.J., et al. (1995). Allelotype of pancreatic adenocarcinoma using xenograft enrichment. Cancer Res 55, 4670-4675.
Harsha, H.C., Kandasamy, K., Ranganathan, P., Rani, S., Ramabadran, S., Gollapudi, S., Balakrishnan, L., Dwivedi, S.B., Telikicherla, D., Selvan, L.D., et al. (2009). A compendium of potential biomarkers of pancreatic cancer. PLoS Med 6, e1000046.
Hatziapostolou, M., Polytarchou, C., Aggelidou, E., Drakaki, A., Poultsides, G.A., Jaeger, S.A., Ogata, H., Karin, M., Struhl, K., Hadzopoulou-Cladaras, M., et al. (2011). An HNF4alpha-miRNA inflammatory feedback circuit regulates hepatocellular oncogenesis. Cell 147, 1233-1247.
Hermann, P.C., Huber, S.L., Herrler, T., Aicher, A., Ellwart, J.W., Guba, M., Bruns, C.J., and Heeschen, C. (2007). Distinct populations of cancer stem cells determine tumor growth and metastatic activity in human pancreatic cancer. Cell Stem Cell 1, 313-323.
Hezel, A.F., Deshpande, V., Zimmerman, S.M., Contino, G., Alagesan, B., O'Dell, M.R., Rivera, L.B., Harper, J., Lonning, S., Brekken, R.A., et al. (2012). TGF-beta and alphavbeta6 Integrin Act in a Common Pathway to Suppress Pancreatic Cancer Progression. Cancer Res 72, 4840-4845.
Hezel, A.F., Kimmelman, A.C., Stanger, B.Z., Bardeesy, N., and Depinho, R.A. (2006). Genetics and biology of pancreatic ductal adenocarcinoma. Genes Dev 20, 1218-1249.
Hingorani, S.R., Petricoin, E.F., Maitra, A., Rajapakse, V., King, C., Jacobetz, M.A., Ross, S., Conrads, T.P., Veenstra, T.D., Hitt, B.A., et al. (2003). Preinvasive and invasive ductal pancreatic cancer and its early detection in the mouse. Cancer Cell 4,437-450.
Hochedlinger, K., Blelloch, R., Brennan, C., Yamada, Y., Kim, M., Chin, L., and Jaenisch, R. (2004). Reprogramming of a melanoma genome by nuclear transplantation. Genes Dev 18, 1875-1885.
Hruban, R.H., Adsay, N.V., Albores-Saavedra, J., Compton, C., Garrett, E.S., Goodman, S.N., Kern, S.E., Klimstra, D.S., Kloppel, G., Longnecker, D.S., et al. (2001). Pancreatic intraepithelial neoplasia: a new nomenclature and classification system for pancreatic duct lesions. Am J Surg Pathol 25, 579-586.
Iacobuzio-Donahue, C.A., Maitra, A., Olsen, M., Lowe, A.W., van Heek, N.T., Rosty, C., Walter, K., Sato, N., Parker, A., Ashfaq, R., et al. (2003). Exploration of global gene expression patterns in pancreatic adenocarcinoma using cDNA microarrays. Am J Pathol 162, 1151-1162.
Ishizawa, K., Rasheed, Z.A., Karisch, R., Wang, Q., Kowalski, J., Susky, E., Pereira, K., Karamboulas, C., Moghal, N., Rajeshkumar, N.V., et al. (2010). Tumor-initiating cells are rare in many human tumors. Cell Stem Cell 7, 279-282.
Jones, S., Zhang, X., Parsons, D.W., Lin, J.C., Leary, R.J., Angenendt, P., Mankoo, P., Carter, H., Kamiyama, H., Jimeno, A., et al. (2008). Core signaling pathways in human pancreatic cancers revealed by global genomic analyses. Science 321, 1801-1806.
Kanda et al. (2012) Gastroenterology 142, 730.
Kim, G.E., Bae, H.I., Park, H.U., Kuan, S.F., Crawley, S.C., Ho, J.J., and Kim, Y.S. (2002). Aberrant expression of MUC5AC and MUC6 gastric mucins and sialyl Tn antigen in intraepithelial neoplasms of the pancreas. Gastroenterology 123, 1052-1060.
Kim, K., Zhao, R., Doi, A., Ng, K., Unternaehrer, J., Cahan, P., Huo, H., Loh, Y.H., Aryee, M.J., Lensch, M.W., et al. (2011). Donor cell type can influence the epigenome and differentiation potential of human induced pluripotent stem cells. Nat Biotechnol29, 1117-1119.
Kim, M.P., Evans, D.B., Wang, H., Abbruzzese, J.L., Fleming, J.B., and Gallick, G.E. (2009). Generation of orthotopic and heterotopic human pancreatic cancer xenografts in immunodeficient mice. Nat Protoc 4, 1670-1680.
Kopp, J.L., von Figura, G., Mayes, E., Liu, F.F., Dubois, C.L., Morris, J.P.t., Pan, F.C., Akiyama, H., Wright, C.V., Jensen, K., et al. (2012). Identification of Sox9-Dependent Acinarto-Ductal Reprogramming as the Principal Mechanism for Initiation of Pancreatic Ductal Adenocarcinoma. Cancer Cell 22, 737-750.
Kunath, T., Saba-El-Leil, M.K., Almousailleakh, M., Wray, J., Meloche, S., and Smith, A. (2007). FGF stimulation of the Erk1/2 signalling cascade triggers transition of pluripotent embryonic stem cells from self-renewal to lineage commitment. Development 134, 2895-2902.
Lee et al. (2007) Nature Methods 4, 359.
Li, C., Heidt, D.G., Dalerba, P., Burant, C.F., Zhang, L., Adsay, V., Wicha, M., Clarke, M.F., and Simeone, D.M. (2007). Identification of pancreatic cancer stem cells. Cancer Res 67, 1030-1037.
Li, L., Connelly, M.C., Wetmore, C., Curran, T., and Morgan, J.I. (2003). Mouse embryos cloned from brain tumors. Cancer Res 63, 2733-2736.
Lieber, M., Mazzetta, J., Nelson-Rees, W., Kaplan, M., and Todaro, G. (1975). Establishment of a continuous tumor-cell line (panc-1) from a human carcinoma of the exocrine pancreas. Int J Cancer 15, 741-747.
Logsdon, C.D., Simeone, D.M., Binkley, C., Arumugam, T., Greenson, J.K., Giordano, T.J., Misek, D.E., Kuick, R., and Hanash, S. (2003). Molecular profiling of pancreatic adenocarcinoma and chronic pancreatitis identifies multiple genes differentially regulated in pancreatic cancer. Cancer Res 63, 2649-2657.
Lonardo, E., Hermann, P.C., Mueller, M.T., Huber, S., Balic, A., Miranda-Lorenzo, I., Zagorac, S., Alcala, S., Rodriguez-Arabaolaza, I., Ramirez, J.C., et al. (2011). Nodal/Activin signaling drives self-renewal and tumorigenicity of pancreatic cancer stem cells and provides a target for combined drug therapy. Cell Stem Cell 9, 433-446.
Lonardo E, H.P., Mueller MT, Huber S, Balic A, Miranda-Lorenzo I, Zagorac S, Alcala S, Rodriguez-Arabaolaza I, Ramirez JC, Torres-Ruíz R, Garcia E, Hidalgo M, Cebrián DA, Heuchel R, Löhr M, Berger F, Bartenstein P, Aicher A, Heeschen C. (2011). Nodal/Activin signaling drives self-renewal and tumorigenicity of pancreatic cancer stem cells and provides a target for combined drug therapy. Cell Stem Cell 9, 433-446.
Maitra, A., and Hruban, R.H. (2008). Pancreatic cancer. Annu Rev Pathol 3, 157-188.
Maser, R.S., Choudhury, B., Campbell, P.J., Feng, B., Wong, K.K., Protopopov, A., O'Neil, J., Gutierrez, A., Ivanova, E., Perna, I., et al. (2007). Chromosomally unstable mouse tumours have genomic alterations similar to diverse human cancers. Nature 447, 966-971.
McKinnell, R.G., Deggins, B.A., and Labat, D.D. (1969). Transplantation of pluripotential nuclei from triploid frog tumors. Science 165, 394-396.
Miyatsuka, T., Kaneto, H., Shiraiwa, T., Matsuoka, T.A., Yamamoto, K., Kato, K., Nakamura, Y., Akira, S., Takeda, K., Kajimoto, Y., et al. (2006). Persistent expression of PDX-1 in the pancreas causes acinar-to-ductal metaplasia through Stat3 activation. Genes Dev 20, 1435-1440.
Miyoshi, N., Ishii, H., Nagai, K., Hoshino, H., Mimori, K., Tanaka, F., Nagano, H., Sekimoto, M., Doki, Y., and Mori, M. (2010). Defined factors induce reprogramming of gastrointestinal cancer cells. Proc Natl Acad Sci USA 107, 40-45.
Morris, J.P.t., Wang, S.C., and Hebrok, M. (2010). KRAS, Hedgehog, Wnt and the twisted developmental biology of pancreatic ductal adenocarcinoma. Nat Rev Cancer 10, 683-695.
Moskaluk, C.A., Hruban, R.H., and Kern, S.E. (1997). p16 and K-ras gene mutations in the intraductal precursors of human pancreatic adenocarcinoma. Cancer Res 57, 2140-2143.
Odom, D.T., Zizlsperger, N., Gordon, D.B., Bell, G.W., Rinaldi, N.J., Murray, H.L., Volkert, T.L., Schreiber, J., Rolfe, P.A., Gifford, D.K., et al. (2004). Control of pancreas and liver gene expression by HNF transcription factors. Science 303, 1378-1381.
Park et al. (2008) Cell 134, 877.
Rhim, A.D., Mirek, E.T., Aiello, N.M., Maitra, A., Bailey, J.M., McAllister, F., Reichert, M., Beatty, G.L., Rustgi, A.K., Vonderheide, R.H., et al. (2012). EMT and dissemination precede pancreatic tumor formation. Cell 148, 349-361.
Rubio-Viqueira, B., Jimeno, A., Cusatis, G., Zhang, X., Iacobuzio-Donahue, C., Karikari, C., Shi, C., Danenberg, K., Danenberg, P.V., Kuramochi, H., et al. (2006). An in vivo platform for translational drug development in pancreatic cancer. Clin Cancer Res 12, 4652-4661.
Rustgi, A.K. (2006). The molecular pathogenesis of pancreatic cancer: clarifying a complex circuitry. Genes Dev 20, 3049-3053.
Serrano, M., Lin, A.W., McCurrach, M.E., Beach, D., and Lowe, S.W. (1997). Oncogenic ras provokes premature cell senescence associated with accumulation of p53 and p16INK4a. Cell 88, 593-602.
Shultz, L.D., Lyons, B.L., Burzenski, L.M., Gott, B., Chen, X., Chaleff, S., Kotb, M., Gillies, S.D., King, M., Mangada, J., et al. (2005). Human lymphoid and myeloid cell development in NOD/LtSz-scid IL2R gamma null mice engrafted with mobilized human hemopoietic stem cells. J Immunol 174, 6477-6489.
Strader (2006) Anal. Chem 78, 125.
Taguchi, A., Politi, K., Pitteri, S.J., Lockwood, W.W., Faca, V.M., Kelly-Spratt, K., Wong, C.H., Zhang, Q., Chin, A., Park, K.S., et al. (2011). Lung cancer signatures in plasma based on proteome profiling of mouse tumor models. Cancer Cell 20, 289-299.
Takahashi, K., and Yamanaka, S. (2006). Induction of pluripotent stem cells from mouse embryonic and adult fibroblast cultures by defined factors. Cell 126, 663-676.
Thomson et al.(1998) Science 282, 1145.
Tost and Gut (2007) Nat Protocols 2, 2265.
Uhlen, M., Oksvold, P., Fagerberg, L., Lundberg, E., Jonasson, K., Forsberg, M., Zwahlen, M., Kampf, C., Wester, K., Hober, S., et al. (2010). Towards a knowledge-based Human Protein Atlas. Nat Biotechnol 28, 1248-1250.
Urlinger et al. (2000) Proc Natl Acad Sci U S A 97, 7963.
Yunis, A.A., Arimura, G.K., and Russin, D.J. (1977). Human pancreatic carcinoma (MIA PaCa-2) in continuous culture: sensitivity to asparaginase. Int J Cancer 19, 128-135.

Various publications, patents and patent applications are cited herein, the contents of which are hereby incorporated by reference herein in their entireties.

## Claims

1. A method of determining whether a subject has pancreatic cancer, comprising obtaining one or more biological samples obtained from the subject and detecting, in the one or more biological samples, one or more biomarkers selected from the group consisting of MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13, DES, IMMT, TPM1, SNRPE, VCAM1, GRB2, SHROOM3, HMOX1, POSTN, MMP10, MMP-2, THBS2, EWSR1, NOD1, ADAMTS9, AFP, SYNE1, SYNE2, EPHB1, UFD1L, TEAD1, RYR3, CMYA5, MYLK, TOP2B, KIAA1109, ODZ3, PMFBP1, EPHB3, LIMCH1, TCF20, ERP29, OBSCN, LOXL3, MLEC, DNAH1, DNAH5, DNAH12, DNAH17, SCYL2, FKBP10, FLRT3, ZHX2 (AFR1), ZNF804A, ACTN2, CA19-9 and combinations thereof, wherein the detection of the one or more biomarkers is an indication that the subject has pancreatic cancer.

2. A method of determining whether a subject has pancreatic cancer, comprising obtaining one or more biological samples obtained from the subject and detecting, in the one or more biological samples, one or more biomarkers selected from the group consisting of RTTN, DNAH12, TPM1, DNAH1, STARD8, ATP2A1, TOP2B, LIMCH1, SYNE1, THBS2, LOXL3, CA19-9 and combinations thereof, wherein the presence of the one or more biomarkers is an indication that the subject has pancreatic cancer.

3. A method of determining whether a subject has pancreatic cancer, the method comprising obtaining a biological sample obtained from the subject and determining if the biological sample from the subject contains at least three biomarkers, wherein one of the at least three biomarkers is a member of the TGFβ/integrin signaling pathway, one of the at least three biomarkers is a member of the HNF4α transcription factor network and the one of the at least three biomarkers is a member of the Ras/p53/JUN/CTNB 1 signaling pathway, and wherein the presence of the at least three biomarkers in the sample is an indication that the subject has pancreatic cancer.

4. The method of claim 1, 2 or 3, wherein the subject is human; and/or
wherein the biological sample is selected from the group consisting of a stool sample, a blood sample, a plasma sample, a pancreatic cyst fluid sample and combinations thereof; and/or
wherein the biomarker is a protein; and/or
wherein the biomarker comprises a transcribed polynucleotide or portion thereof.

5. The method of claim 4, wherein the presence of the protein biomarker is detected using a reagent which specifically binds to the protein biomarker; and optionally
wherein the reagent is a monoclonal antibody or antigen-binding fragment thereof, or a polyclonal antibody or antigen-binding fragment thereof; and optionally wherein the presence of the protein is detected by enzyme-linked immunosorbent assay.

6. The method of claims 1 or 2, wherein the biomarker comprises THBS2 or THBS2 and CA19-9, and optionally comprises one or more biomarkers selected from the group consisting of ACTN2, SVEP1, OBSCN, IMPA1, U2SURP, PMFBP1, DNAH17, ZNF160, RTTN, ABCA13, RYR3 and combinations thereof.

7. A kit for diagnosing whether a subject has pancreatic cancer or assessing the efficacy of a therapeutic treatment, comprising reagents useful for detecting one or more biomarkers selected from the group consisting of MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13, DES, IMMT, TPM1, SNRPE, VCAM1, GRB2, SHROOM3, HMOX1, POSTN, MMP10, MMP-2, THBS2, EWSR1, NOD1, ADAMTS9, AFP, SYNE1, SYNE2, EPHB1, UFD1L, TEAD1, RYR3, CMYA5, MYLK, TOP2B, KIAA1109, ODZ3, PMFBP1, EPHB3, LIMCH1, TCF20, ERP29, OBSCN, LOXL3, MLEC, DNAH1, DNAH5, DNAH12, DNAH17, SCYL2, FKBP10, FLRT3, ZHX2 (AFR1), ZNF804A, ACTN2, CA19-9 and combinations thereof, in one or more biological samples from the subject.

8. The kit of claim 7, comprising one or more of packaged probe and primer sets, arrays/microarrays, biomarker-specific antibodies or beads for detecting the one or more biomarkers; or
comprising at least one monoclonal antibody or antigen-binding fragment thereof, or a polyclonal antibody or antigen-binding fragment thereof, for detecting the one or more biomarkers to be identified.

9. A method of determining whether a subject has pancreatic cancer comprising, obtaining a biological sample obtained from the subject and determining if the biological sample from the subject contains at least two biomarkers, wherein one of the at least two biomarkers is a member of the TGFβ/integrin signaling pathway and the other biomarker of the at least two biomarkers is a member of the HNF4α transcription factor network, and wherein the presence of the at least two biomarkers in the sample is an indication that the subject has pancreatic cancer.

10. A method of determining whether a subject has pancreatic cancer, the method comprising obtaining a biological sample obtained from the subject and determining if the biological sample from the subject contains at least two biomarkers, wherein one of the at least two biomarkers is a member of the Ras/p53/JUN/CTNB1 signaling pathway and the other biomarker of the at least two biomarkers is a member of the HNF4α transcription factor network, and wherein the presence of the at least two biomarkers in the sample is an indication that the subject has pancreatic cancer.

11. A method of determining whether a subject has pancreatic cancer, the method comprising obtaining a biological sample obtained from the subject and determining if the biological sample from the subject contains at least two biomarkers, wherein one of the at least two biomarkers is a member of a pathway selected from the group consisting of the Ras/p53/JUN/CTNB 1 signaling pathway, the HNF4α transcription factor network, the TGFβ/integrin signaling pathway and combinations thereof and the other biomarker of the at least two biomarkers is selected from the group consisting of MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13, CA19-9 and combinations thereof, wherein the presence of the at least two biomarkers in the sample is an indication that the subject has pancreatic cancer.

12. A method of assessing the efficacy of a therapy for preventing or treating pancreatic cancer in a subject, comprising:
(a) determining the level of one or more biomarkers in a biological sample obtained from the subject, wherein the biomarkers are selected from the group consisting of MANF, ZNF485, IMPA1, SVEP1, KIAA1671, KIAA1529, GNN, DOS, STARD8 (DLC3), SCN8A, U2SURP, TCHP, IPI00026665, RAD51C, ATP2A1, NLRX1, ZNF160, RTTN, ABCA13, DES, IMMT, TPM1, SNRPE, VCAM1, GRB2, SHROOM3, HMOX1, POSTN, MMP10, MMP-2, THBS2, EWSR1, NOD1, ADAMTS9, AFP, SYNE1, SYNE2, EPHB1, UFD1L, TEAD1, RYR3, CMYA5, MYLK, TOP2B, KIAA1109, ODZ3, PMFBP1, EPHB3, LIMCH1, TCF20, ERP29, OBSCN, LOXL3, MLEC, DNAH1, DNAH5, DNAH12, DNAH17, SCYL2, FKBP10, FLRT3, ZHX2 (AFR1), ZNF804A, ACTN2, CA19-9 and combinations thereof, prior to therapy; and
(b) determining the level of the one or more biomarkers in a biological sample obtained from the subject, at one of more time points during therapy,
wherein the therapy is efficacious for preventing or treating the cancer in the subject when there is a lower level of the one or more biomarkers in the second or subsequent samples, relative to the first sample.

13. A method for producing a pancreatic tumor cell model system comprising:
(a) isolating pancreatic ductal adenocarcinoma cells from a pancreatic ductal adenocarcinoma sample;
(b) overexpressing Klf4, Sox2, Oct4 and c-Myc in the isolated pancreatic ductal adenocarcinoma cells to produce induced pluripotent stem cells;
(c) injecting the induced pluripotent stem cells into an immunocompromised animal to produce a teratoma in the animal;
(d) isolating the teratoma from the animal; and
(e) culturing the teratoma to obtain the pancreatic tumor cell model system.

14. A pancreatic tumor cell model system generated by the method of claim 13.

15. The use of the pancreatic tumor cell model system of claim 14 to identify one or more biomarkers for pancreatic cancer.


SEQUENCE LISTING

<110> THE TRUSTEES OF THE UNIVERSITY OF PENNSYLVANIA

<120> METHODS FOR DIAGNOSING PANCREATIC CANCER

<130> JA81363P.EPPD1

<140>
<141> 2014-06-20

<150> EP14813181.6
<151> 2014-06-20

<150> PCT/US2014/043457
<151> 2014-06-20

<150> 61/837,358
<151> 2013-06-20

<160> 89

<170> PatentIn version 3.5

<210> 1
<211> 24
<212> DNA
<213> Artificial Sequence

<220>
<221> source
<223> /note="Description of Artificial Sequence: Synthetic
      primer"

<400> 1
gacaggggga ggggaggagc tagg                                              24


<210> 2
<211> 26
<212> DNA
<213> Artificial Sequence

<220>
<221> source
<223> /note="Description of Artificial Sequence: Synthetic
      primer"

<400> 2
cttccctcca accagttgcc ccaaac                                            26


<210> 3
<211> 26
<212> DNA
<213> Artificial Sequence

<220>
<221> source
<223> /note="Description of Artificial Sequence: Synthetic
      primer"

<400> 3

gggaaatggg aggggtgcaa aagagg                                          26

<210> 4
<211> 26
<212> DNA
<213> Artificial Sequence

<220>
<221> source
<223> /note="Description of Artificial Sequence: Synthetic
      primer"

<400> 4
ttgcgtgagt gtggatggga ttggtg                                          26

<210> 5
<211> 20
<212> DNA
<213> Artificial Sequence

<220>
<221> source
<223> /note="Description of Artificial Sequence: Synthetic
      primer"

<400> 5
tatgacccac actgccagaa                                                 20

<210> 6
<211> 20
<212> DNA
<213> Artificial Sequence

<220>
<221> source
<223> /note="Description of Artificial Sequence: Synthetic
      primer"

<400> 6
tgggaacttg accatgattg                                                 20

<210> 7
<211> 26
<212> DNA
<213> Artificial Sequence

<220>
<221> source
<223> /note="Description of Artificial Sequence: Synthetic
      primer"

<400> 7
gcgtcctggg aagggagatc cggagc                                          26

<210> 8
<211> 26
<212> DNA
<213> Artificial Sequence

<220>
<221> source
<223> /note="Description of Artificial Sequence: Synthetic
      primer"

<400> 8
ttgaggggca tcgtcgcggg aggctg                                          26


<210> 9
<211> 25
<212> DNA
<213> Artificial Sequence

<220>
<221> source
<223> /note="Description of Artificial Sequence: Synthetic
      primer"

<400> 9
cagccccgat tcttccacca gtccc                                           25


<210> 10
<211> 25
<212> DNA
<213> Artificial Sequence

<220>
<221> source
<223> /note="Description of Artificial Sequence: Synthetic
      primer"

<400> 10
cggaagattc ccagtcgggt tcacc                                           25


<210> 11
<211> 24
<212> DNA
<213> Artificial Sequence

<220>
<221> source
<223> /note="Description of Artificial Sequence: Synthetic
      primer"

<400> 11
cagatcctaa acagctcgca gaat                                            24


<210> 12
<211> 23
<212> DNA
<213> Artificial Sequence

<220>
<221> source
<223> /note="Description of Artificial Sequence: Synthetic
      primer"

<400> 12

gcgtacgcaa attaaagtcc aga                                              23

<210> 13
<211> 20
<212> DNA
<213> Artificial Sequence

<220>
<221> source
<223> /note="Description of Artificial Sequence: Synthetic
      primer"

<400> 13
tgtgcaccaa catctacaag                                                   20

<210> 14
<211> 20
<212> DNA
<213> Artificial Sequence

<220>
<221> source
<223> /note="Description of Artificial Sequence: Synthetic
      primer"

<400> 14
gcgttcttgg ctttcaggat                                                   20

<210> 15
<211> 20
<212> DNA
<213> Artificial Sequence

<220>
<221> source
<223> /note="Description of Artificial Sequence: Synthetic
      primer"

<400> 15
aaatgtttgt gttgcggtca                                                   20

<210> 16
<211> 20
<212> DNA
<213> Artificial Sequence

<220>
<221> source
<223> /note="Description of Artificial Sequence: Synthetic
      primer"

<400> 16
tctggcacag gtgtcttcag                                                   20

<210> 17
<211> 21
<212> DNA
<213> Artificial Sequence

<220>
<221> source
<223> /note="Description of Artificial Sequence: Synthetic
      primer"

<400> 17
tgttgccatc aatgacccct t                                                21


<210> 18
<211> 19
<212> DNA
<213> Artificial Sequence

<220>
<221> source
<223> /note="Description of Artificial Sequence: Synthetic
      primer"

<400> 18
ctccacgacg tactcagcg                                                   19


<210> 19
<211> 20
<212> DNA
<213> Artificial Sequence

<220>
<221> source
<223> /note="Description of Artificial Sequence: Synthetic
      primer"

<400> 19
cgaatgccag agaaggtcac                                                  20


<210> 20
<211> 20
<212> DNA
<213> Artificial Sequence

<220>
<221> source
<223> /note="Description of Artificial Sequence: Synthetic
      primer"

<400> 20
ccatgagaat ccgcttgttt                                                  20


<210> 21
<211> 26
<212> DNA
<213> Artificial Sequence

<220>
<221> source
<223> /note="Description of Artificial Sequence: Synthetic
      primer"

<400> 21

atggaaactc tattaaagtg aacctg                                          26

<210> 22
<211> 25
<212> DNA
<213> Artificial Sequence

<220>
<221> source
<223> /note="Description of Artificial Sequence: Synthetic
      primer"

<400> 22
tagacctcat actcagcatt ccagt                                           25

<210> 23
<211> 20
<212> DNA
<213> Artificial Sequence

<220>
<221> source
<223> /note="Description of Artificial Sequence: Synthetic
      primer"

<400> 23
cagatgctgt gtccctgtgt                                                 20

<210> 24
<211> 20
<212> DNA
<213> Artificial Sequence

<220>
<221> source
<223> /note="Description of Artificial Sequence: Synthetic
      primer"

<400> 24
ttcagatcca gaaggggatg                                                 20

<210> 25
<211> 23
<212> DNA
<213> Artificial Sequence

<220>
<221> source
<223> /note="Description of Artificial Sequence: Synthetic
      primer"

<400> 25
ccagaaagga tgcctcataa agg                                             23

<210> 26
<211> 18
<212> DNA
<213> Artificial Sequence

<220>
<221> source
<223> /note="Description of Artificial Sequence: Synthetic
      primer"

<400> 26
tctgcgcgcc cctagtta                                                    18


<210> 27
<211> 19
<212> DNA
<213> Artificial Sequence

<220>
<221> source
<223> /note="Description of Artificial Sequence: Synthetic
      primer"

<400> 27
atgcaccgct acgacatgg                                                   19


<210> 28
<211> 21
<212> DNA
<213> Artificial Sequence

<220>
<221> source
<223> /note="Description of Artificial Sequence: Synthetic
      primer"

<400> 28
ctcatgtagc cctgcgagtt g                                                21


<210> 29
<211> 20
<212> DNA
<213> Artificial Sequence

<220>
<221> source
<223> /note="Description of Artificial Sequence: Synthetic
      primer"

<400> 29
ccctagggga tgttccagat                                                  20


<210> 30
<211> 20
<212> DNA
<213> Artificial Sequence

<220>
<221> source
<223> /note="Description of Artificial Sequence: Synthetic
      primer"

<400> 30

tgaagctttt ccctcttcca                                                    20

<210> 31
<211> 20
<212> DNA
<213> Artificial Sequence

<220>
<221> source
<223> /note="Description of Artificial Sequence: Synthetic
      primer"

<400> 31
acccagttca tagcggtgac                                                    20

<210> 32
<211> 20
<212> DNA
<213> Artificial Sequence

<220>
<221> source
<223> /note="Description of Artificial Sequence: Synthetic
      primer"

<400> 32
ccattgggag tacccaggtt                                                    20

<210> 33
<211> 23
<212> DNA
<213> Artificial Sequence

<220>
<221> source
<223> /note="Description of Artificial Sequence: Synthetic
      primer"

<400> 33
aaggtgacag gaaaagacga act                                                23

<210> 34
<211> 23
<212> DNA
<213> Artificial Sequence

<220>
<221> source
<223> /note="Description of Artificial Sequence: Synthetic
      primer"

<400> 34
tcccctccat tggcccgctt aac                                                23

<210> 35
<211> 20
<212> DNA
<213> Artificial Sequence

<220>
<221> source
<223> /note="Description of Artificial Sequence: Synthetic
      primer"

<400> 35
ccggagccag gactacatta                                                   20


<210> 36
<211> 20
<212> DNA
<213> Artificial Sequence

<220>
<221> source
<223> /note="Description of Artificial Sequence: Synthetic
      primer"

<400> 36
ggtcttgaag tcagccgtgt                                                   20


<210> 37
<211> 20
<212> DNA
<213> Artificial Sequence

<220>
<221> source
<223> /note="Description of Artificial Sequence: Synthetic
      primer"

<400> 37
agcttggtgg tggatgaaac                                                   20


<210> 38
<211> 20
<212> DNA
<213> Artificial Sequence

<220>
<221> source
<223> /note="Description of Artificial Sequence: Synthetic
      primer"

<400> 38
ccctcttcag caaagcagac                                                   20


<210> 39
<211> 20
<212> DNA
<213> Artificial Sequence

<220>
<221> source
<223> /note="Description of Artificial Sequence: Synthetic
      primer"

<400> 39

catctgtttg aatggcgatg                                                    20

<210> 40
<211> 20
<212> DNA
<213> Artificial Sequence

<220>
<221> source
<223> /note="Description of Artificial Sequence: Synthetic
      primer"

<400> 40
ttcccaccaa ggtctgaaag                                                    20

<210> 41
<211> 19
<212> DNA
<213> Artificial Sequence

<220>
<221> source
<223> /note="Description of Artificial Sequence: Synthetic
      primer"

<400> 41
caccgcatct ggagaacca                                                     19

<210> 42
<211> 21
<212> DNA
<213> Artificial Sequence

<220>
<221> source
<223> /note="Description of Artificial Sequence: Synthetic
      primer"

<400> 42
gcccatttcc tcggtgtagt t                                                  21

<210> 43
<211> 27
<212> DNA
<213> Artificial Sequence

<220>
<221> source
<223> /note="Description of Artificial Sequence: Synthetic
      primer"

<400> 43
aaaggatcca ccatgtctag actggac                                            27

<210> 44
<211> 27
<212> DNA
<213> Artificial Sequence

<220>
<221> source
<223> /note="Description of Artificial Sequence: Synthetic
      primer"

<400> 44
aaagtcgact tacccgggga gcatgtc                                          27


<210> 45
<211> 30
<212> DNA
<213> Artificial Sequence

<220>
<221> source
<223> /note="Description of Artificial Sequence: Synthetic
      primer"

<400> 45
aaagaattcg ccaccatggt gagcaagggc                                       30


<210> 46
<211> 27
<212> DNA
<213> Artificial Sequence

<220>
<221> source
<223> /note="Description of Artificial Sequence: Synthetic
      primer"

<400> 46
aaagaattcc tagctactag ctagtcg                                          27


<210> 47
<211> 22
<212> DNA
<213> Artificial Sequence

<220>
<221> source
<223> /note="Description of Artificial Sequence: Synthetic
      primer"

<400> 47
tttgagagcc tttagccgcc gc                                               22


<210> 48
<211> 23
<212> DNA
<213> Artificial Sequence

<220>
<221> source
<223> /note="Description of Artificial Sequence: Synthetic
      primer"

<400> 48

cgtcaaggca ctcttgccta cgc                                                23

<210> 49
<211> 22
<212> DNA
<213> Artificial Sequence

<220>
<221> source
<223> /note="Description of Artificial Sequence: Synthetic
      primer"

<400> 49
cgtcaaggca ctcttgccta cg                                                 22

<210> 50
<211> 18
<212> DNA
<213> Artificial Sequence

<220>
<221> source
<223> /note="Description of Artificial Sequence: Synthetic
      primer"

<400> 50
aaacttgtgg tagttgga                                                      18

<210> 51
<211> 20
<212> DNA
<213> Artificial Sequence

<220>
<221> source
<223> /note="Description of Artificial Sequence: Synthetic
      primer"

<400> 51
tcaagtcctt tgcccatttt                                                    20

<210> 52
<211> 20
<212> DNA
<213> Artificial Sequence

<220>
<221> source
<223> /note="Description of Artificial Sequence: Synthetic
      primer"

<400> 52
tgcatggcat tagcaaagac                                                    20

<210> 53
<211> 19
<212> DNA
<213> Artificial Sequence

<220>
<221> source
<223> /note="Description of Artificial Sequence: Synthetic
      primer"

<400> 53
gaagaaagag gaggggctg                                                    19


<210> 54
<211> 20
<212> DNA
<213> Artificial Sequence

<220>
<221> source
<223> /note="Description of Artificial Sequence: Synthetic
      primer"

<400> 54
gcgctacctg attccaattc                                                   20


<210> 55
<211> 20
<212> DNA
<213> Artificial Sequence

<220>
<221> source
<223> /note="Description of Artificial Sequence: Synthetic
      primer"

<400> 55
acacaagctt cctttccgtc                                                   20


<210> 56
<211> 20
<212> DNA
<213> Artificial Sequence

<220>
<221> source
<223> /note="Description of Artificial Sequence: Synthetic
      primer"

<400> 56
tctgagcttt ggaagctctc                                                   20


<210> 57
<211> 21
<212> DNA
<213> Artificial Sequence

<220>
<221> source
<223> /note="Description of Artificial Sequence: Synthetic
      primer"

<400> 57

tccctctcag gcataaggta a                                                21

<210> 58
<211> 24
<212> DNA
<213> Artificial Sequence

<220>
<221> source
<223> /note="Description of Artificial Sequence: Synthetic
      primer"

<400> 58
cgaacagtga atatttcctt tgat                                             24

<210> 59
<211> 23
<212> DNA
<213> Artificial Sequence

<220>
<221> source
<223> /note="Description of Artificial Sequence: Synthetic
      primer"

<400> 59
tcataatgct tgctctgata gga                                              23

<210> 60
<211> 22
<212> DNA
<213> Artificial Sequence

<220>
<221> source
<223> /note="Description of Artificial Sequence: Synthetic
      primer"

<400> 60
ggccaaaaat ttaatcagtg ga                                               22

<210> 61
<211> 25
<212> DNA
<213> Artificial Sequence

<220>
<221> source
<223> /note="Description of Artificial Sequence: Synthetic
      primer"

<400> 61
agagatagga gggtaagttt ttttt                                            25

<210> 62
<211> 29
<212> DNA
<213> Artificial Sequence

<220>
<221> source
<223> /note="Description of Artificial Sequence: Synthetic
      primer"

<400> 62
ccaaaacaac taactttact cccacacaa                                         29


<210> 63
<211> 22
<212> DNA
<213> Artificial Sequence

<220>
<221> source
<223> /note="Description of Artificial Sequence: Synthetic
      primer"

<400> 63
tgtttgaagt atgatgtatt ag                                                22


<210> 64
<211> 30
<212> DNA
<213> Artificial Sequence

<220>
<221> source
<223> /note="Description of Artificial Sequence: Synthetic
      primer"

<400> 64
gagttaaaga gttttgtttt taaaaattat                                        30


<210> 65
<211> 30
<212> DNA
<213> Artificial Sequence

<220>
<221> source
<223> /note="Description of Artificial Sequence: Synthetic
      primer"

<400> 65
tcccaaatct aataatttat catatctttc                                        30


<210> 66
<211> 21
<212> DNA
<213> Artificial Sequence

<220>
<221> source
<223> /note="Description of Artificial Sequence: Synthetic
      primer"

<400> 66

catatctttc aaaactactc c                                              21

<210> 67
<211> 24
<212> DNA
<213> Artificial Sequence

<220>
<221> source
<223> /note="Description of Artificial Sequence: Synthetic
      primer"

<400> 67
acactaacat ttataaacaa aatc                                           24

<210> 68
<211> 18
<212> DNA
<213> Artificial Sequence

<220>
<221> source
<223> /note="Description of Artificial Sequence: Synthetic
      primer"

<400> 68
ctttctcttt tccaatct                                                  18

<210> 69
<211> 21
<212> DNA
<213> Artificial Sequence

<220>
<221> source
<223> /note="Description of Artificial Sequence: Synthetic
      primer"

<400> 69
aatttattgg gattataggg g                                              21

<210> 70
<211> 24
<212> DNA
<213> Artificial Sequence

<220>
<221> source
<223> /note="Description of Artificial Sequence: Synthetic
      primer"

<400> 70
aacaacaaaa cctaaaaaca aacc                                           24

<210> 71
<211> 18
<212> DNA
<213> Artificial Sequence

<220>
<221> source
<223> /note="Description of Artificial Sequence: Synthetic
      primer"

<400> 71
acaaacccaa caacaaat                                                    18


<210> 72
<211> 29
<212> DNA
<213> Artificial Sequence

<220>
<221> source
<223> /note="Description of Artificial Sequence: Synthetic
      primer"

<400> 72
aagttggaaa agtggtgaat ttagaagta                                        29


<210> 73
<211> 25
<212> DNA
<213> Artificial Sequence

<220>
<221> source
<223> /note="Description of Artificial Sequence: Synthetic
      primer"

<400> 73
aacaaactaa atccacactc atatt                                            25


<210> 74
<211> 23
<212> DNA
<213> Artificial Sequence

<220>
<221> source
<223> /note="Description of Artificial Sequence: Synthetic
      primer"

<400> 74
aaaagtggtg aatttagaag tat                                              23


<210> 75
<211> 23
<212> DNA
<213> Artificial Sequence

<220>
<221> source
<223> /note="Description of Artificial Sequence: Synthetic
      primer"

<400> 75

gttgtttttt gggtattaaa ggt                                              23

<210> 76
<211> 27
<212> DNA
<213> Artificial Sequence

<220>
<221> source
<223> /note="Description of Artificial Sequence: Synthetic
      primer"

<400> 76
ccaactatct tcatcttaat aacatcc                                          27

<210> 77
<211> 25
<212> DNA
<213> Artificial Sequence

<220>
<221> source
<223> /note="Description of Artificial Sequence: Synthetic
      primer"

<400> 77
gtgaatatag ttgtaatttt attgt                                            25

<210> 78
<211> 18
<212> DNA
<213> Artificial Sequence

<220>
<221> source
<223> /note="Description of Artificial Sequence: Synthetic
      primer"

<400> 78
gatttgtggt aggtattg                                                    18

<210> 79
<211> 25
<212> DNA
<213> Artificial Sequence

<220>
<221> source
<223> /note="Description of Artificial Sequence: Synthetic
      primer"

<400> 79
tagttgggat gtgtagagtt tgaga                                            25

<210> 80
<211> 22
<212> DNA
<213> Artificial Sequence

<220>
<221> source
<223> /note="Description of Artificial Sequence: Synthetic
      primer"

<400> 80
aaaccaaaac aatccttcta ct                                                22


<210> 81
<211> 19
<212> DNA
<213> Artificial Sequence

<220>
<221> source
<223> /note="Description of Artificial Sequence: Synthetic
      primer"

<400> 81
atgtgtagag tttgagaga                                                    19


<210> 82
<211> 15
<212> DNA
<213> Artificial Sequence

<220>
<221> source
<223> /note="Description of Artificial Sequence: Synthetic
      primer"

<400> 82
aaagttgggt gtggt                                                        15


<210> 83
<211> 23
<212> DNA
<213> Artificial Sequence

<220>
<221> source
<223> /note="Description of Artificial Sequence: Synthetic
      primer"

<400> 83
aagtttttgt gggggatttg tat                                               23


<210> 84
<211> 23
<212> DNA
<213> Artificial Sequence

<220>
<221> source
<223> /note="Description of Artificial Sequence: Synthetic
      primer"

<400> 84

ccacccacta accttaacct cta                                              23

<210> 85
<211> 17
<212> DNA
<213> Artificial Sequence

<220>
<221> source
<223> /note="Description of Artificial Sequence: Synthetic
      primer"

<400> 85
tgggggattt gtattga                                                    17

<210> 86
<211> 24
<212> DNA
<213> Artificial Sequence

<220>
<221> source
<223> /note="Description of Artificial Sequence: Synthetic
      primer"

<400> 86
gttagaggtt aaggttagtg ggtg                                            24

<210> 87
<211> 25
<212> DNA
<213> Artificial Sequence

<220>
<221> source
<223> /note="Description of Artificial Sequence: Synthetic
      primer"

<400> 87
aaaccttaaa aacttaacca aatcc                                           25

<210> 88
<211> 15
<212> DNA
<213> Artificial Sequence

<220>
<221> source
<223> /note="Description of Artificial Sequence: Synthetic
      primer"

<400> 88
catcacctcc accac                                                      15

<210> 89
<211> 20
<212> DNA
<213> Artificial Sequence

<220>
<221> source
<223> /note="Description of Artificial Sequence: Synthetic
      primer"

<400> 89
atcccaaaat caacccaacc                                                    20